# EUROPEAN PATENT APPLICATION

(11) **EP 4 628 481 A1**
(43) Date of publication of application: **08.10.2025**
(21) Application number: 23896953.9
(22) Date of filing: 01.12.2023
(51) Int. Cl.: C07C 271/44, C07C 43/21, C07C 43/23, C07D 241/02, A61K 31/495, A61K 31/215, A61P 17/00, A61P 31/04, A61P 37/00

(54) **COMPOUND AND USE THEREOF IN TREATMENT OF TREG-RELATED DISEASES**

(30) Priority: 02.12.2022 CN 202211544528
(71) Applicant: Benethera (Shaoxing) Biotechnology Co., Ltd., Shaoxing, Zhejiang 312025 (CN)
(72) Inventor: WANG, Tie, Calgary Alberta T3A4J1 (CA); FAN, Futian, Beijing 100085 (CN)
(74) Representative: Bayramoglu et al.
(86) International application number: PCT/CN2023/135952
(87) International publication number: WO 2024/114811

(57) **Abstract**

Provided are a compound and a use thereof in treatment of Treg-related diseases, in particular, a use thereof in contributing to prevention and/or treatment of diseases prevented or treated by the compound by increasing the quantity of Tregs. It is found by means of experiments that O-1602 can effectively promote proliferation of Tregs, increase the quantity of Tregs, and is expected to contribute to prevention or treatment of some diseases by increasing the quantity of Tregs, for example, an autoimmune disease, an allergic disease, transplant rejection, a graft-versus-host disease (GvHD), and an inflammatory disease. In addition, a series of new compounds are prepared, and can also effectively promote proliferation of Tregs, increase the quantity of Tregs, have good physiochemical properties such as solubility and stability, have diversified expected administration routes, and have very good application and research value. very good application and research value.

## Description

### Technical Field

The present invention relates to the field of biomedical technology, specifically to a compound and use thereof in the treatment of Treg-related diseases, in particular in contributing to the prevention and/or treatment of diseases by increasing the quantity of Tregs, for example, an autoimmune disease, an allergic disease, transplant rejection reaction, a graft-versus-host disease (GvHD), and an inflammatory disease.

### Background Art

Regulatory T cells (regulatory T cells, Tregs) are a subset of T cells that express Foxp3, CD25, CD4 phenotypes, and have significant immunosuppression effects. It is able to inhibit the immune response of other cells and is a major controller of self-tolerance. For example, when immune cells such as T cells, B cells, and NK cells are exposed to foreign invading harmful substances such as bacteria and viruses, an immune response can be generated to recognize and remove the harmful substances so as to protect the body from attack. When the war between these cells and the harmful substances is over, Tregs will signal the stop of attack. If immune cells are always in a hyperresponsive state, normal tissues of the body can be treated as an attackers, resulting in damage to the body, causing, for example, autoimmune diseases. Tregs plays an important role in patients with autoimmune diseases by inhibiting the activation and proliferation of latent autoreactive T cells in the normal body through active regulation, exerting the characteristics of immunosuppression and immune homeostasis.

Autoimmune diseases are currently primarily treated by inhibiting or blocking pathological immune responses in the body, e.g., by using immunosuppressants (e.g., prednisone, cyclosporine, azathioprine), etc. which are characterized by a variety of systemic side effects and interactions. Researchers have therefore sought to develop a new drug that can specifically affect the mechanisms involved in disease events, examples of which include natalizumab and infliximab. Natalizumab is a monoclonal antibody and selective inhibitor of IgG4, an adhesion molecule located on the surface of leukocytes. Natalizumab inhibits leukocyte migration to inflammatory foci and is used to treat a particularly aggressive form of plaque-progressive multiple sclerosis. Infliximab is a chimeric monoclonal antibody against tumor necrosis factor alpha (TNFα), which plays a key role in the autoimmune inflammatory response. Infliximab is used in rheumatoid arthritis, Crohn's disease, Bechterew's disease, and psoriasis. Although these newly emerging drugs may act specifically, serious side effects, such as progressive multifocal leukoencephalopathy, may also occur. For this reason, natalizumab was withdrawn from the market only three months after its first registration in the United States. Moreover, the cost of these new active substances is very high. Therefore, there is still a need to develop new drugs that are low in cost, high in efficiency, and have few side effects.

### Summary of the Invention

To overcome the shortcomings of existing technology, the present invention provides a compound and a use thereof in the treatment of Treg-related diseases, in particular, a use thereof in the prevention and/or treatment of diseases that banefit from an increase in the quantity of Tregss, for example, as autoimmune diseases, an allergic diseases, transplant rejection reactions, graft-versus-host disease (GvHD), and inflammatory diseases.

In a first aspect of the present invention, provided is a compound having the following structure: 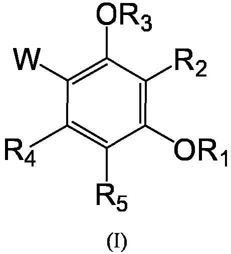 wherein
R₁ has the following structure: 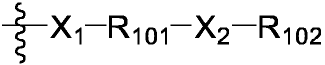 , wherein X₁ is selected from a single bond, -C(O)-, -C(O)O-, -C(O)N(R₁₀₃)-, 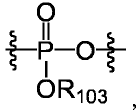 -S(O)₂-; X₂ is selected from a single bond, -O-, -S-, -S-S-, -N(R₁₀₄)-, -S(O)₂-, -S(O)₂N(R₁₀₄)-, -S(O)-, -S(O)N(R₁₀₄)-, -C(O)-, -C(O)O-, -C(O)N(R₁₀₄)-, -OC(O)-, -OC(O)N(R₁₀₄)-, -N(R₁₀₄)C(O)O-, -N(R₁₀₄)C(O)-, -N(R₁₀₄)S(O)₂-, 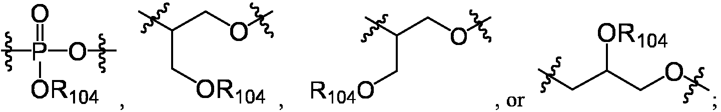
R₁₀₁ is selected from a single bond, alkylene, alkenylene, phenylene (e.g., 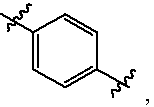 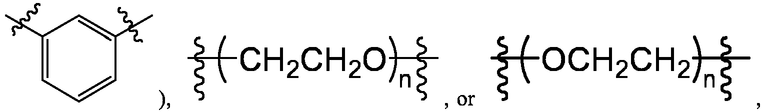 amino acid residue, oligopeptide residue, polypeptide residue, monosaccharide residue, oligosaccharide residue, or polysaccharide residue (e.g., 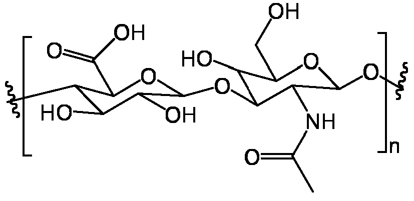 ); n is an integer of 1-100; R₁₀₂ is selected from H, alkyl, cycloalkyl, phenyl, or heterocyclyl (in particular saturated heterocyclyl); R₁₀₃ and R₁₀₄ are independently selected from H, or alkyl; wherein the alkylene, alkenylene, phenylene, alkyl, cycloalkyl, phenyl, and heterocyclyl are optionally substituted with one or more independent R';
R₂, R₄, and R₅ are independently selected from H, halogen, hydroxyl, mercapto, amino, cyano, azido, isocyanate, sulfonyl chloride, sulfonyl fluoride, amido, sulfonamido, formic acid group, nitro, formaldehyde group, trifluoromethyl, sulfonic acid group, phosphite group, trialkyltin group, trialkylsilyl, alkyl, alkenyl , or alkynyl;
R₃ has the following structure: 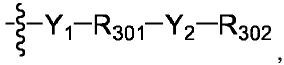 wherein Y₁ is selected from a single bond, -C(O)-, -C(O)O-, -C(O)N(R₃₀₃)-, 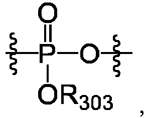 -S(O)₂-; Y₂ is selected from a single bond, -O-, -S-, -S-S-, -N(R₃₀₄)-, -S(O)z-, -S(O)₂N(R₃₀₄)-, -S(O)-, -S(O)N(R₃₀₄)-, -C(O)-, -C(O)O-, -C(O)N(R₃₀₄)-, -OC(O)-, -OC(O)N(R₃₀₄)-, -N(R₃₀₄)C(O)O-, -N(R₃₀₄)C(O)-, -N(R₃₀₄)S(O)₂-, 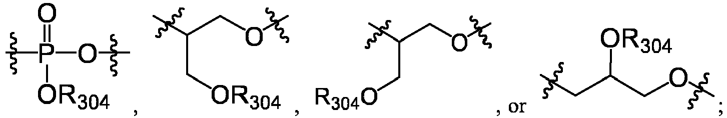
R₃₀₁ is selected from a single bond, alkylene, alkenylene, phenylene (e.g., 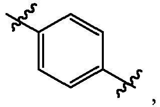 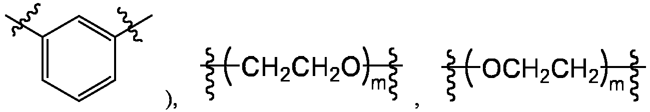 , amino acid residue, oligopeptide residue, polypeptide residue, monosaccharide residue, oligosaccharide residue, or polysaccharide residue (e.g., 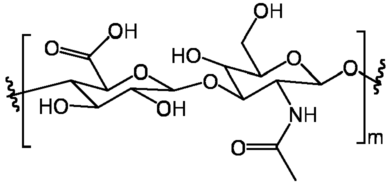 ); m is an integer of 1-100; R₃₀₂ is selected from H, alkyl, cycloalkyl, phenyl, or heterocyclyl; R₃₀₃ and R₃₀₄ are independently selected from H, or alkyl; wherein the alkyl and alkylene are optionally substituted with one or more independent R';
or, R₄ and R₅, together with the carbon atom to which they are attached, form a cyclic group (in particular a saturated cyclic group such as cycloalkyl, heterocyclyl); wherein the cyclic group is optionally substituted with one or more independent R';
W is selected from the following structures: 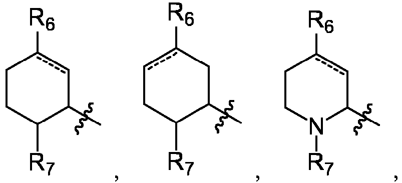 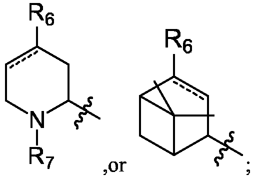
wherein -̅ -̅ -̅ -̅ -̅ -̅ represents a single bond or a double bond;
each R₆ independently has the following structure: 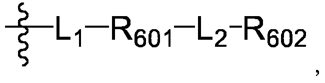 wherein L₁ and L₂ are independently selected from a single bond, -O-, -S-, -N(R₆₀₃)-, -S(O)₂-, -S(O)₂N(R₆₀₃)-, -S(O)-, -S(O)N(R₆₀₃)-, -C(O)-, -C(O)O-, -C(O)N(R₆₀₃)-, -OC(O)-, -OC(O)N(R₆₀₃)-, -N(R₆₀₃)C(O)O-, -N(R₆₀₃)C(O)-, or -N(R₆₀₃)S(O)₂-; R₆₀₁ is selected from a single bond, or alkylene; R₆₀₂ is selected from H, or alkyl; and R₆₀₃ is selected from H, or alkyl; wherein the alkyl and alkylene are optionally substituted with one or more independent R';
each R₇ is independently selected from H, alkyl, alkenyl, alkynyl, wherein the alkyl, alkenyl, and alkynyl are optionally substituted with one or more independent R';
each R' is independently selected from halogen, hydroxyl, mercapto, amino, cyano, azido, isocyanate, sulfonyl chloride, sulfonyl fluoride, amido, sulfonamido, formic acid group, nitro, formaldehyde group, trifluoromethyl, sulfonic acid group, phosphite group, trialkyltin group, trialkylsilyl, -CH=NH, 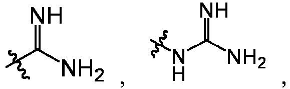 alkoxy, alkylamino, substituted or unsubstituted alkyl, substituted or unsubstituted alkenyl, substituted or unsubstituted alkynyl, substituted or unsubstituted cycloalkyl, substituted or unsubstituted cycloalkylalkyl, substituted or unsubstituted aryl, substituted or unsubstituted aralkyl, or substituted or unsubstituted heterocyclyl, substituted or unsubstituted heterocyclylalkyl; and
the compound represented by formula I is not 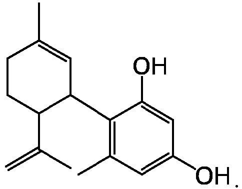 Specifically, X₁ can be selected from a single bond, -C(O)-, -C(O)O-, -C(O)N(H)-, 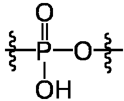 , or -S(O)₂-.

In some embodiments of the present invention, X₁ is -C(O)-.

In other embodiments of the present invention, X₁ is -C(O)O-.

In some embodiments of the present invention, X₁ is -C(O)N(H)-.

Specifically, the amino acids in the amino acids and oligopeptides, polypeptides can be selected from alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, valine, citrulline, or ornithine.

Specifically, the monosaccharide of the monosaccharides and oligosaccharides, polysaccharides can be selected from glyceraldehyde, erythrose, arabinose, ribose, xylose, lyxose, glucose, mannose, fructose, galactose, N-acetylglucosamine, N-acetylglucosamine, fucose, or N-acetylneuraminic acid.

In an embodiment of the present invention, the monosaccharide residue is a glucuronic acid residue.

In an embodiment of the present invention, the polysaccharide residue is a hyaluronic acid residue. Specifically, R₁₀₁ can be selected from a single bond, C1-6 alkylene (e.g. methylene, ethylene, propylene, butylene), 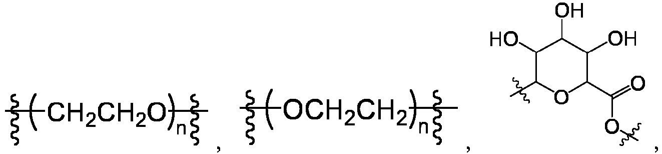 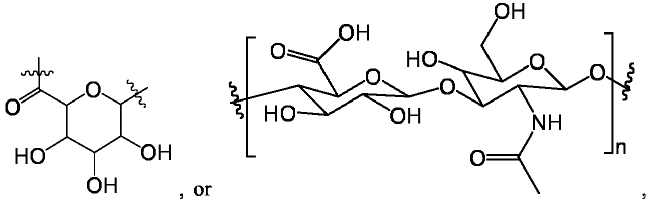 wherein the alkylene is optionally substituted with one or more independent R'; specifically, R' can be selected from hydroxyl, mercapto, amino, amido, sulfonamido, formic acid group, nitro, formaldehyde group, trifluoromethyl, 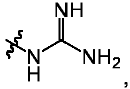 vinyl, ethynyl, substituted or unsubstituted phenyl (e.g., 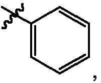 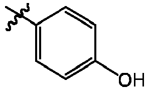 ), or heterocyclyl (e.g., 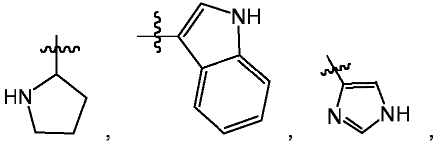 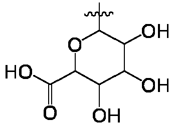 ).

Specifically, n can be 1, 2, 3, 4, 5, 6, 7, 8, 9, 10, 20, 30, 40, 50, 60, 70, 80, 90, or 100; in some embodiments of the present invention, n is an integer of 1-10.

In some embodiments of the present invention, R₁₀₁ is selected from a single bond, methylene, ethylene,

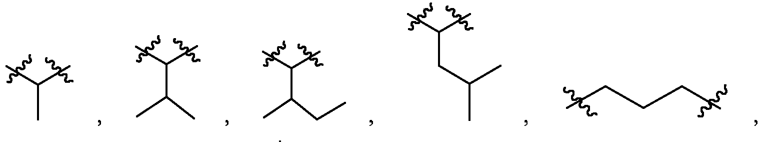

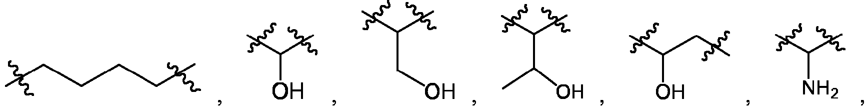

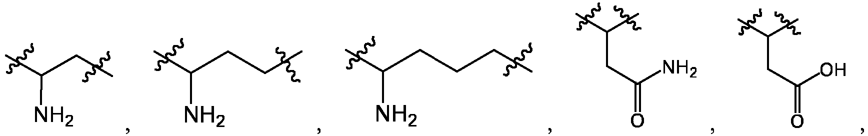

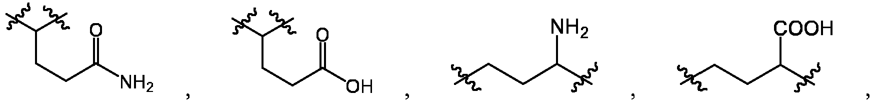

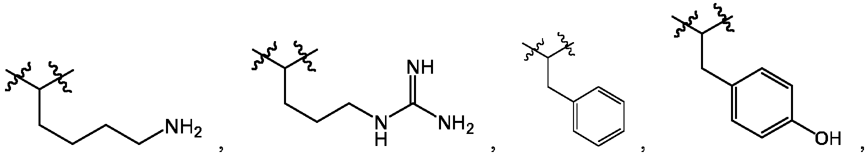

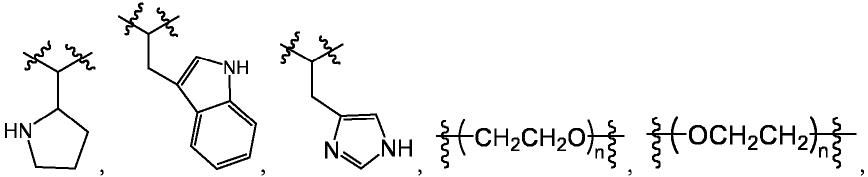

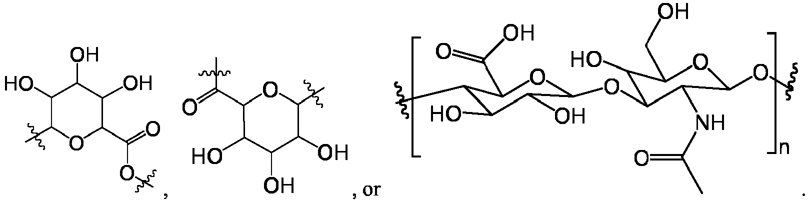

Specifically, X₂ is selected from a single bond, -O-, -S-, -S-S-, -N(R₁₀₄)-, -S(O)₂-, -S(O)₂N(R₁₀₄)-, -C(O)O-, or -C(O)N(R₁₀₄)-, wherein R₁₀₄ can be selected from H, or C1-6 alkyl.

Specifically, R₁₀₂ can be selected from H, C1-6 alkyl (in particular C1-3 alkyl, e.g., methyl, ethyl, n-propyl, isopropyl), 4- to 12-membered saturated heterocyclyl (including monocyclic heterocyclyl, or polycyclic heterocyclyl (in particular bicyclic heterocyclyl), including fused, spiro, bridged polycyclic heterocyclyl, (e.g., 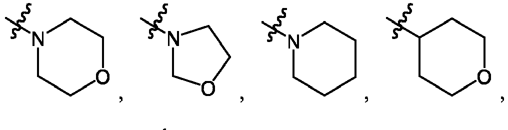 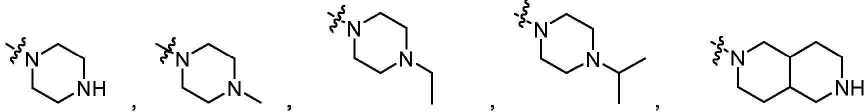 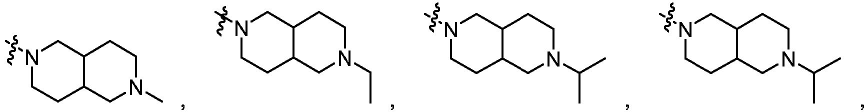 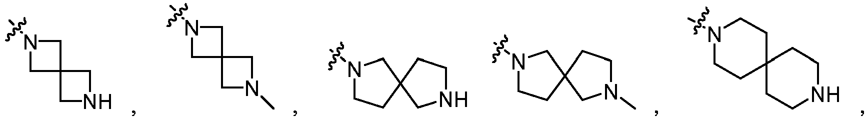 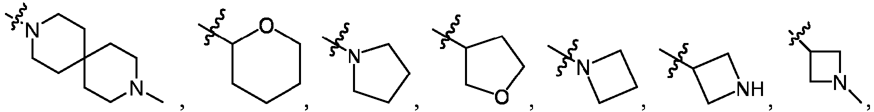 or 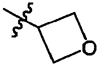 ) (in particular 4- to 6-membered monocyclic saturated heterocyclyl); wherein the alkyl and heterocyclyl are optionally substituted with one or more independent R'; Specifically, R' can be selected from C1-3 alkyl (e.g., methyl, ethyl, n-propyl, isopropyl), hydroxyl, mercapto, amino, amido, sulfonamido, formic acid group, nitro, formaldehyde group, trifluoromethyl, 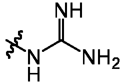 , vinyl, ethynyl, substituted or unsubstituted phenyl (e.g., 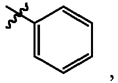 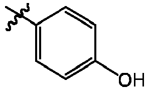 ), or heterocyclyl (e.g., 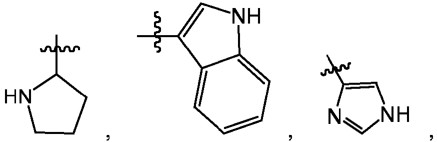 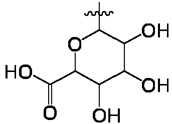 ).

In an embodiment of the present invention, R₁ has the following structure: 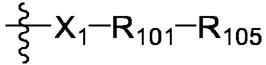 , wherein R₁₀₅ is selected from H, 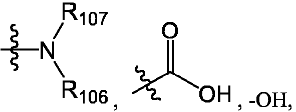 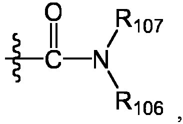 4- to 12-membered substituted or unsubstituted saturated heterocyclyl (including monocyclic heterocyclyl, or polycyclic heterocyclyls (in particular bicyclic heterocyclyls), polycyclic heterocycles including fused, spiro, bridged polycyclic heterocyclyls, (e.g., 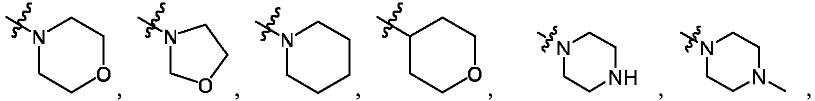 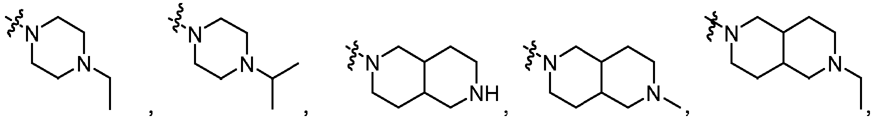 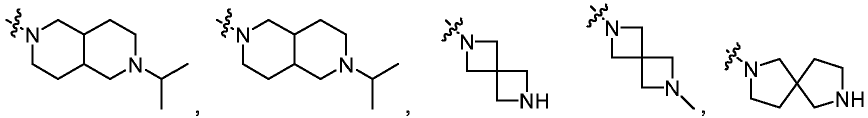 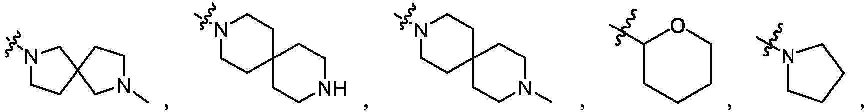 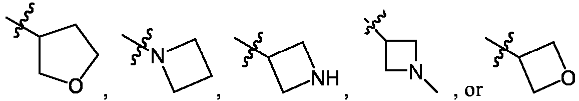 ) (in particular 4- to 6-membered monocyclic saturated heterocyclyls), R₁₀₆ and R₁₀₇ are independently selected from H, or C1-6 alkyl (in particular C1-3 alkyl, e.g. methyl, ethyl, n-propyl).

In some embodiments of the present invention, R₁₀₅ can be selected from H, amino,

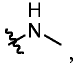

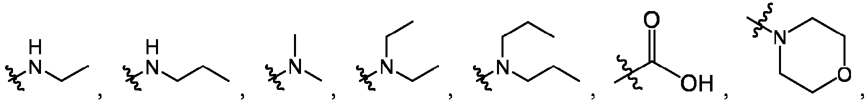

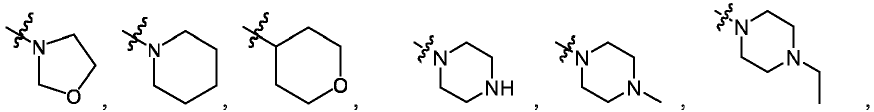

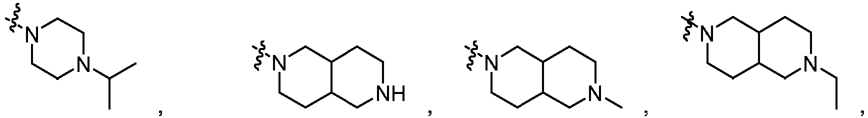

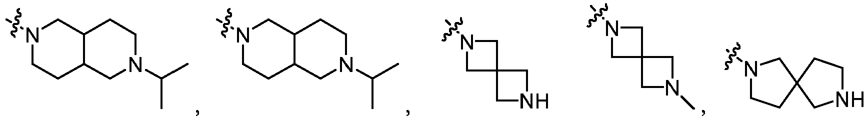

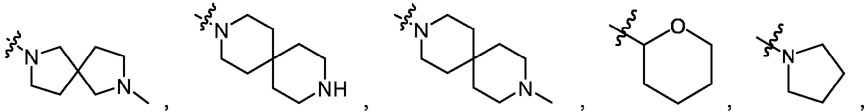

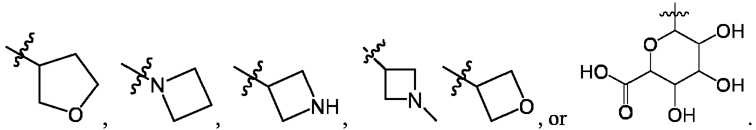

In some embodiments of the present invention, R₁ is H.

In still other embodiments of the present invention, R₁ is selected from 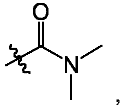 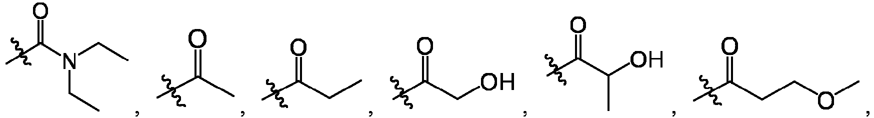 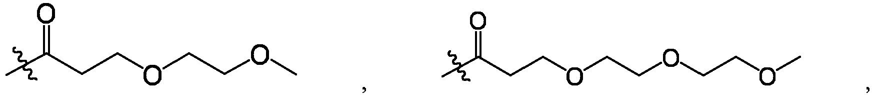 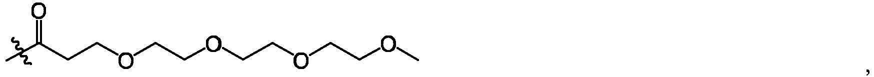 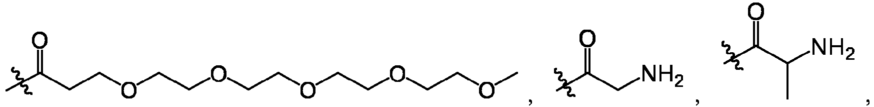 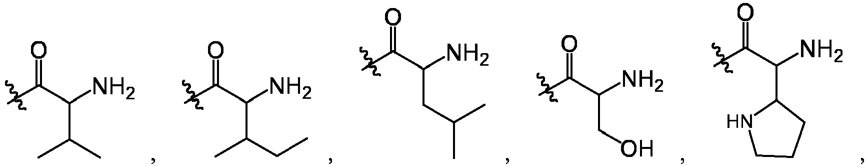 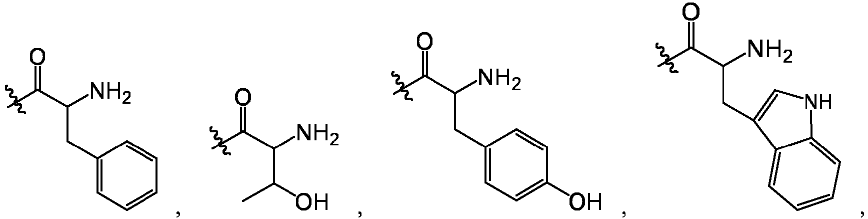 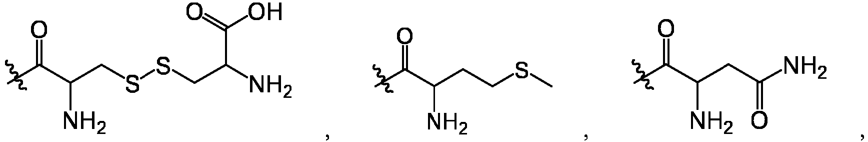 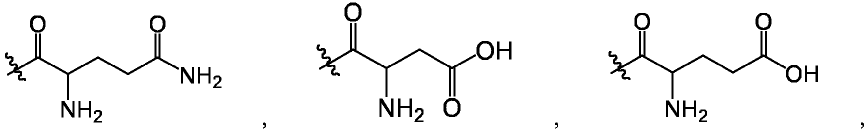 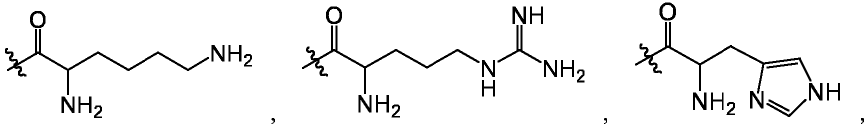 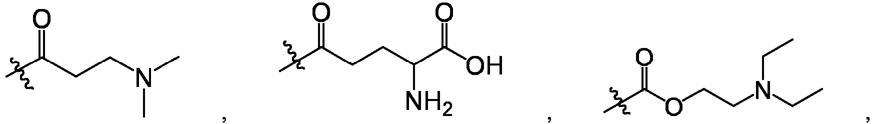 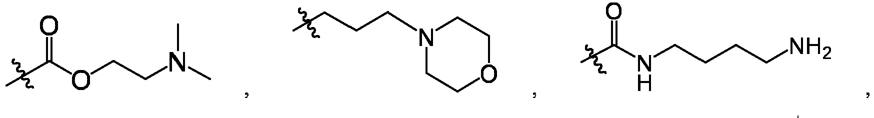 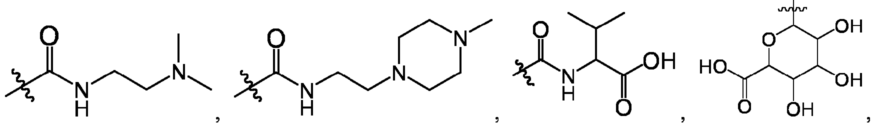 or

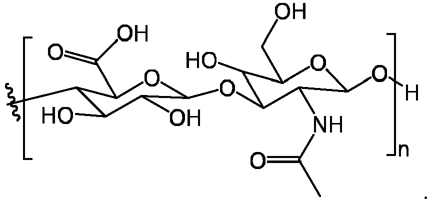

In some embodiments of the present invention, R₂ is H.

Specifically, Y₁ can be selected from a single bond, -C(O)-, -C(O)O-, -C(O)N(H)-, 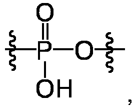 or -S(O)₂-.

In some embodiments of the present invention, Y₁ is -C(O)-.

In other embodiments of the present invention, Y₁ is -C(O)O-.

In some embodiments of the present invention, Y₁ is -C(O)N(H)-.

Specifically, R₃₀₁ can be selected from a single bond, C1-6 alkylene (e.g. methylene, ethylene, propylene, butylene), 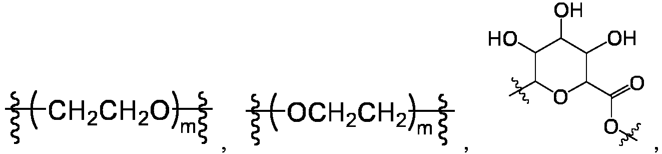 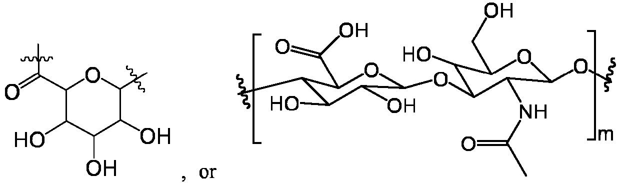 , wherein the alkylene is optionally substituted with one or more independent R'; specifically, R' can be selected from hydroxyl, mercapto, amino, amido, sulfonamido, formic acid group, nitro, formaldehyde group, trifluoromethyl, 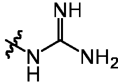 , vinyl, ethynyl, substituted or unsubstituted phenyl (e.g., 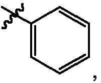 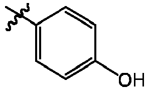 ), or heterocyclyl (e.g., 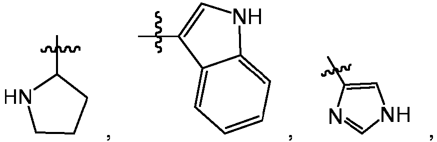 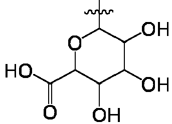 ).

Specifically, m can be 1, 2, 3, 4, 5, 6, 7, 8, 9, 10, 20, 30, 40, 50, 60, 70, 80, 90, or 100; in some embodiments of the present invention, m is an integer of 1-10.

In some embodiments of the present invention, R₃₀₁ is selected from a single bond, methylene, ethylene, 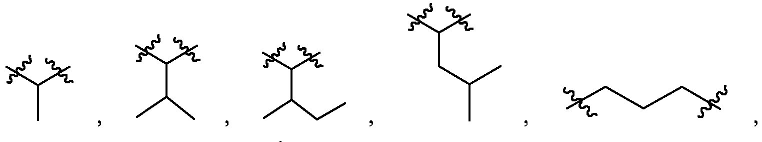 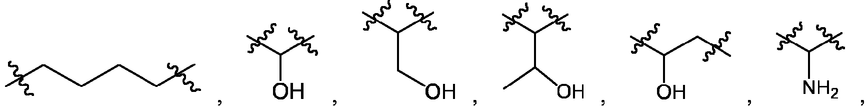 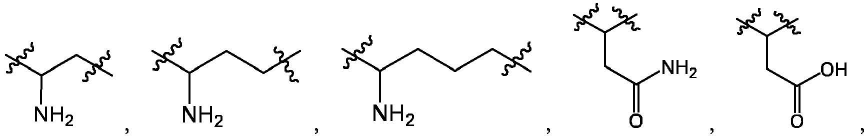 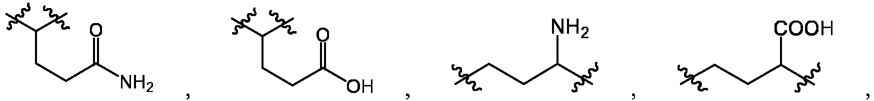 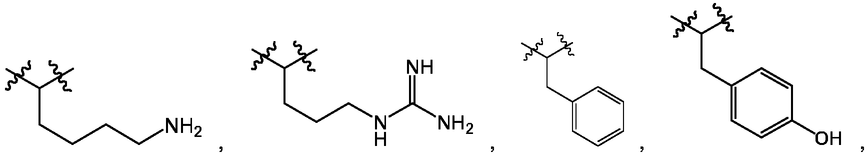 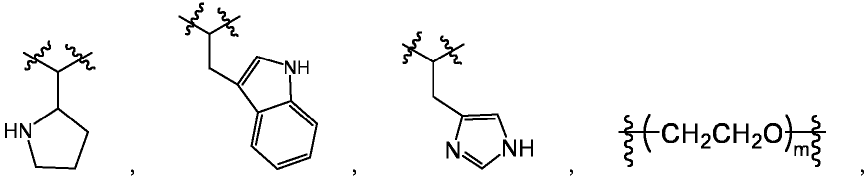 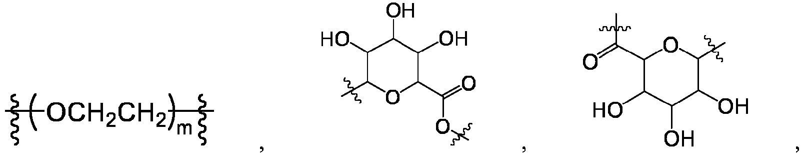 or

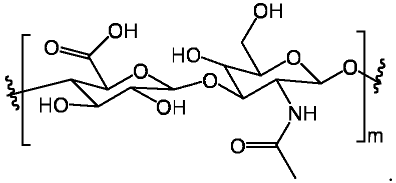

Specifically, Y₂ is selected from a single bond, -O-, -S-, -S-S-, -N(R₃₀₄)-, -S(O)z-, -S(O)₂N(R₃₀₄)-, -C(O)O-, or -C(O)N(R₃₀₄)-, wherein R₃₀₄ can be selected from H, or C1-6 alkyl.

Specifically, R₃₀₂ can be selected from H, C1-6 alkyl (in particular C1-3 alkyl, e.g., methyl, ethyl, n-propyl, isopropyl), 4- to 12-membered saturated heterocyclyl (including monocyclic heterocyclyl, or polycyclic heterocyclyl (in particular bicyclic heterocyclyl), including fused, spiro, bridged polycyclic heterocyclyl, (e.g., 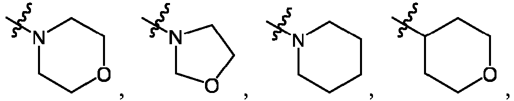 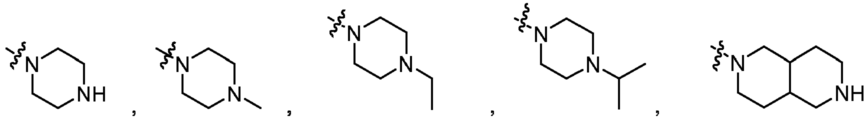 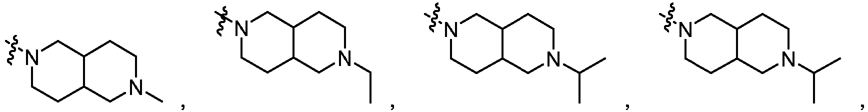 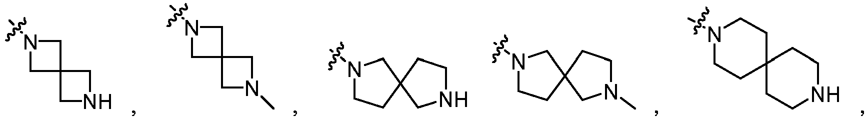 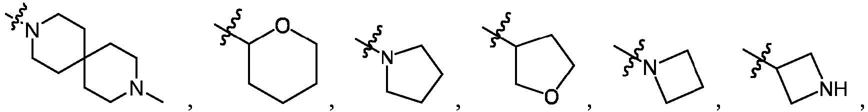 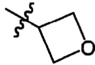 ) (in particular 4- to 6-membered monocyclic saturated heterocyclyl); wherein the alkyl and heterocyclyl are optionally substituted with one or more independent R'; Specifically, R' can be selected from C1-3 alkyl (e.g., methyl, ethyl, n-propyl, isopropyl), hydroxyl, mercapto, amino, amido, sulfonamido, formic acid group, nitro, formaldehyde group, trifluoromethyl, 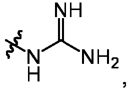 vinyl, ethynyl, substituted or unsubstituted phenyl (e.g., 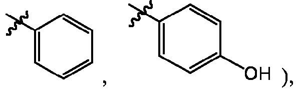 or heterocyclyl (e.g.,

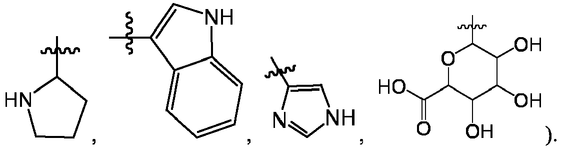

In an embodiment of the present invention, R₃ has the following structure: 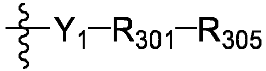 , wherein R₃₀₅ is selected from H, 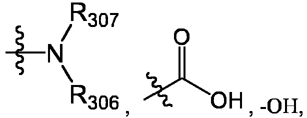 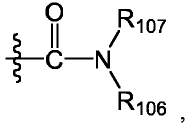 4- to 12-membered substituted or unsubstituted saturated heterocyclyl (including monocyclic heterocyclyls, or polycyclic heterocyclyls (in particular bicyclic heterocyclyls), polycyclic heterocycles including fused, spiro, bridged polycyclic heterocycles, e.g., 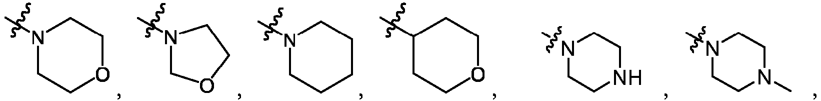 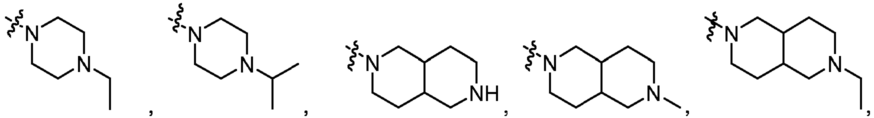 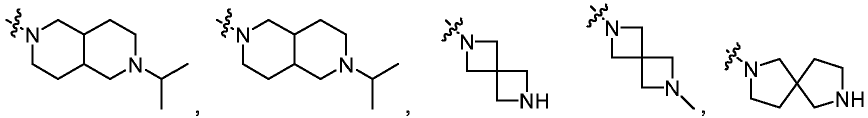 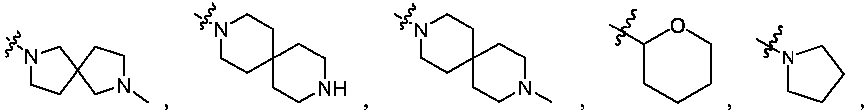 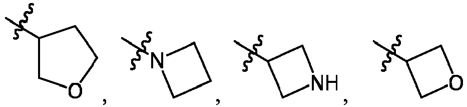 ) (in particular 4- to 6-membered monocyclic saturated heterocyclyls), R₃₀₆ and R₃₀₇ are independently selected from H, or C1-6 alkyl (in particular C1-3 alkyl, e.g. methyl, ethyl, n-propyl).

In some embodiments of the present invention, R₃₀₅ can be selected from H, amino,

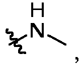

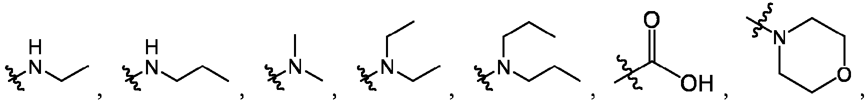

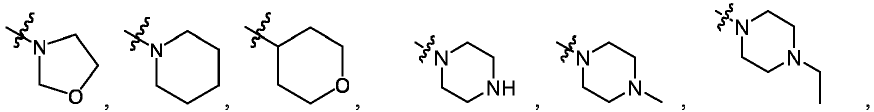

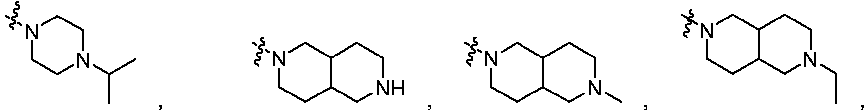

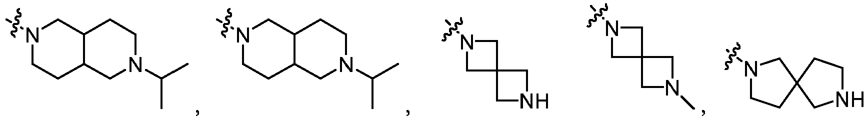

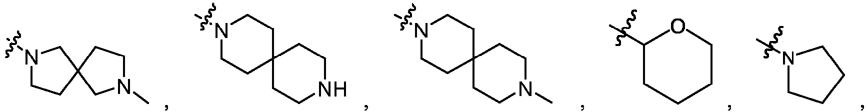

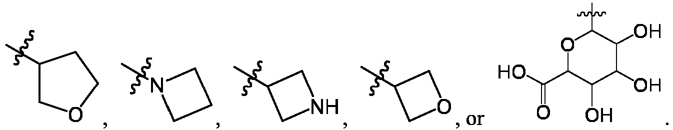

In some embodiments of the present invention, R₃ is H.

In still other embodiments of the present invention, R₃ is selected from

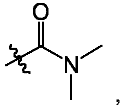

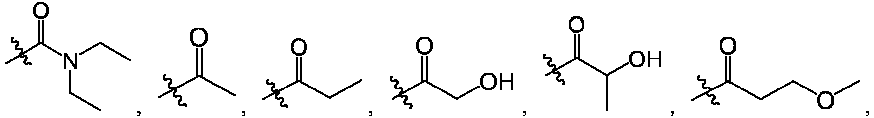

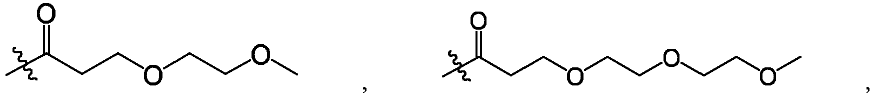

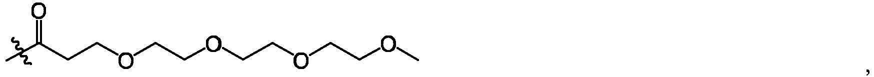

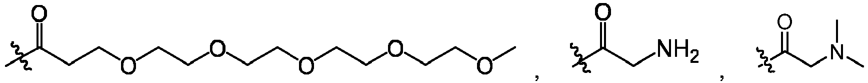

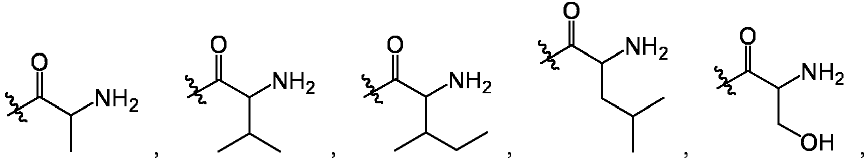

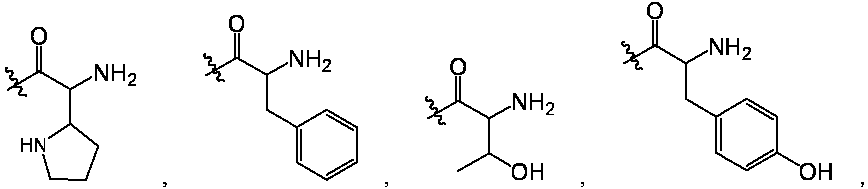

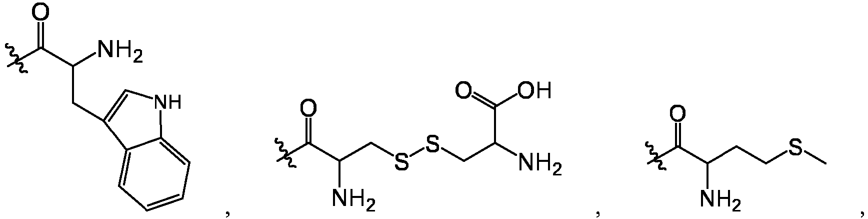

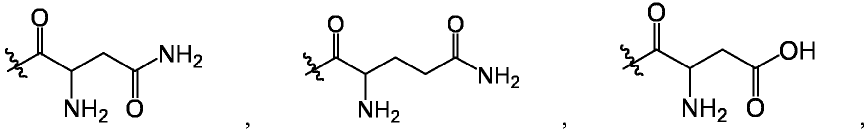

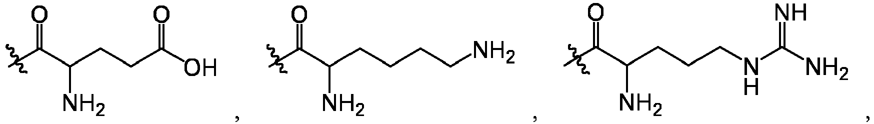

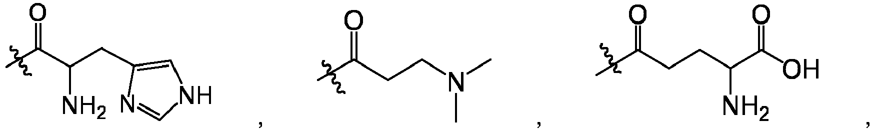

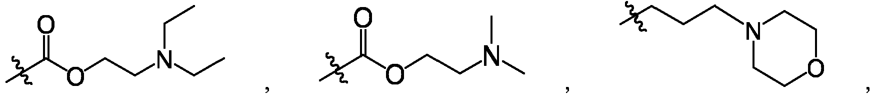

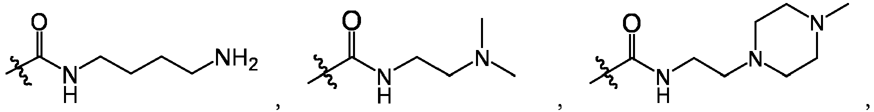

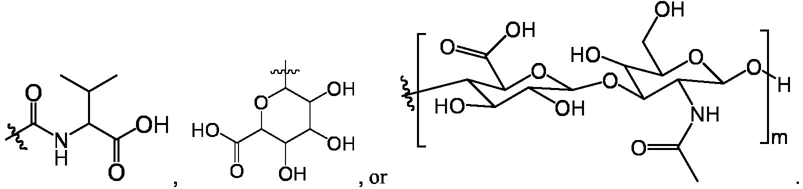

In some embodiments of the present invention, R₄ is H.

In still other embodiments of the present invention, R₄ is C1-6 alkyl, in particular C1-3 alkyl, wherein the alkyl is optionally substituted with one or more independent R'; Specifically, R' can be selected from fluorine, chlorine, bromine, iodine, hydroxyl, mercapto, amino, formic acid group, nitro, formaldehyde group, trifluoromethyl, vinyl, or ethynyl. In an embodiment of the present invention, R₄ is methyl.

In some embodiments of the present invention, R₅ is H.

In still other embodiments of the present invention, R₅ is C1-6 alkyl, in particular C1-3 alkyl, wherein the alkyl is optionally substituted with one or more independent R'; Specifically, R' can be selected from fluorine, chlorine, bromine, iodine, hydroxyl, mercapto, amino, formic acid group, nitro, formaldehyde group, trifluoromethyl, vinyl, or ethynyl. In an embodiment of the present invention, R₅ is methyl.

Specifically, R₆₀₁ can be selected from a single bond, C1-6 alkylene (in particular C1-3 alkylene, e.g., methylene, ethylene).

Specifically, R₆₀₂ can be selected from H, or C1-6 alkyl (in particular C1-3 alkyl, e.g. methyl, ethyl, n-propyl, isopropyl).

Specifically, R₆₀₃ can be selected from H, or C1-6 alkyl, in particular H.

Specifically, L₁ and L₂ can be independently selected from a single bond, -O-, -S-, -N(H), -S(O)₂-, -S(O)₂N(H)-, -C(O)-, -C(O)O-, -C(O)N(H)-, -OC(O)-, or -N(H)C(O)-.

Specifically, each R₆ can be independently selected from H, alkyl, or 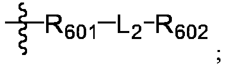 more specifically, each R₆ can be independently selected from H, C1-6 alkyl, 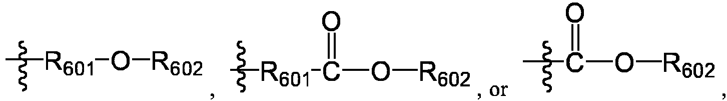 wherein R₆₀₁ is C1-6 alkylene, and R₆₀₂ is selected from H, or C1-6 alkyl.

In some embodiments of the present invention, each R₆ is independently C1-6 alkyl, in particular C1-3 alkyl, for example, methyl, ethyl, n-propyl, or isopropyl, in particular methyl.

In some embodiments of the present invention, each R₇ is independently C2-6 alkenyl, in particular C2-3 alkenyl, for example, vinyl, propenyl, allyl, or prop-1-en-2-yl ( 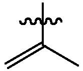 ), in particular

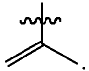

In some embodiments of the present invention, ------ represents a single bond.

In other still embodiments of the present invention, -̅ -̅ -̅ -̅ -̅ -̅ represents a double bond.

In some embodiments of the present invention, W is

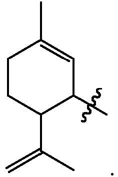

In an embodiment of the present invention, the compound has the following structure: 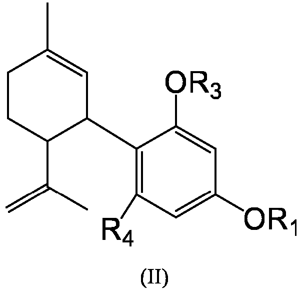 wherein R₁, R₃, and R₄ have the same meanings as defined above.

In an embodiment of the present invention, R₁ is H and R₃ is not H. For example, the compound may have the following structure:

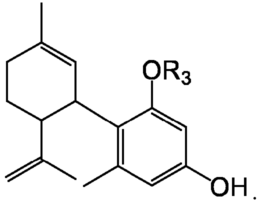

In another embodiment of the present invention, R₁ is not H and R₃ is H, for example, the compound may have the following structure:

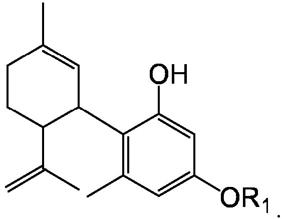

In another embodiment of the present invention, neither R₁ nor R₃ is H. For example, the compound may have the following structure:

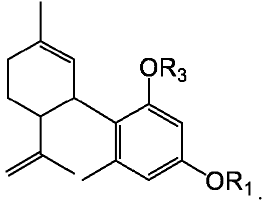

In some embodiments of the present invention, the compound has the following structure:

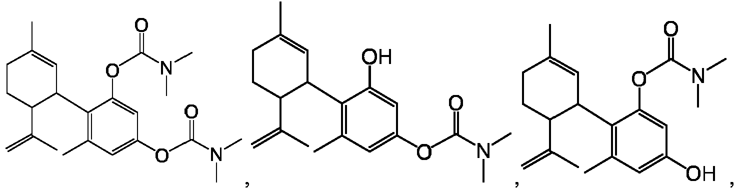

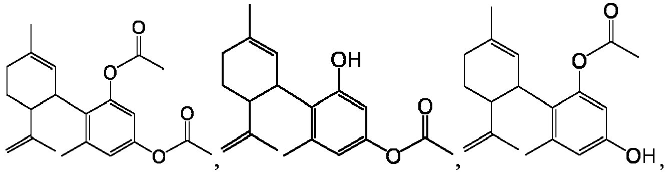

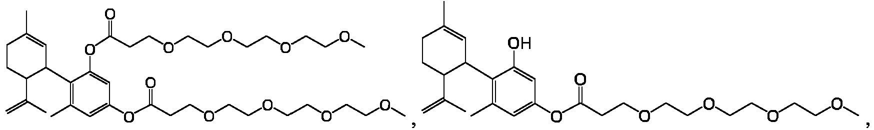

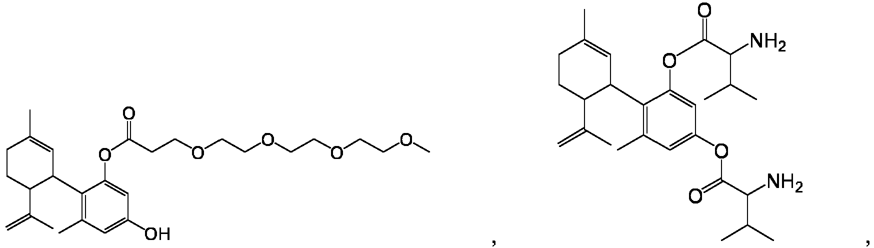

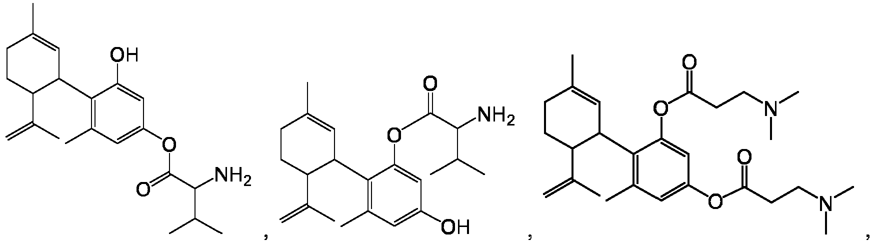

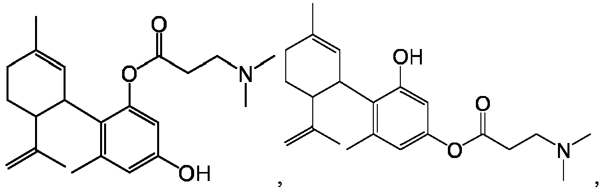

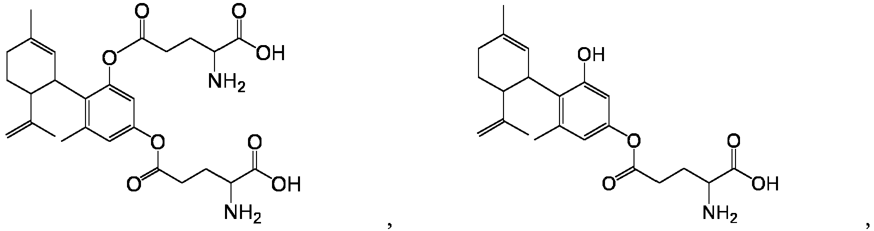

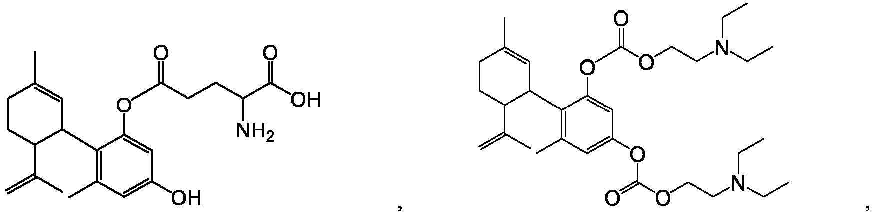

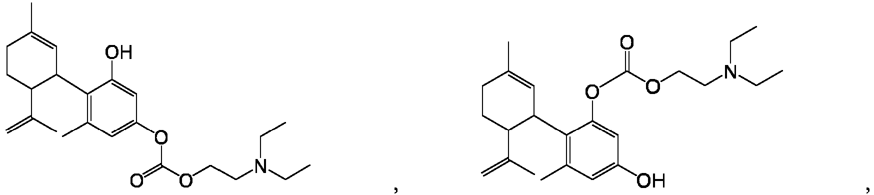

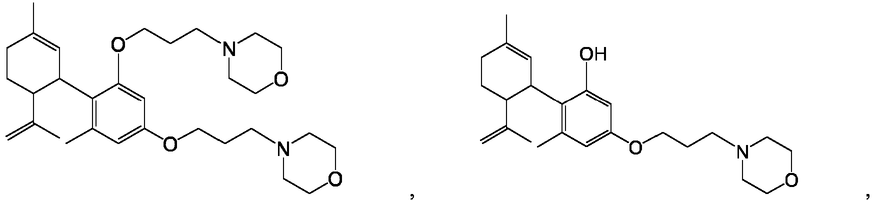

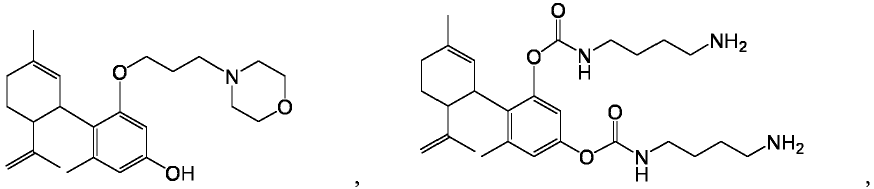

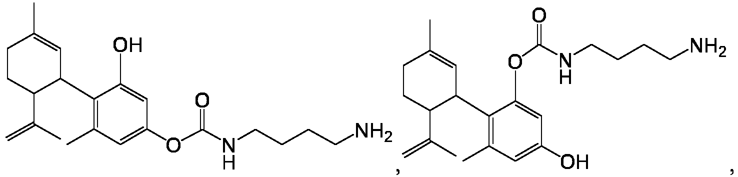

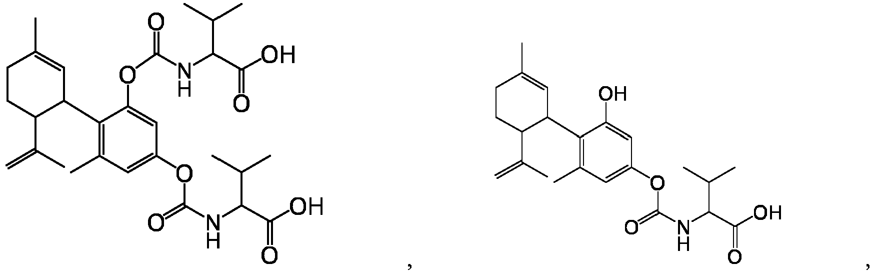

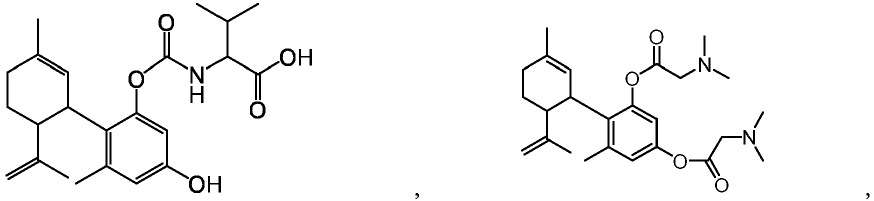

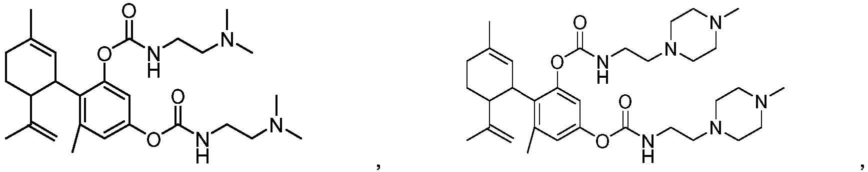

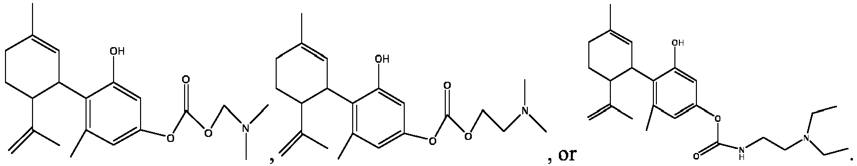

In a second aspect of the present invention, provided is the compound of the first aspect, a stereoisomer of the compound, any composition of the compound and the stereoisomer, any composition of the stereoisomers of the compound, or a pharmaceutically acceptable salt, ester, ether, optical isomer, isotope derivative, solvate (e.g., hydrate), prodrug, metal chelate, crystal form of the foregoing compound (or composition).

Specifically, the stereoisomer can be selected from the following structures: 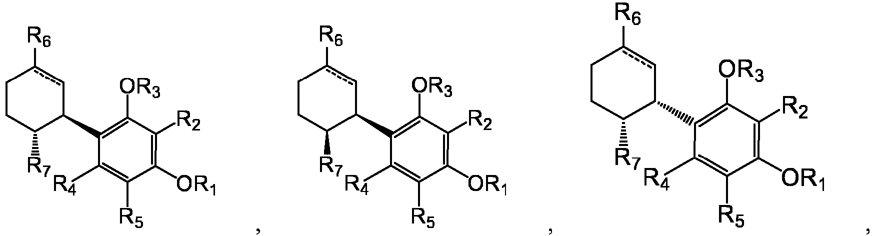 or 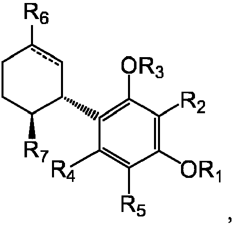 in particular 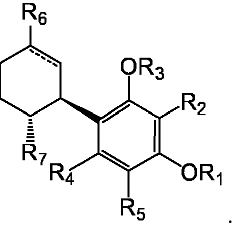 wherein R₁ to R₇ have the same meanings as defined in the first aspect of the present invention.

More specifically, the stereoisomer can be selected from the following structures: 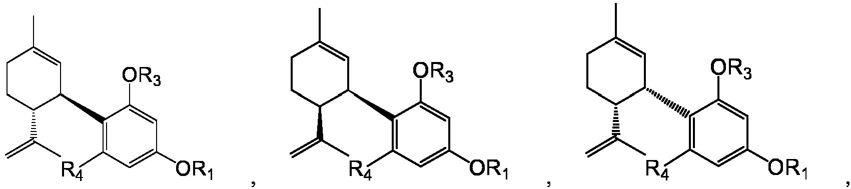 or 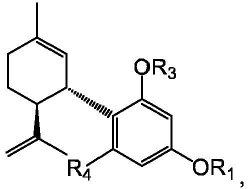 in particular 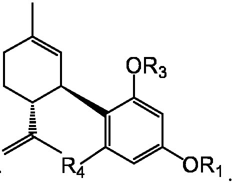 wherein R₁, R₃, and R₄ have the same meanings as defined in the first aspect of the present invention.

In some embodiments of the present invention, the stereoisomer has the following structure:

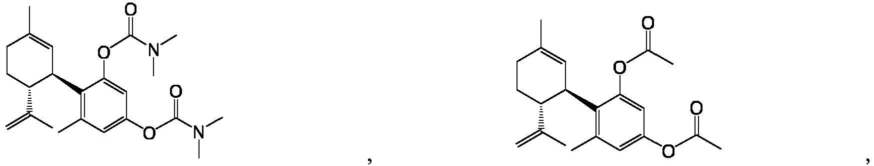

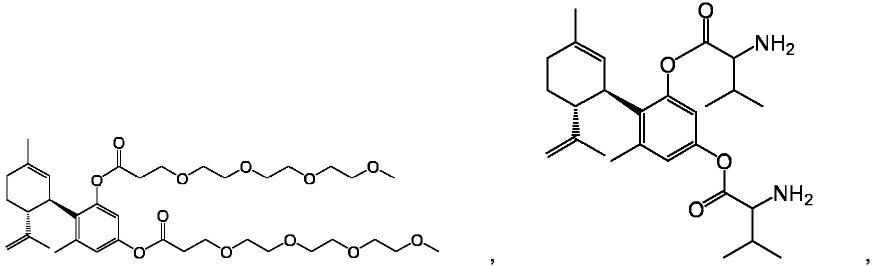

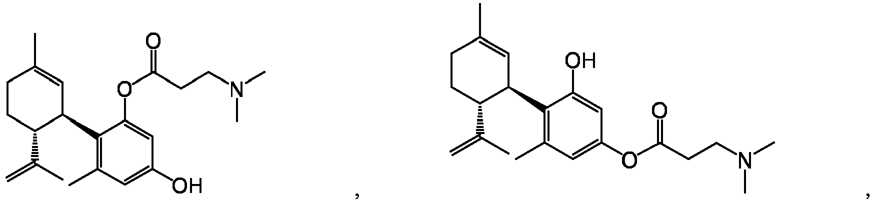

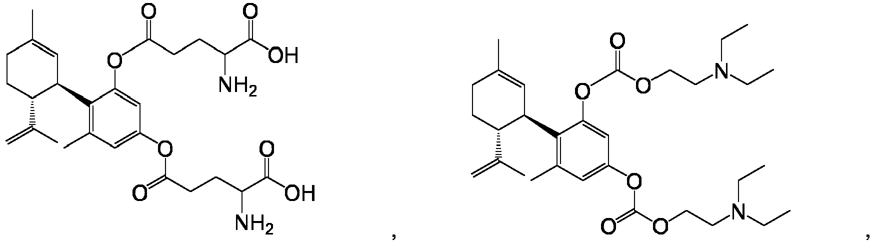

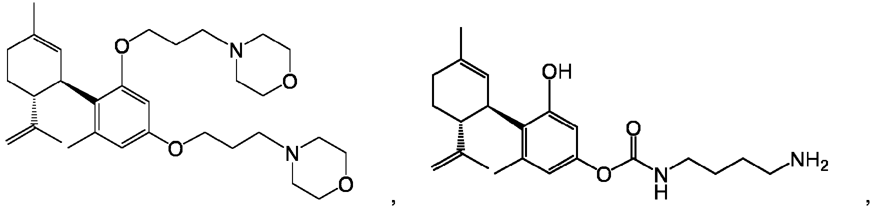

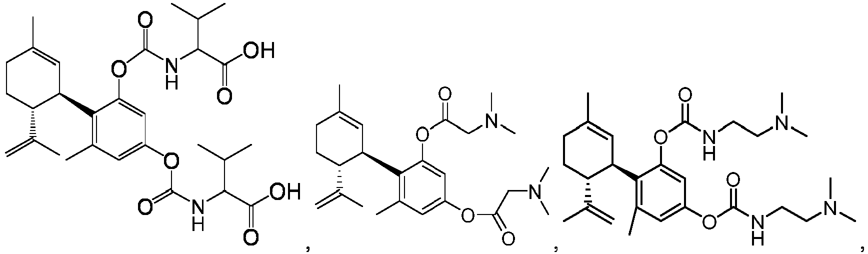

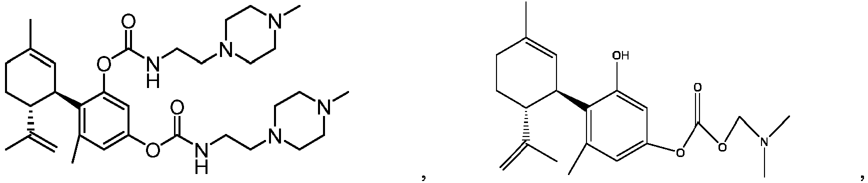

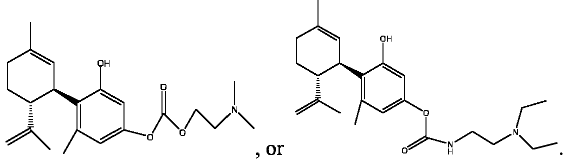

Isotope derivatives as described herein include compounds wherein at least one atom is replaced by an atom having the same atomic number but a different atomic mass, such as stable and radioactive isotopes of hydrogen, carbon, nitrogen, oxygen, phosphorous, sulfur, fluorine, chlorine, bromine, iodine, and the like, specifically ²H (deuterium, D), ³H (tritium, T), ¹¹C, ¹³C, ¹⁴C, ¹⁵N, ¹⁷O, ¹⁸O, and the like, preferably deuterium.

In a third aspect of the present invention, provided is a preparation method of the compound of the first aspect, a stereoisomer of the compound, any composition of the compound and the stereoisomer, any composition of the stereoisomers of the compound, or a pharmaceutically acceptable salt, ester, ether, optical isomer, isotope derivative, solvate (e.g., hydrate), prodrug, metal chelate, crystal form of the foregoing compound (or composition), including a step of reacting 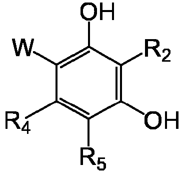 with Z-X₁-R₁₀₁-X₂-R₁₀₂' and/or Z'-Y₁-R₃₀₁-Y₂-R₃₀₂', or by a step of first preparing 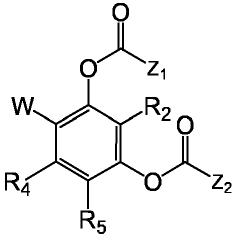 from 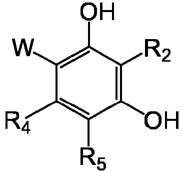 , wherein Z and Z' can be independently selected from H, halogen (e.g., Cl), hydroxyl, OC(O)-alkyl, and the like, Z₁ and Z₂ can be independently selected from halogen (e.g. Cl), hydroxyl, etc.; R₁₀₂' and R₃₀₂' can be R₁₀₂ and R₃₀₂, respectively, or R₁₀₂ and R₃₀₂ in protected form (e.g. protected hydroxyl, amino, where the method further comprising a step of deprotection).

In a fourth aspect of the present invention, provided is a pharmaceutical composition including the compound of the first aspect, a stereoisomer of the compound, any composition of the compound and the stereoisomer, any composition of the stereoisomers of the compound, or a pharmaceutically acceptable salt, ester, ether, optical isomer, isotope derivative, solvate (e.g., hydrate), prodrug, metal chelate, crystal form of the foregoing compound (or composition), and one or more pharmaceutically acceptable excipients.

Specifically, the pharmaceutically acceptable excipient can be selected from one or more of a disintegrant, binder, lubricant, suspending agent, stabilizer, filler, absorption enhancer, surfactant, flavoring agent, antioxidant, preservative, and the like.

Specifically, the pharmaceutical composition can be administered by any suitable administration route, for example, gastrointestinal administration (e.g., oral, sublingual, rectal administration) or parenteral administration (e.g., intravenous, intramuscular, intranasal, intraocular, intracerebral, intravaginal, intraperitoneal, transdermal, subcutaneous, intradermal, respiratory administration, etc.), preferably the gastrointestinal administration route, in particular the oral administration route.

Specifically, the pharmaceutical composition can be in any suitable dosage form, e.g., a gastrointestinal administration dosage form, including, but not limited to, tablet, pill, powder, granule, capsule, lozenge, syrup, liquid, emulsion, suspension, and the like; a parenteral dosage form, e.g. an injectable dosage form such as injection (for example, for subcutaneous injection, intravenous injection, intramuscular injection, intraperitoneal injection), a respiratory administration dosage form such as spray, aerosol, dry powder inhaler, etc., a formulation for dermal administration, such as topical solution, lotion, ointment, plaster, paste, patch, etc., a formulation for mucosal administration such as eye drops, ophthalmic ointment, nasal drops, gargarisms, etc., dosage forms for cavity administration such as a suppository, aerosol, effervescent tablets, drops, drop pills and the like, used for vagina, urethra, nasal cavity, ear canal and the like.

Specifically, the various dosage forms of the above pharmaceutical composition can be prepared according to conventional production methods in the pharmaceutical field. For example, the active ingredient is mixed with one or more pharmaceutically acceptable excipients and then brought into the desired dosage form.

Specifically, in the above pharmaceutical composition, the weight percentage of the compound of the first aspect, a stereoisomer of the compound, any composition of the compound and the stereoisomer, any composition of the stereoisomers of the compound, or a pharmaceutically acceptable salt, ester, ether, optical isomer, isotope derivative, solvate (e.g., hydrate), prodrug, metal chelate, crystal form of the foregoing compound (or composition) can be 0.1-99.5%, such as 0.5%, 1%, 5%, 10%, 20%, 30%, 40%, 50%, 60%, 70%, 80%, 90%, 95%, or 99%.

In a fifth aspect of the present invention, provided is the use of the compound of the first aspect, a stereoisomer of the compound, any composition of the compound and the stereoisomer, any composition of the stereoisomers of the compound, or a pharmaceutically acceptable salt, ester, ether, optical isomer, isotope derivative, solvate (e.g., hydrate), prodrug, metal chelate, crystal form of the foregoing compound (or composition), in preparing a medicament for preventing and/or treating a diseases.

Specifically, the diseases are those diseases that benefit from an increase in the quantity of Tregs for their prevention or treatment. For example, an autoimmune disease, an allergic disease, transplant rejection, a graft-versus-host disease (GvHD), and an inflammatory disease.

Specifically, the autoimmune diseases include, but are not limited to, organ-specific autoimmune disease and systemic autoimmune disease, e.g. achalasia, Addison's disease, adult Still's disease, agammaglobulinemia, alopecia areata, amyloidosis, ankylosing spondylitis, immune-mediated nephropathy (such as glomerulonephritis), antiphospholipid syndrome (APS), autoimmune angioedema, autoimmune dysautonomia, autoimmune encephalomyelitis, autoimmune hepatitis, autoimmune inner ear disease (AIED), autoimmune myocarditis, autoimmune oophoritis, autoimmune orchitis, autoimmune pancreatitis, autoimmune retinopathy, autoimmune urticaria, axonal & neuronal neuropathy (AMAN), Baló disease (also known as concentricsclerosis), Behcet's disease, benign mucosal pemphigoid (also caolled cicatricial pemphigoid), bullous pemphigoid, castleman disease (CD), food allergy related diseases (such as Celiac disease), Chagas disease, chronic inflammatory demyelinating polyneuropathy (CIDP), chronic recurrent multifocal osteomyelitis (CRMO), Churg-Strauss Syndrome (CSS) (also known as allergic Granulomatosis with Polyangiitis (AGPA) or eosinophilic granulomatous polyangitis), Cogan's syndrome, cold agglutinin disease, congenital heart block, Coxsackie myocarditis, CREST syndrome, Crohn's disease, dermatitis herpetiformis, dermatomyositis, Devic's disease (also known as neuromyelitis optica), discoid lupus, Dressler's syndrome, endometriosis, eosinophilic esophagitis (EoE), eosinophilic fasciitis, erythema nodosum, essential mixed cryoglobulinemia, Evans syndrome, fibromyalgia, fibrosing alveolitis, giant cell arteritis, giant cell myocarditis, Goodpasture's syndrome, granulomatosis with polyangiitis (GPA), Graves' disease, Guillain-Barré syndrome, Hashimoto's thyroiditis, hemolytic anemia, Henoch-Schonlein purpura (HSP) (also known as allergic purpura), herpes gestationis or pemphigoid gestationis, hidradenitis Suppurativa, hypogammaglobulinemia, IgG4-related sclerosing disease (also known as IgG4 related systemic diseases, high IgG4 diseases, and IgG4 related diseases), immune thrombocytopenic purpura (ITP), inclusion body myositis (IBM), interstitial cystitis (IC), juvenile arthritis, juvenile myositis, Kawasaki disease, Lambert-Eaton syndrome, leukocytoclastic vasculitis (also known as hypersensitivity vasculitis), lichen planus, lichen sclerosus, ligneous conjunctivitis, linear IgA disease, chronic Lyme disease, Meniere's disease, microscopic polyangiitis, mixed connective tissue disease, Mooren's ulcer, Mucha-Habermann disease (also known as Pityriasis Lichenoides Et Varioliformis Acuta), multifocal motor neuropathy, multiple sclerosis, myasthenia gravis, myositis,narcolepsy, neonatal lupus, neutropenia, ocular cicatricial pemphigoid, optic neuritis, palindromic rheumatism, PANDAS (Pediatric Autoimmune Neuropsychiatric Disorders Associated with Streptococcal Infections), paraneoplastic cerebellar degeneration, paroxysmal nocturnal hemoglobinuria, Parry Romberg syndrome, pars planitis (also known as intermediate uveitis), Parsonage-Turner syndrome (also known as brachial neuritis), pemphigus, peripheral neuropathy, perivenous encephalomyelitis, pernicious anemia, POEMS syndrome, polyarteritis nodosa, polyglandular syndromes type I, II, III, polymyalgia rheumatica, polymyositis, postmyocardial infarction syndrome, postpericardiotomy syndrome, primary biliary cirrhosis, primary sclerosing cholangitis, progesterone dermatitis, psoriasis, psoriatic arthritis, pure red cell aplasia, pyoderma gangrenosum, Raynaud's phenomenon, reactive Arthritis, reflex sympathetic dystrophy (also known as pain syndrome), relapsing polychondritis, restless legs syndrome, retroperitoneal fibrosis, rheumatic fever, rheumatoid arthritis, sarcoidosis, Schmidt syndrome (also known as type 2 polyglandular-autoimmune syndrome), scleritis, scleroderma, Sjögren's syndrome, Sperm & testicular autoimmunity, stiff person syndrome, subacute bacterial endocarditis, Susac's syndrome, sympathetic ophthalmia, systemic lupus erythematosus (SLE), Takayasu's arteritis, temporal arteritis, thyroid eye disease, Tolosa-Hunt syndrome, Type I diabetes (also known as autoimmune diabetes and insulin-dependent diabetes mellitus), ulcerative colitis, undifferentiated connective tissue disease, uveitis, vasculitis, vitiligo, Vogt-Koyanagi-Harada disease. In some embodiments of the present invention, the autoimmune diseases are selected from systemic lupus erythematosus, type I diabetes, rheumatoid arthritis, multiple sclerosis, ankylosing spondylitis, psoriasis, food allergy and related diseases (e.g. Celiac diseases), ulcerative colitis, or Crohn's disease.

Specifically, the immune nephropathy includes, but is not limited to, anti-glomerular basement membrane antibody nephritis, glomerulonephritis, IgA nephropathy, purpuric nephritis, or lupus nephritis.

Specifically, the allergic disease includes, but is not limited to, allergic conjunctivitis, allergic rhinitis, allergic asthma, allergic bronchopulmonary aspergillosis (also known as allergic bronchopulmonary aspergillosis), allergic gastroenteritis, atopic dermatitis, allergic urticaria, allergic angioedema, allergic immediate contact reaction, anaphylactic shock, or food allergy and related diseases (e.g. Celiac disease), etc.

Specifically, for transplant rejection, graft-versus-host disease (GVHD), the transplantation can be cell transplantation (e.g. hematopoietic stem cell transplantation), tissue transplantation (e.g. skin transplantation, bone marrow transplantation, corneal transplantation), organ transplantation (e.g. kidney transplantation, heart transplantation, liver transplantation, pancreas and islet transplantation, parathyroid transplantation, lung transplantation, small intestine transplantation), and in particular organ transplantation.

Specifically, inflammatory disease is a disease with inflammation caused by an immune response, including, but not limited to, interstitial lung disease, inflammatory bowel disease (including chronic colitis, acute colitis, ulcerative colitis (UC), and Crohn's disease (CD)), chronic obstructive pulmonary disease (COPD), acute lung injury, neuroinflammation, sepsis, asthma, allergy, etc.

In an embodiment of the present invention, the use is the use of the compound according to the first aspect, or a pharmaceutically acceptable salt, ester, ether, optical isomer, isotope derivative, stereoisomer, solvate (e.g. hydrate), prodrug, metal chelate, crystal form, in the manufacture of a medicament for the prevention and/or treatment of diseases.

Specifically, the medicament can be administrated by any suitable administration route, for example, gastrointestinal administration (e.g., oral, sublingual, rectal administration) or parenteral administration (e.g., intravenous, intramuscular, intranasal, intraocular, intracerebral, intravaginal, intraperitoneal, transdermal, subcutaneous, intradermal, respiratory administration, etc.), preferably the gastrointestinal administration route, in particular the oral administration route.

In an embodiment of the present invention, the disease is inflammatory bowel disease and the medicament is administered by gastrointestinal administration route, in particular by oral administration route.

Specifically, a dosage of 0.01-5.0 mg/kg, such as 0.01, 0.02, 0.03, 0.033, 0.1, 0.15, 0.2, 0.25, 0.3, 0.35, 0.4, 0.407, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, or 5.0 mg/kg, based on body weight, is administered.

Specifically, the dosing frequency is once daily.

In a specific embodiment of the present invention, the subject is a non-human mammal and is preferably administered at a dosage of 0.4-5.0 mg/kg, e.g., 0.4, 0.407, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, or 5.0 mg/kg.

In a specific embodiment of the present invention, the subject is a human being and is preferably administered at a dosage of 0.033-0.407 mg/kg, e.g., 0.033, 0.1, 0.15, 0.2, 0.25, 0.3, 0.35, 0.4, or 0.407 mg/kg, based on body weight.

In a sixth aspect of the present invention, provided is the use of the compound of the first aspect, a stereoisomer of the compound, any composition of the compound and the stereoisomer, any composition of the stereoisomers of the compound, or a pharmaceutically acceptable salt, ester, ether, optical isomer, isotope derivative, solvate (e.g., hydrate), prodrug, metal chelate, crystal form of the foregoing compound (or composition), in the preparing reagent for inducing or promoting Tregs amplification.

In an embodiment of the present invention, the Tregs amplification is performed *in vivo.*

In another still embodiment of the present invention, the Tregs amplification is performed *in vitro.* In an embodiment of the present invention, for the use described above, the Tregs amplification is performed *in vivo,* and the regent can be administered by any suitable administration route, for example, gastrointestinal administration (e.g., oral, sublingual, rectal administration) or parenteral administration (e.g., intravenous, intramuscular, intranasal, intraocular, intracerebral, intravaginal, intraperitoneal, transdermal, subcutaneous, intradermal, respiratory administration, etc.), preferably the gastrointestinal administration route, in particular the oral administration route.

In a seventh aspect of the present invention, provided is an agent for inducing or promoting Tregs amplification including the compound of the first aspect, a stereoisomer of the compound, any composition of the compound and the stereoisomer, any composition of the stereoisomers of the compound, or a pharmaceutically acceptable salt, ester, ether, optical isomer, isotope derivative, solvate (e.g., hydrate), prodrug, metal chelate, crystal form of the foregoing compound (or composition).

In an embodiment of the present invention, the Tregs amplification is performed *in vivo.*

In another still embodiment of the present invention, the Tregs amplification is performed *in vitro.* Specifically, the agent can be in any suitable form, e.g. solid, liquid.

In an eighth aspect of the present invention, provided is a method for the prevention and/or treatment of diseases, comprising the step of administering to a subject in need thereof the compound according to the first aspect, or a pharmaceutically acceptable salt, ester, ether, optical isomer, isotope derivative, stereoisomer, solvate (e.g., hydrate), prodrug, metal chelate, crystal form, or a pharmaceutical composition including the same.

Specifically, the disease has the definition as described in the fifth aspect of the present invention. Specifically, the subject is a mammal, in particular a human.

Specifically, the compound of the first aspect, a stereoisomer of the compound, any composition of the compound and the stereoisomer, any composition of the stereoisomers of the compound, or a pharmaceutically acceptable salt, ester, ether, optical isomer, isotope derivative, prodrug, solvate of the foregoing compound (or composition) can be used alone or in combination with other types of pharmaceutical formulations and/or treatment methods, in such methods.

Specifically, the administration route can be any suitable route, such as gastrointestinal administration (e.g., oral, sublingual, rectal administration) or parenteral administration (e.g., intravenous, intramuscular, intranasal, intraocular, intracerebral, intravaginal, intraperitoneal, transdermal, subcutaneous, intradermal, respiratory administration, etc.) route.

In an embodiment of the present invention, the method comprises the step of administering to a subject in need thereof a compound of the first aspect, or a pharmaceutically acceptable salt, ester, ether, optical isomer, isotope derivative, stereoisomer, solvate (e.g., hydrate), prodrug, metal chelate, crystal form, or a pharmaceutical composition including the same. The administration route can be any suitable route, for example, gastrointestinal administration (e.g., oral, sublingual, rectal administration) or parenteral administration (e.g., intravenous, intramuscular, intranasal, intraocular, intracerebral, intravaginal, intraperitoneal, transdermal, subcutaneous, intradermal, respiratory administration, etc.), preferably gastrointestinal administration route, in particular oral administration route.

Specifically, a dosage of 0.01-5.0 mg/kg, such as 0.01, 0.02, 0.03, 0.033, 0.1, 0.15, 0.2, 0.25, 0.3, 0.35, 0.4, 0.407, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, or 5.0 mg/kg, based on body weight, is administered.

Specifically, the dosing frequency is once daily.

In a specific embodiment of the present invention, the subject is a non-human mammal and is preferably administered at a dosage of 0.4-5.0 mg/kg, such as 0.4, 0.407, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, or 5.0 mg/kg.

In a specific embodiment of the present invention, the subject is a human being and is preferably administered at a dosage of 0.033-0.407 mg/kg, such as 0.033, 0.1, 0.15, 0.2, 0.25, 0.3, 0.35, 0.4, or 0.407 mg/kg, based on body weight.

In a ninth aspect of the present invention, provided is a method of modulating immune response, comprising the step of administering to a subject in need thereof the compound of the first aspect, a stereoisomer of the compound, any composition of the compound, and the stereoisomer, any composition of the stereoisomers of the compound, or a pharmaceutically acceptable salt, ester, ether, optical isomer, isotope derivative, solvate (e.g., hydrate), prodrug, metal chelate, crystal form of the foregoing compound (or composition), or a pharmaceutical composition including the same.

Specifically, the subject is a mammal, in particular a human.

Specifically, the modulation is a negative modulation of the body's immune response, i.e. inhibition of the body' s immune response.

In an embodiment of the present invention, the method comprises the step of administering to a subject in need thereof the compound of the first aspect, a stereoisomer of the compound, any composition of the compound and the stereoisomer, any composition of the stereoisomers of the compound, or a pharmaceutically acceptable salt, ester, ether, optical isomer, isotope derivative, solvate (e.g., hydrate), prodrug, metal chelate, crystal form of the foregoing compound (or composition), or a pharmaceutical composition including the same. The administration route can be any suitable route, for example, gastrointestinal administration (e.g., oral, sublingual, rectal administration) or parenteral administration (e.g., intravenous, intramuscular, intranasal, intraocular, intracerebral, intravaginal, intraperitoneal, transdermal, subcutaneous, intradermal, respiratory administration, etc.), preferably gastrointestinal administration route, in particular oral administration route.

In a tenth aspect of the present invention, provided is a method for Tregs amplification, including the step of administering to a subject in need thereof the compound of the first aspect, a stereoisomer of the compound, any composition of the compound, and the stereoisomer, any composition of the stereoisomers of the compound, or a pharmaceutically acceptable salt, ester, ether, optical isomer, isotope derivative, solvate (e.g., hydrate), prodrug, metal chelate, crystal form of the foregoing compound (or composition), or a pharmaceutical composition including the same.

In an embodiment of the present invention, the method is performed *in vivo.*

In another embodiment of the present invention, the method is performed *in vitro.*

In some embodiments of the present invention, the method is performed *in vivo,* comprising the step of administering to a subject in need thereof the compound of the first aspect, a stereoisomer of the compound, any composition of the compound and the stereoisomer, any composition of the stereoisomers of the compound, or a pharmaceutically acceptable salt, ester, ether, optical isomer, isotope derivative, solvate (e.g., hydrate), prodrug, metal chelate, crystal form of the foregoing compound (or composition), or a pharmaceutical composition including the same. The administration route can be any suitable route, for example, gastrointestinal administration (e.g., oral, sublingual, rectal administration) or parenteral administration (e.g., intravenous, intramuscular, intranasal, intraocular, intracerebral, intravaginal, intraperitoneal, transdermal, subcutaneous, intradermal, respiratory administration, etc.), preferably gastrointestinal administration route, in particular oral administration route.

In some embodiments of the present invention, the method is performed *in vitro,* comprising the step of culturing Tregs *in vitro* with a medium including the compound of the first aspect, a stereoisomer of the compound, any composition of the compound and the stereoisomer, any composition of the stereoisomers of the compound, or a pharmaceutically acceptable salt, ester, ether, optical isomer, isotope derivative, solvate (e.g., hydrate), prodrug, metal chelate, crystal form of the foregoing compound (or composition).

In an eleventh aspect of the present invention, provided is a population of Tregs prepared by a method (*in vitro*) as described in the tenth aspect.

Specifically, the Tregs population can be used to modulate (inhibit) an immune response in a subject, to prevent or treat a disease, such as an autoimmune disease, an allergic disease, transplant rejection, graft-versus-host disease (GvHD), an inflammatory disease (in particular as described in the fifth aspect of the present invention).

It is found by means of experiments that O-1602 can effectively promote the proliferation of Tregs, increase the quantity of Tregs, and is expected to contribute to the prevention or treatment of some diseases by increasing the quantity of Tregs, for example, an autoimmune disease, an allergic disease, transplant rejection, a graft-versus-host disease (GvHD), and an inflammatory disease. In addition, a series of new compounds are prepared, and can also effectively promote the proliferation of Tregs, increase the quantity of Tregs, have good physiochemical properties such as solubility and stability, have diversified expected administration routes, and have very good application and research value.

### Brief Description of the Drawings

FIG. 1: alleviation of the moderate to mild PSORIASIS induced in mice by an O-1602 derivative.
FIG. 2: effects of different compounds on body weight change in the treatment of TNBS-induced acute enteritis, where TOFACITINIB is tofacitinib.
FIG. 3: scores after treatment of TNBS-induced acute enteritis for different compounds, where TOFACITINIB is tofacitinib.
FIG. 4: effects of different compounds on colon length after TNBS-induced acute enteritis treatment dissection.
FIG. 5: effects of different compounds on colon length and appearance after dissection for the treatment of TNBS-induced acute enteritis, wherein from left to right are compounds 1 to 13, Control, and Tofacitinib.

### Detailed Description of the Invention

Unless otherwise defined, all scientific and technical terms used in the present invention have the same meanings as those commonly understood by those skilled in the art to which the present invention relates.

The term "alkyl" refers to a straight or branched hydrocarbon chain radical that does not contain unsaturated bonds and is connected to other parts of the molecule by a single bond. Typical alkyl groups may contain from 1 to 20 (e.g., 1, 2, 3, 4, 5, 6, 7, 8, 9, 10, 12, 14, 16, 18, 10) carbon atoms, in particular from 1 to 10 carbon atoms, preferably from 1 to 6 carbon atoms, such as methyl, ethyl, n-propyl, isopropyl, n-butyl, isobutyl, tert-butyl, n-pentyl, isopentyl, neopentyl, tert-pentyl, n-hexyl, isohexyl and the like. If an alkyl group is substituted by cycloalkyl, it corresponds to "cycloalkylalkyl", e.g., cyclopropylmethyl, cyclopropylethyl, cyclobutylmethyl, cyclopentylmethyl, cyclohexylmethyl, and the like. If an alkyl group is substituted by an aryl group, it corresponds to an "aralkyl" group, such as benzyl, benzhydryl, or phenethyl. If an alkyl group is substituted with a heterocyclyl group, it is accordingly a "heterocyclylalkyl".

The term "alkylene" refers to a hydrocarbon radical (a divalent alkyl group) derived from the loss of two hydrogen atoms from an alkane molecule, which may be straight or branched and which is attached to the rest of the molecule by a single bond. Typical alkylene groups herein have 1 to 10 (e.g., 1, 2, 3, 4, 5, 6, 7, 8, 9, 10) carbon atoms, preferably 1 to 6 carbon atoms, such as methylene (-CH₂-), ethylene, propylene, butylene, and the like.

The term "cycloalkyl" refers to alicyclic hydrocarbons, e.g., containing 1 to 4 monocyclic and/or fused rings, containing 3 to 18 carbon atoms, preferably 3 to 10 (e.g., 3, 4, 5, 6, 7, 8, 9, 10) carbon atoms, such as cyclopropyl, cyclobutyl, cyclopentyl, cyclohexyl, adamantyl, and the like.

The term "alkoxy" refers to a substituent formed by substitution of a hydrogen in a hydroxyl group with an alkyl group, e.g., an alkoxy group containing from 1 to 10 carbon atoms, e.g., methoxy, ethoxy, propoxy, butoxy, etc.

The term "alkylamino" refers to a substituent formed by substitution of one or two hydrogens of an amino group (-NH₂) with an alkyl group, such as an alkylamino group containing 1-10 carbon atoms, for example

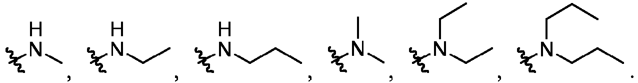

The term "halogen" means fluorine, chlorine, bromine, or iodine.

The term "haloalkyl" refers to a group formed by substitution of one or more hydrogens of an alkyl group with a halogen atom (e.g., fluorine, chlorine, bromine, or iodine), such as -CHF₂, -CH₂F, -CF₃, -CH₂-CF₃, -CH₂CH₂-CF₃, -CH₂CH₂CH₂-CF₃.

The term "aryl" refers to monocyclic or polycyclic radicals, including polycyclic radicals containing a monoaryl group and/or a fused aryl group, e.g., containing from 1-3 monocyclic or fused rings and 6-18 (e.g., 6, 8, 10, 12, 14, 16, 18) carbon ring atoms. As used herein, C₆-C₁₂ aryl refers to an aryl group containing 6-12 carbon ring atoms, e.g., phenyl, naphthyl, biphenyl, indenyl, and the like.

The term "heterocyclyl" refers to a 3- to 18-membered cyclic group including 2-17 carbon atoms and 1-10 heteroatoms selected from N, O, or S atoms. The heterocyclyl can be a monocyclic, bicyclic, tricyclic, or tetracyclic ring system, and the polycyclic ring system can include fused (two rings share two ring atoms), spiro (two rings share one ring atom), or bridged (two rings share more than three ring atoms) ring systems (excluding bicyclic cases). The heterocyclyl can be partially saturated (heteroaromatic) or fully saturated (heterocyclic alkyl). Suitable heteroaryl groups in the compounds of the present invention contain 1, 2 or 3 kinds of heteroatoms selected from N, O or S atoms and include, for example, coumarin, including 8-coumarin, quinolinyl, including 8-quinolinyl, isoquinolinyl, pyridinyl, pyrazinyl, pyrazolyl, pyrimidinyl, furanyl, pyrrolyl, thienyl, thiazolyl, isothiazolyl, triazolyl, tetrazolyl, isoxazolyl, oxazolyl, imidazolyl, indolyl, isoindolyl, indazolyl, indolizinyl, phthalazinyl, pteridinyl, purinyl, oxadiazolyl, thiadiazolyl, furazanyl, pyridazinyl, triazinyl, cinnolinyl, benzimidazolyl, benzofuranyl, benzofurazanyl, benzothienyl, benzothiazolyl, benzoxazolyl, quinazolinyl, quinoxalinyl, naphthyridinyl, furopyridinyl, pyridooxazepinyl, pyridoazepinyl, pyridodiazepinyl, pyridooxepanyl, pyridocycloheptanyl, benzoxazepinyl, benzoazepinyl, benzodiazepinyl, benzooxepanyl. Suitable heterocycloalkyl groups in the compounds of the present invention contain 1, 2, or 3 kinds of heteroatoms selected from N, O, or S atoms and include, for example, pyrrolidinyl, tetrahydrofuranyl, dihydrofuranyl, tetrahydrothienyl, tetrahydrothiopyranyl, piperidinyl, morpholinyl, thiomorpholinyl, oxathianyl, piperazinyl, azetidinyl, oxetanyl, thietanyl, azepanyl, oxiranyl, thietanyl, azepinyl, oxepinyl, oxazepinyl, diazepinyl, triazepinyl, 1,2,3,6-tetrahydropyridinyl, 2-pyrrolinyl, 3-pyrrolinyl, indolinyl, 2H-pyranyl, 4H-pyranyl, dioxanyl, 1,3-dioxolanyl, pyrazolinyl, dithianyl, dithiolanyl, dihydropyranyl, dihydrothienyl, pyrazolidinyl, imidazolinyl, imidazolidinyl, 3-azabicyclo [3.1.0] hexyl, 3-azabicyclo [4.1.0] heptyl, 3H-indolyl, and quinolizinyl.

The term "amino acid" refers to an organic compound containing a basic amino group and an acidic carboxyl group. Amino acids can be classified as α-, β-, γ-, ω-amino acids, in particular α-amino acids, depending on the position at which the amino group is attached to the carbon chain. The term "amino acid residue" refers to the structural part obtained by the absence of hydrogen on the amino group and a hydroxyl group on the carboxyl group of an amino acid. The term "oligopeptide" refers to a compound formed by the condensation of 2-10 (e.g., 2, 3, 4, 5, 6, 7, 8, 9, 10, in particular 2-6) amino acids with each other. The term "polypeptide" refers to a compound formed by the condensation of 10-50 (e.g., 10, 15, 20, 25, 30, 35, 40, 45, 50) amino acids with each other. In the present invention, the amino acid in the amino acids and oligopeptides or polypeptides can be selected from alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, valine, citrulline, ornithine.

The term "monosaccharide" refers to a molecule that can no longer be simply hydrolyzed to a smaller saccharide. According to the different positions of carbonyl groups, the monosaccharide can be divided into two categories: aldose and ketose. The monosaccharide can also be classified into triose, tetrose, pentose, hexose, and heptose based on the number of carbon atoms, such as glyceraldehyde (a three-carbon sugar); erythrose (a four-carbon sugar); arabinose, ribose, xylose, and lyxose (five-carbon sugars); and glucose, mannose, fructose, and galactose (six-carbon sugars). In the present invention, monosaccharide also includes N-acetylgalactosamine, N-acetylglucosamine, fucose, N-acetylneuraminic acid. The term "oligosaccharide" refers to a compound containing 2-10 (e.g., 2, 3, 4, 5, 6, 7, 8, 9, or 10, in particular, 2-6) glycosidic bonds formed by the dehydrative condensation between a glycosidic hydroxyl group of one monosaccharide and a hydroxyl group of another monosaccharide. The term "polysaccharide" refers to a polymeric carbohydrate composed of glycosidically bonded sugar chains consisting of at least 10 monosaccharides. Polysaccharides formed from identical monosaccharides are termed homopolysaccharides, such as starch, cellulose, and glycogen; those composed of different monosaccharides are called heteropolysaccharides, such as hyaluronic acid and chondroitin sulfate. In an embodiment of the present invention, the monosaccharide residue is a glucuronic acid residue. In an embodiment of the present invention, the polysaccharide residue is a hyaluronic acid residue.

The term "pharmaceutically acceptable salt" includes acid addition salts and base addition salts.

The term "acid addition salt" includes, but is not limited to, salts derived from inorganic acids such as hydrochloric, nitric, phosphoric, sulfuric, hydrobromic, hydroiodic, and phosphonic acids, as well as salts derived from organic acids such as aliphatic mono- and dicarboxylic acids, phenyl-substituted alkanoic acids, hydroxyl alkanoic acids, alkanedioic acids, aromatic acids, aliphatic and aromatic sulfonic acids. Thus, these salts include, but are not limited to, sulfate, pyrosulfate, bisulfate, sulfite, bisulfite, nitrate, phosphate, monohydrogen phosphate, dihydrogen phosphate, metaphosphate, pyrophosphate, hydrochloride, hydrobromate, iodate, acetate, propionate, octanoate, isobutyrate, oxalate, malonate, succinate, suberate, sebacate, fumarate, maleate, mandelate, benzoate, chlorobenzoate, methylbenzoate, dinitrobenzoate, phthalate, benzenesulfonate, tosylate, phenylacetate, citrate, lactate, maleate, tartrate and methanesulfonate; also include salts of amino acids such as arginine salt, gluconate, as galacturonate. The acid addition salts may be prepared by contacting the base in a free form with a sufficient amount of the desired acid to produce the salt in the conventional manner. The base in the free form may be regenerated by contacting the salt form with a base and isolating the free base in the conventional manner.

The term "base addition salt" refers to salts formed with metals or amines, such as the hydroxides of alkali and alkaline earth metals, or with organic amines. Examples of metals used as cations include but are not limited to sodium, potassium, magnesium, and calcium. Examples of suitable amines include but are not limited to, N, N'-dibenzylethylenediamine, chloroprocaine, choline, diethanolamine, ethylenediamine (ethane-1,2-diamine), N-methylglucamine, and procaine. The base addition salts may be prepared by contacting the acid in a free form with a sufficient amount of the desired base to produce the salt in the conventional manner. The acid in the free form may be regenerated by contacting the salt form with an acid and isolating the free acid in the conventional manner.

The term "stereoisomer" includes enantiomer, diastereomer, and geometric isomer forms. Some of the compounds of the present invention have a cyclic hydrocarbon group which may be substituted at more than one carbon atom, in which case all geometrical forms thereof, both cis and trans, and mixtures thereof, are within the scope of the present invention.

The term "solvate" refers to the physical combination of the compounds of the present invention with one or more solvent molecules. This physical association includes various degrees of ionic and covalent bonding, including hydrogen bonding. In some cases, the solvate may be isolated, such as when one or more solvent molecules are doped into the lattice of the crystalline solid.

Solvate encompasses both solution-phase and an isolable solvate. Representative solvates include hydrates, ethanolates, and methanolates.

The term "prodrug" refers to forms of the compounds of Formula I that are suitable for administration to a patient without undue toxicity, irritation, hypersensitivity, and the like, and that are effective for their intended use, including acetal, ester, and zwitterion forms. The prodrug is converted *in vivo,* for example, by hydrolysis in blood, to yield the parent compound.

The term "metal chelate", also referred to as a metal chelate compound, refers to a compound that becomes stable and of larger molecular weight by the inclusion of a metal ion into the interior of a chelating agent through strong binding of the chelating agent molecule to the metal ion.

The term "crystal form" refers to a crystalline substance that has an ordered arrangement in three-dimensional space due to the molecules, atoms, and ions that make up the substance, with a periodic arrangement rule.

The terms "patient" or "subject" and the like are used interchangeably herein and refer to any animal or cell thereof treated according to the methods described herein, whether *in vitro* or *in situ.* Specifically, the aforementioned animals include mammals, e.g., rats, mice, guinea pigs, rabbits, cats, dogs, cattle, pigs, sheep, horses, monkeys or humans, in particular humans.

The term "treatment" refers to preventing, curing, reversing, attenuating, alleviating, minimizing, inhibiting, arresting, and/or stopping one or more clinical symptoms of a disease after the onset of the disease.

The term "preventing" refers to avoiding, minimizing, or making difficult the onset or progression of a disease by treatment prior to the onset of the disease.

The terms "Treg", "regulatory T cell" and "regulatory T cell" have the same meaning and are used interchangeably, and are a subset of T cells that control autoimmunity *in vivo,* and are also referred to earlier as suppressor T cell because they have the effect of inhibiting the immune response.

The term "Treg-related disease" primarily refers to diseases associated with abnormal or absent regulatory T cell function, in particular diseases of which the prevention or treatment is contributed by increasing the quantity of Tregs, for example, an autoimmune disease, an allergic disease, transplant rejection, a graft-versus-host disease (GvHD), and an inflammatory disease. The term "autoimmune disease" refers to a disease caused by the body's immune response to self-antigens resulting in self-tissue damage, which can be divided into organ-specific autoimmune diseases (mainly affecting a single organ) and systemic autoimmune diseases (affecting multiple tissues or organs throughout the body). The American Autoimmune Related Diseases Association lists a more comprehensive list of autoimmune diseases, including (in alphabetical order of disease name, not related to morbidity): achalasia, Addison's disease, adult Still's disease, agammaglobulinemia, alopecia areata, amyloidosis, ankylosing spondylitis, immune-mediated nephropathy (such as glomerulonephritis), antiphospholipid syndrome (APS), autoimmune angioedema, autoimmune dysautonomia, autoimmune encephalomyelitis, autoimmune hepatitis, autoimmune inner ear disease (AIED), autoimmune myocarditis, autoimmune oophoritis, autoimmune orchitis, autoimmune pancreatitis, autoimmune retinopathy, autoimmune urticaria, axonal & neuronal neuropathy (AMAN), Baló disease (also known as cncentricsclerosis), Behcet's disease, benign mucosal pemphigoid (also caolled cicatricial pemphigoid), bullous pemphigoid, castleman disease (CD), food allergy related diseases (such as Celiac disease), Chagas disease, chronic inflammatory demyelinating polyneuropathy (CIDP), chronic recurrent multifocal osteomyelitis (CRMO), Churg-Strauss Syndrome (CSS) (also known as allergic Granulomatosis with Polyangiitis (AGPA) or eosinophilic granulomatous polyangitis), Cogan's syndrome, cold agglutinin disease, congenital heart block, Coxsackie myocarditis, CREST syndrome, Crohn's disease, dermatitis herpetiformis, dermatomyositis, Devic's disease (also known as neuromyelitis optica), discoid lupus, Dressler's syndrome, endometriosis, eosinophilic esophagitis (EoE), eosinophilic fasciitis, erythema nodosum, essential mixed cryoglobulinemia, Evans syndrome, fibromyalgia, fibrosing alveolitis, giant cell arteritis, giant cell myocarditis, Goodpasture's syndrome, granulomatosis with polyangiitis (GPA), Graves' disease, Guillain-Barré syndrome, Hashimoto's thyroiditis, hemolytic anemia, Henoch-Schonlein purpura (HSP) (also known as allergic purpura), herpes gestationis or pemphigoid gestationis, hidradenitis Suppurativa, hypogammaglobulinemia, IgG4-related sclerosing disease (also known as IgG4 related systemic diseases, high IgG4 diseases, and IgG4 related diseases), immune thrombocytopenic purpura (ITP), inclusion body myositis (IBM), interstitial cystitis (IC), juvenile arthritis, juvenile myositis, Kawasaki disease, Lambert-Eaton syndrome, leukocytoclastic vasculitis (also known as hypersensitivity vasculitis), lichen planus, lichen sclerosus, ligneous conjunctivitis, linear IgA disease, chronic Lyme disease, Meniere's disease, microscopic polyangiitis, mixed connective tissue disease, Mooren's ulcer, Mucha-Habermann disease (also known as Pityriasis Lichenoides Et Varioliformis Acuta), multifocal motor neuropathy, multiple sclerosis, myasthenia gravis, myositis, narcolepsy, neonatal lupus, neutropenia, ocular cicatricial pemphigoid, optic neuritis, palindromic rheumatism, PANDAS (Pediatric Autoimmune Neuropsychiatric Disorders Associated with Streptococcal Infections), paraneoplastic cerebellar degeneration, paroxysmal nocturnal hemoglobinuria, Parry Romberg syndrome, pars planitis (also known as intermediate uveitis), Parsonage-Turner syndrome (also known as brachial neuritis), pemphigus, peripheral neuropathy, perivenous encephalomyelitis, pernicious anemia, POEMS syndrome, polyarteritis nodosa, polyglandular syndromes type I, II, III, polymyalgia rheumatica, polymyositis, postmyocardial infarction syndrome, postpericardiotomy syndrome, primary biliary cirrhosis, primary sclerosing cholangitis, progesterone dermatitis, psoriasis, psoriatic arthritis, pure red cell aplasia, pyoderma gangrenosum, Raynaud's phenomenon, reactive Arthritis, reflex sympathetic dystrophy (also known as pain syndrome), relapsing polychondritis, restless legs syndrome, retroperitoneal fibrosis, rheumatic fever, rheumatoid arthritis, sarcoidosis, Schmidt syndrome (also known as type 2 polyglandular-autoimmune syndrome), scleritis, scleroderma, Sjögren's syndrome, Sperm & testicular autoimmunity, stiff person syndrome, subacute bacterial endocarditis, Susac's syndrome, sympathetic ophthalmia, systemic lupus erythematosus (SLE), Takayasu's arteritis, temporal arteritis, thyroid eye disease, Tolosa-Hunt syndrome, Type I diabetes (also known as autoimmune diabetes and insulin-dependent diabetes mellitus), ulcerative colitis, undifferentiated connective tissue disease, uveitis, vasculitis, vitiligo, Vogt-Koyanagi-Harada disease. Among the common autoimmune diseases usually include systemic lupus erythematosus, type I diabetes, rheumatoid arthritis, multiple sclerosis, ankylosing spondylitis, psoriasis, food allergies, and related diseases (e.g., Celiac diseases), ulcerative colitis, Crohn's disease.

The term "allergic diseases" refers to an abnormal immune response of the body caused by an allergen, resulting in tissue inflammation or organ dysfunction. Skin, respiratory tract and digestive tract involvement are the most common in allergic diseases. Allergic diseases in the broad sense include all diseases caused by hypersensitivity mechanisms, while hypersensitivity in the narrow sense often refers to type I allergy mediated by IgE. Diseases caused by type I allergy include: eye: allergic conjunctivitis, nasal: allergic rhinitis, trachea and lung: allergic asthma, allergic bronchopulmonary aspergillosis (also known as allergic bronchopulmonary aspergillosis), etc., digestive tract: allergic gastroenteritis, skin: atopic dermatitis, allergic urticaria, allergic angioedema, allergic immediate contact reaction, etc., severe systemic reactions: anaphylaxis, anaphylactic shock. Food allergy can manifest in various ways, with rash being the most common, frequently occurring perioral erythema, also commonly seen on the trunk, accompanied by itching and desquamation, and may have pigmentation, as well as nausea, diarrhea, and abdominal pain. The term "allergic reaction" is also referred to as hypersensitivity, i.e. abnormal, excessive immune response. That is, under certain conditions, the body interacts with antigenic substances to produce sensitized lymphocytes or specific antibodies. If combined with re-entering antigens, it can lead to pathological immune responses that disrupt physiological functions and tissue damage. According to the speed of reaction occurrence, it is divided into immediate-type hypersensitivity and delayed-type hypersensitivity. Gell and Coombs classified hypersensitivity reactions into four types: type I hypersensitivity, also known as immediate-type hypersensitivity, is characterized by rapid onset and resolution, and common examples of Type I hypersensitivity include anaphylactic shock, drug-induced skin eruptions, food-induced allergic gastroenteritis, and pollen-or dust-induced allergic rhinitis. Type II hypersensitivity, also known as cytotoxic hypersensitivity or cytolytic hypersensitivity, involves pathological reactions primarily mediated by cell lysis or tissue damage, which occurs when IgG or IgM antibodies bind to specific antigens on target cell surfaces, with subsequent participation of complement, phagocytes, and NK cells. Examples include incompatible blood transfusion reactions, neonatal hemolytic disease, and drug-induced hemolytic anemia, all of which belong to Type II hypersensitivity. Type III hypersensitivity, is also known as immune complex-mediated hypersensitivity or vasculitic hypersensitivity. It is caused by the deposition of medium-sized soluble antigen antibody complexes onto the local or systemic capillary basement membrane, activating complement and involving platelets, basophil granulocyte, and neutrophils, leading to inflammatory reactions and tissue damage characterized by congestion, edema, local necrosis, and neutrophil infiltration, such as exogenous asthma. Type IV hypersensitivity reaction, also known as delayed hypersensitivity reaction, is mediated by T cells, such as organ transplant rejection and post-vaccination encephalomyelitis.

The term "transplantation" refers to the transplantation of autologous or allogeneic cells, tissues, and organs into a specific part of the body to restore the anatomical structure and function of the damaged organ or tissue. Blood transfusion was the earliest use of cell transplantation, followed by tissue transplantation such as skin, mucosa, omentum, bone marrow, fat, fascia, muscle, tendon, blood vessel, nerve, bone, and cartilage transplantation. Organ transplantation mainly includes the kidney, heart, liver, pancreas and pancreatic islet, parathyroid gland, lung, small intestine, and so on.

The term "transplant rejection reaction" refers to the immune system of a recipient who undergoes allogeneic tissue or organ transplantation, where the foreign tissue or organ transplant is recognized as an "alien component" by the recipient's immune system, which initiates an immune response to attack, destroy, and eliminate the transplant.

The term "graft-versus-host disease (GVHD)" refers to the damage of various organs of the body caused by transplanted cells against recipient cells after transplantation. According to the time when graft-versus-host disease (GVHD) occurs after transplantation, it can be divided into hyperacute GVHD (occurring within 10 days after transplantation), acute GVHD (occurring within 3 months after transplantation), and chronic GVHD (occurring after 3 months of transplantation).

The term "inflammation" is the body's defensive response to stimuli, manifested as redness, swelling, heat, pain, dysfunction, and the like; it may be infectious inflammation due to infection, or it may be non-infectious inflammation not due to infection, such as inflammation due to immune response (e.g., various types of hypersensitivity, inflammation due to some autoimmune diseases). The term "inflammatory diseases" refers to diseases with inflammation, in particular with inflammation caused by an immune response, such as: interstitial lung disease, inflammatory bowel disease (an idiopathic inflammatory bowel disease involving the ileum, rectum, and colon, including chronic colitis, acute colitis, ulcerative colitis (UC), and Crohn's disease (CD)), chronic obstructive pulmonary disease (COPD), acute lung injury, neuroinflammation, sepsis, asthma, allergy, and the like.

The disclosures of the various publications, patents, and published patent specifications cited herein are hereby incorporated by reference in their entirety.

The technical solutions of the present invention will be described clearly and completely in conjunction with the examples of the present invention. Obviously, the described examples are only a part of the embodiments of the present invention, rather than all the embodiments. Based on the embodiments of the present invention, all other embodiments obtained by a person of ordinary skill in the art without inventive effort fall within the scope of the present invention.

The chemicals used in the experiments described in the following examples included: 3,5-dihydroxytoluene, (1S, 4R)-1-methyl-4-(1-propen-2-yl)-2-cyclohexenol, p-toluenesulfonic acid, *N, N*'-dimethylformyl chloride, sodium hydride, acetyl chloride, 4,7,10,13-tetraoxatetradecanoic acid, Boc-DL-valine, 3-*N, N*'-dimethylaminopropionic acid, N-tert-butoxycarbonyl-L-glutamate-1-tert butyl ester, triphosgene, diethylaminoethanol, 3-(4-morpholino)-1-propanol, 1,4-butanediamine, D, L-tert-leucine, *N, N*'-dicyclohexylcarbodiimide, 4-dimethylaminopyridine, dichloromethane, diethyl ether, petroleum ether, ethyl acetate, methanol, acetonitrile, n-hexane. All of the above chemicals were chemically pure.

The synthesis examples are as follows.

### 1. Synthesis of compound 1

### Synthesis route

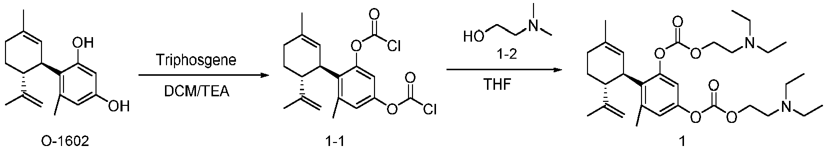

### Operating procedures

Triphosgene (1.15 g, 3.88 mmol) was dissolved in anhydrous dichloromethane (10 mL). The temperature was lowered to -10°C. In another reaction flask, compound 0-1602 (1 g, 3.88 mmol) and triethylamine (1.18 g, 11.64 mmol) were dissolved in dichloromethane (5 mL), and slowly added dropwise to the triphosgene solution. The reaction was carried out at -10°C for 2 hours, and then allowed to warm to room temperature and carried out overnight. The dichloromethane was concentrated under reduced pressure to obtain the crude product of compound 1-1. Dry tetrahydrofuran was added to the residue to precipitate a solid, which was removed by filtration. The filtrate was transferred to a reaction flask and compound 1-2 (2.27g, 19.4mmol) was added.

The mixture was stirred at room temperature overnight and concentrated under reduced pressure to give a pale yellow oil, which was purified on a reverse-phase column with the filler as follows: 2.5 µm ostade-cylsilane (ODS), eluent: 0.25% aqueous trifluoroacetic acid/acetonitrile = 4: 6. The product solution was collected and directly lyophilized to give compound 1 as an oil (400 mg, yield: 19%)
The product was identified by LCMS, MS=545 (M+1)
¹HNMR:(400MHz,CDCl₃),δ7.02(d,2H),5.01(d,1H),4.5(d,2H),4.4(s,2H),3.52-3.76(m,2H),3.11-3.2 6(m,10H),2.77-2.89(m,2H),1.65-1.75(m,3H),1.69(t.2H),1.61(s,2H),1.54(s,3H),1.18-1.22(t,12H), 1.13-1.80(m,3H)

### 2. Synthesis of compound 2

### Synthesis route

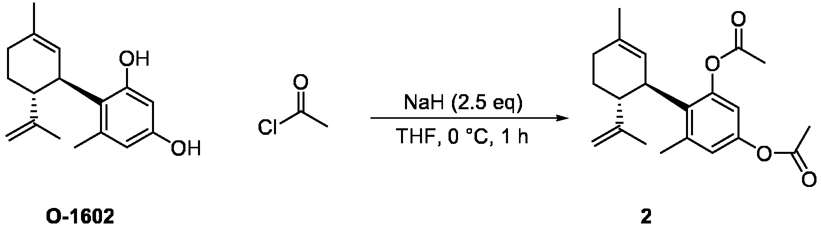

### Operating procedures

0-1602 (645 mg, 2.5 mmol) was dissolved in 25 mL of anhydrous THF. NaH (250 mg, 6.25 mmol) was added at -5°C under a nitrogen atmosphere. After the addition was completed, the mixture was continuously stirred at -5°C for 30 min. The temperature was maintained constant, and then, acetyl chloride (488 mg, 6.25 mmol, dissolved in 10 mL of anhydrous THF) was added dropwise to the reaction solution within 5 min. After the addition was completed, the reaction was continued at -5°C for 1 hour. The reaction was quenched with 10 mL of saturated ammonium chloride solution. The mixture was extracted with ethyl acetate (50 mL x 2). The organic layers were combined, washed with saturated brine (50 mL x 1), dried over anhydrous sodium sulfate, filtered by suction, and concentrated under reduced pressure (40°C water temperature) to remove the solvent to obtain the crude product, which was purified by normal phase column chromatography, with the filler as follows: 200-300 mesh silica gel (A: petroleum ether; B: ethyl acetate; 0% A-30% B, 60 min; 7% of B in A), followed by reverse phase column chromatography, with the filler as follows: 2.5 µm ODS (A: 5 mmol/L aqueous ammonium bicarbonate solution; B: acetonitrile; 0% B-100% B, 45 min; ~80% of B in A), followed by a normal phase column chromatography (A: petroleum ether; B: ethyl acetate; 0% A-30% B, 60 min; 5% of B in A) to give compound 2 as a pale yellow oil (330 mg, 0.965 mmol, yield: 38.6%; purity: 97.28%, 254 nm).
LCMS-Product2: [M+1]⁺= 343, [M+18]⁺= 360 and [M+Na]⁺= 365;
¹H-NMR -Product 2: (400 MHz, CDCl3) δ 6.71 (s, 1H), 6.67 (d, J = 2.0 Hz, 1H), 5.25 (d, J = 24 Hz, 1H), 4.60 (d, J = 20.0 Hz, 1H), 4.49 (d, J = 32 Hz, 1H), 3.70 - 3.56 (m, 1H), 2.74 (t, J = 8.0 Hz, 1H), 2.26 (s, 3H), 2.24 (s, 3H), 2.14 (s, 3H), 2.06 - 2.02 (m, 1H), 1.81 - 1.71 (m, 2H), 1.68 (m, 3H), 1.60 (m, 1H), 1.54 (s, 3H).

### 3. Synthesis of compound 3

### Synthesis route

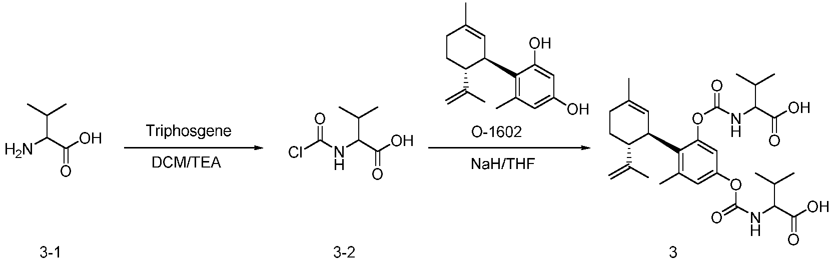

### Operating procedures

First step: triphosgene (2.54 g, 8.55 mmol) was dissolved in anhydrous dichloromethane (10 mL), and the temperature was lowered to -10°C. In another reaction flask, compound 3-1 (1 g, 8.55 mmol) and triethylamine (2.59 g, 25.65 mmol) were dissolved in dichloromethane (5 mL), and slowly added dropwise to the triphosgene solution. The reaction was carried out at -10°C for 2 hours, and then allowed to warm to room temperature and carried out overnight. The dichloromethane in the system was concentrated under reduced pressure. Dry tetrahydrofuran was added to the residue, and a solid precipitated, which was removed by filtration. The filtrate was concentrated under reduced pressure to give the crude product of compound 3-2, which was used for the next step synthesis without purification.

Second step: compound 3-2 (528 mg, 2.05 mmol) was dissolved in tetrahydrofuran, cooled to 0°C, and added with NaH (205 mg, 5.13 mmol) for reaction. The reaction was carried out for 30 minutes. Compound O-1602 (1.1 g, 6.15 mmol) was added and reacted at room temperature overnight. The reaction was quenched with a saturated ammonium chloride solution. The reaction mixture was extracted with ethyl acetate. The organic phase was dried over anhydrous sodium sulfate and filtered. The filtrate was concentrated under reduced pressure to give the crude product, which was purified by reverse phase column chromatography with the filler as follows: 2.5 µm ODS, and the eluent was 0.25% aqueous trifluoroacetic acid/acetonitrile = 3: 7. The product solution was collected and directly lyophilized to give compound 3 as a yellow solid (180 mg, yield: 16%). The structure was confirmed by LCMS and HNMR.
LCMS: MS=545 [M+1]⁺
¹HNMR: (400MHz, CDCl₃) δ8.71(d, 2H), 6.25(d, *J*=2.4Hz,1H), 6.23(d, *J*=2.4Hz,1H), 5.11(d, 1H),
4.5(s, 2H), 3.61-4.19(m, 2H), 2.78-3.21(m, 1H), 2.15-2.22(d, 1H), 2.01-2.17(m, 6H), 1.80-1.97(m, 3H), 1.35-1.76(m, 14H), 1.20-1.32(d, 2H), 1.01-1.18(m,1H), 0.76-0.99(m, 2H)

### 4. Synthesis of compound 4

### Synthesis route

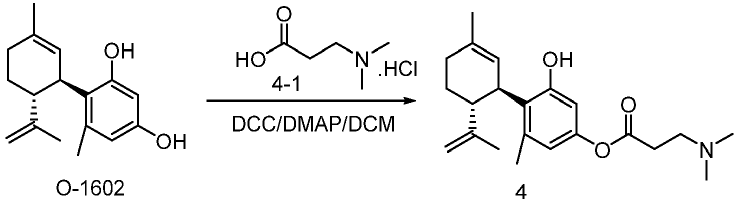

### Operating procedures

To a flask containing compound O-1602 (500 mg, 1.94 mmol), compound 4-1 (895 mg, 5.82 mmol), and DMAP (289 mg, 2.33 mmol) was added anhydrous dichloromethane (20 mL). The mixture was stirred for 5 minutes. Then DCC (1.2 g, 5.82 mmol) was added and the reaction was carried out at room temperature overnight. After adding n-hexane to the system, a solid precipitated. The solid was removed by filtration. The filtrate was directly mixed with silica gel and purified by normal phase column chromatography. Eluent: 10% ethyl acetate in petroleum ether. The product was collected and concentrated under reduced pressure to give a colorless oil, 250 mg, yield: 36%. The structure was confirmed by LCMS and HNMR.
LCMS: MS=358.2 [M+1]⁺
¹HNMR:(400 MHz, CDCl₃) δ 9.26(s,1H), 6.25(d, *J*=2.4Hz,1H), 6.23(d, *J*=2.4Hz,1H), 5.11(d,1H) 4.5(d, 2H), 3.0(s,1H), 2.37(s, 4H), 2.18-2.25(m, 9H), 2.13(s, 3H),1.96(d,1H),1.69(t,2H),1.57-1.65(m,3H), 1.47-1,53(d,3H)

### 5. Synthesis of compound 5

### Synthesis route

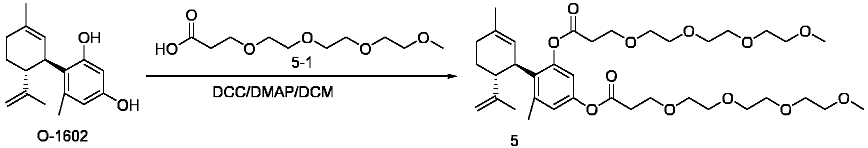

### Operating procedures

Compound 0-1602 (300 mg, 1.16 mmol), compound 5-1 (686 mg, 2.90 mmol), DMAP (50 mg) were dissolved in anhydrous dichloromethane (20 mL), and DCC (717 mg, 3.48 mmol) was added. After stirring at room temperature overnight, a lot of solids were produced after adding n-hexane. The solid was removed by filtration. The filtrate was directly mixed with silica gel and purified by normal phase column chromatography, eluent: 10% ethyl acetate in petroleum ether. The product was collected and concentrated under reduced pressure to give the product as a colorless oil (320 mg, yield: 39%). The chemical structure was confirmed by LCMS and ¹HNMR.
LCMS: MS=695.41 [M+1]⁺
¹HNMR:(400 MHz, CDCl₃) δ 6.25(d,*J*=2.4Hz,1H),6.23(d,*J*=2.4Hz,1H),5.09(d,1H),4.5(m,2H) 3.71(t,14H),3.23(d,6H),2.76(t,3H),2.52-2.65(m,2H),2.21-2.37(d,2H),2.10-2.18(s,1H),1.94-2.04(d, 1H),1.67-1.75(2,2H),1.47-1.67(m,6H)

### 6. Synthesis of compound 6

### Synthesis route

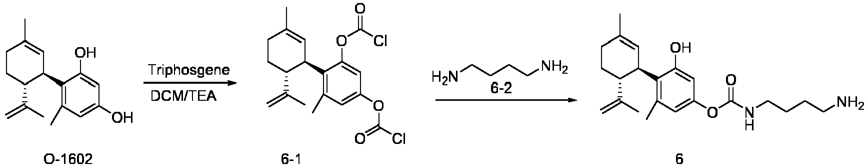

### Operating procedures

Triphosgene (576 mg, 1.94 mmol) was dissolved in anhydrous dichloromethane (10 mL). The temperature was lowered to -10°C. In another reaction flask, compound O-1602 (500 mg, 1,94 mmol) and triethylamine (588 mg, 5.82 mmol) were dissolved in dichloromethane (5 mL) and slowly added dropwise to the triphosgene solution. The reaction was carried out at -10°C for 2 hours, and then allowed to warm to room temperature and carried out overnight. The dichloromethane was concentrated under reduced pressure to obtain the crude product of compound 6-1. Dry tetrahydrofuran was added to the residue to precipitate a solid, which was removed by filtration. The filtrate was transferred to a reaction flask and compound 6-1 (1.094 g, 5.82 mmol) was added. The mixture was stirred at room temperature overnight and concentrated under reduced pressure to give a pale yellow oil, which was purified on a reverse-phase column with the filler as follows: 2.5 µm ostade-cylsilane (ODS), eluent: 0.25% aqueous trifluoroacetic acid/acetonitrile = 4: 6. The product solution was collected and directly lyophilized to give compound 6 as a white powder (270 mg, yield: 37%)
LCMS: MS= 373.21 M+1]⁺;
¹HNMR: (400 MHz, CDCl₃) δ 9.21 (s, 1H), 6.25 (d, *J =* 2.4 Hz, 1H), 6.16 (d, *J =* 2.4 Hz, 1H) 5.98 (d, 1H), 4.52-4.41 (d, 2H), 3.20-3.04(m, 2H), 3.03-2.92 (q, 3H), 2.76 (s, 1H), 2.26-2.15 (m, 2H), 2.16 - 2.04 (m, 3H), 1.74 - 1.65 (m, 3H), 1.52-1.44 (m, 7H), 1.47-1.35 (m, 2H). 1.29-1.16 (m, 2H).

### 7. Synthesis of compound 7

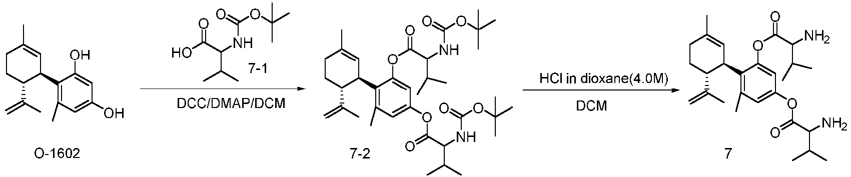

### Operating procedures

### Synthesis of compound 7-2

Compound O-1602 (516 mg, 2.0 mmol), compound 7-1 (1085 mg, 5.0 mmol), and DMAP (24.4 mg, 0.2 mmol) were dispersed in 25 mL of anhydrous dichloromethane. DCC (1030 mg, 5.0 mmol) was added under a nitrogen atmosphere at room temperature. After the addition was completed, the mixture was stirred at room temperature for 3 hours. The reaction mixture was diluted with 60 mL of dichloromethane, and then washed with a saturated ammonium chloride solution (50 mL x 2) and saturated brine (30 mL x 2). The organic layer was dried over anhydrous sodium sulfate, suction-filtered, and concentrated under reduced pressure to remove the solvent to obtain the crude product, which was purified by normal phase column chromatography, with the filler as follows: 200-300 mesh silica gel (A: petroleum ether; B: ethyl acetate; 0% A-50% B, 60 min) to give the product 7-2s (950 mg, 1.547 mmol, yield: 77.4%).
LCMS: MS=615.21 [M+1]⁺
¹HNMR: (400 MHz, CDCl₃) δ 6.77 (s, 1H), 6.64 (d, *J* = 24.0 Hz, 1H), 5.22 (s, 1H), 5.06 (d, *J* = 12.0 Hz, 1H), 4.59 - 4.35 (m, 4H), 3.70 - 3.62 (m, 1H), 2.56 - 2.51 (m, 1H), 2.38 - 2.28 (m, 6H), 2.14 - 1.99 (m, 2H), 1.79 - 1.66 (m, 8H), 1.62 - 1.43 (m, 21H), 1.06 - 0.96 (m, 12H).

### Synthesis of compound 7

Product 3-1 (350 mg, 0.57 mmol) was dissolved in anhydrous dichloromethane (15 mL). Dioxane hydrochloride (4.0 M, 7.5 mL) was added under a nitrogen atmosphere at room temperature. After the addition was completed, the reaction mixture was stirred at room temperature for 12 hours. The reaction mixture was concentrated under reduced pressure (40°C water temperature) and the solvent was removed. The crude product was purified by slurrying with dichloromethane/petroleum ether (1: 5) for 3 hours to give compound 7 as a white solid hydrochloride salt (260 mg, 0.684 mmol, yield: 86.6%).
LCMS: MS=457.40[M+1]⁺
¹HNMR: (400 MHz, CDCl₃) δ 6.77 (s, 1H), 6.64 (d, *J* = 24.0 Hz, 1H), 5.22 (s, 1H), 5.06 (d, *J* = 12.0 Hz, 1H), 4.59 - 4.35 (m, 4H), 3.70 - 3.62 (m, 1H), 2.56 - 2.51 (m, 1H), 2.38 - 2.28 (m, 6H), 2.14 - 1.99 (m, 2H), 1.79 - 1.66 (m, 8H), 1.06 - 0.96 (m, 12H).

### 8. Synthesis of compound 8

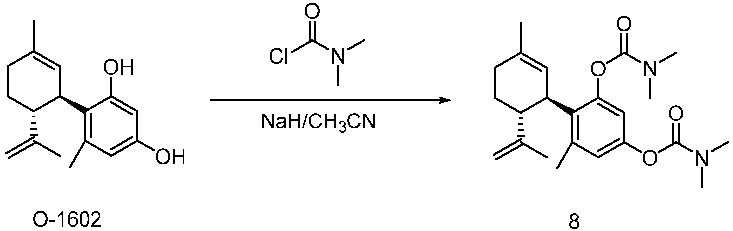

### Operating procedures

0-1602 (645 mg, 2.5 mmol) was dissolved in 25 mL of anhydrous tetrahydrofuran. NaH (250 mg, 6.25 mmol) was added at -5°C under a nitrogen atmosphere. After the addition was completed, the mixture was continuously stirred at -5°C for 30 min. The temperature was maintained constant, and then, N, N-dimethylformyl chloride (669 mg, 6.25 mmol, dissolved in 10 mL of anhydrous tetrahydrofuran) was added dropwise to the reaction solution within 10 minutes. After the addition was completed, the reaction was continued at -5°C for 1 hour. The reaction was quenched with 10 mL of saturated ammonium chloride solution. The mixture was extracted with ethyl acetate (50 mL x 2). The organic layers were combined, washed with saturated brine (50 mL x 1), dried over anhydrous sodium sulfate, filtered by suction, and concentrated under reduced pressure (40°C water temperature) to remove the solvent to give the crude product, which was purified by normal phase column chromatography, with the filler as follows: 200-300 mesh silica gel (A: petroleum ether; B: ethyl acetate; 0% A-30% B, 60 min; in 10% of B in A), followed by a reverse phase column chromatography, with the filler as follows: 2.5 µm ODS (A: 5 mmol/L aqueous ammonium bicarbonate solution; B: acetonitrile; 0% B-100% B, 50 min; 75% B in A). The peak of interest was collected, concentrated, and lyophilized to give product 8 as a white solid (540 mg, 1.35 mmol, yield: 54%)
LCMS: MS= 401[M+1]⁺;
¹H-NMR: (400 MHz, CDCl₃) δ 6.76 (d, *J =* 2.4 Hz, 1H), 6.69 (d, *J =* 2.4 Hz, 1H), 5.31 (s, 1H), 4.60 (d, *J =* 20.0 Hz, 1H), 4.51 (d, *J =* 20.0 Hz, 1H), 3.56 (d, *J =* 8.0 Hz, 1H), 3.02 (s, 6H), 2.96 (s, 6H), 2.61 - 2.54 (m, 1H), 2.24 (s, 3H), 2.05 - 1.81 (m, 2H), 1.79 - 1.75 (m, 2H), 1.68 - 1.63 (m, 3H), 1.54 (s, 3H).

### 9. Synthesis of compound 9

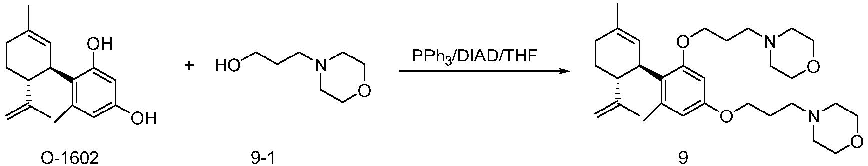

### Operating procedures

0-1602 (516 mg, 2.0 mmol), compound 9-1 (725 mg, 5.0 mmol), and triphenylphosphine (1031 mg, 5.0 mmol) were dispersed in 25 mL of anhydrous tetrahydrofuran. Diisopropyl azodicarboxylate (1.01 g, 5.0 mmol) was added dropwise under a nitrogen atmosphere at 0°C. After the addition was complete, the reaction mixture was stirred at room temperature for 16 hours. The reaction mixture was concentrated under reduced pressure (40°C water temperature) to remove the solvent. The crude product was diluted with 80 mL of dichloromethane. The mixture was washed successively with a saturated ammonium chloride solution (50 mL x 2) and saturated brine solution (30 mL x 2). The organic layer was dried over anhydrous sodium sulfate, suction-filtered, and concentrated under reduced pressure (water temperature of 40°C) to obtain a crude product, which was purified by reverse phase column chromatography, with the filler as follows: 2.5 µm ODS) (A: 5 mmol/L ammonium bicarbonate solution; B: acetonitrile; 0% B-100% B, 60 min) to give product 9 as a pale yellow wax (350 mg, 0.684 mmol, yield: 34.2%).
LCMS: MS=513[M+1]⁺ 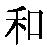 MS= 257[M/2+1]⁺;
¹HNMR: (400 MHz, CDCl₃) δ 6.27 (d, *J* = 8.0 Hz, 2H), 5.24 (d, *J* = 12.0 Hz, 1H), 4.46 (t, *J* = 36 Hz, 2H), 3.99 - 3.89 (m, 4H), 3.79 - 3.75 (m, 8H), 2.63 - 2.53 (m, 13H), 2.31 - 2.20 (m, 3H), 2.14 - 1.97 (m, 7H), 1.80 - 1.79 (m, 2H), 1.78 (s, 3H), 1.67 - 1.51 (m, 3H).

### 10. Synthesis of compound 10

### Synthesis route

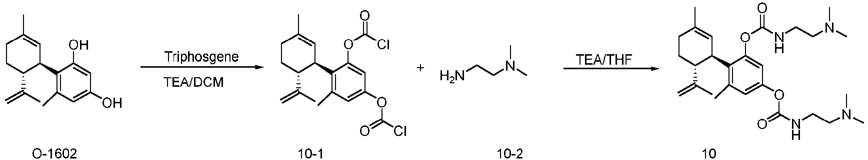

### Operating procedures

Triphosgene (1.15 g, 3.88 mmol) was dissolved in 15 mL of anhydrous tetrahydrofuran. A mixed solution of O-1602 (1.0 g, 3.88 mmol) and triethylamine (1.18 g, 11.64 mmol) was added under a nitrogen atmosphere at 0°C within 5 min. After the addition was completed, the reaction was continued at 0°C for 2 hours, then at room temperature for 18 hours. The solvent was removed by concentrated under reduced pressure (30°C water temperature) to obtain a crude product of compound 10-1. The residue was redissolved in 15 mL of anhydrous tetrahydrofuran and suction-filtered. The collected filtrate was transferred to a separatory funnel and added dropwise to a mixed solution of compound 10-2 (1.02 g, 11.64 mmol) and triethylamine (1.18 g, 11.64 mmol) at 0°C within 5 minutes. After the completion of the dropwise addition, the reaction was continued for 2 hours at room temperature. The solvent was removed by concentrated under reduced pressure (30°C water temperature) to obtain a crude product, which was purified by reverse phase column chromatography, with the filler as follows: 2.5 µm ODS) (A: 0.1% trifluoroacetic acid in water; B: acetonitrile; 0% B-100% B, 60 min; ~50% B in A) twice. The peak of interest was collected and lyophilized to give a solid product 10 (360 mg, 0.741 mmol, yield: 19.1%).
LCMS: MS= 487 [M+1]⁺ and MS= 244 [M/2+1]⁺;
¹HNMR: (400 MHz, CDCl₃) δ 7.290 (s, 1H), 6.968 (s, 1H), 6.753 (s, 1H), 6.714 (s, 1H), 5.123 (d, *J =* 21.6 Hz, 1H), 4.553 (s, 1H), 4.405 (s, 1H), 3.689 - 3.441 (m, 5H), 3.204 (s, 4H), 2.793 (s, 12H), 2.339 (s, 1H),2.227 (s, 3H) 2.001 - 1.956 (m, 1H), 1.763 - 1.605 (m, 4H), 1.530 (s, 3H), 1.311 - 1.255 (d, *J* = 22.4 Hz, 2H).

### 11. Synthesis of compound 11

### Synthesis route

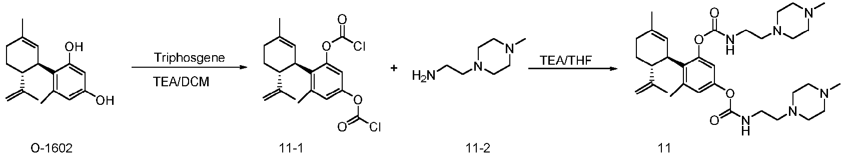

### Operating procedures

Triphosgene (1.15 g, 3.88 mmol) was dissolved in 15 mL of anhydrous tetrahydrofuran. A mixed solution of O-1602 (1.0 g, 3.88 mmol) and triethylamine (1.18 g, 11.64 mmol) (O-1602 and triethylamine were dissolved in 10 mL of anhydrous tetrahydrofuran) was added under a nitrogen atmosphere at 0°C within 5 min. After completion of the addition, the reaction was continued at 0°C for 2 hours and then at room temperature for 18 hours. The solvent was removed under reduced pressure (30°C water temperature) to obtain a crude product of compound 11-1. The residue was redissolved in 15 mL of anhydrous tetrahydrofuran and suction-filtered. The collected filtrate was transferred to a separatory funnel and added dropwise to a solution of compound 11-2 (1.66 g, 11.64 mmol) and triethylamine (1.18 g, 11.64 mmol) (Compound 11-2 and triethylamine were dissolved in 15 mL of anhydrous tetrahydrofuran) at 0°C within 5 min. After the addition was completed, the reaction was continued at room temperature for 2 hours. The solvent was removed by concentration under reduced pressure (30°C water temperature) to give a crude product, which was purified by reverse phase column chromatography, with the filler as follows: 2.5 µm ODS) (A: 0.1% trifluoroacetic acid in water; B: acetonitrile; 0% B-100% B, 60 min; ~48% B in A) 3 times. The peak of interest was collected and lyophilized to give a solid product 11 (330 mg, 0.554 mmol, yield: 14.3%).
LCMS: MS=597[M+1]⁺ and MS=299[M/2+1]⁺;
¹HNMR: (400 MHz, DMSO^{-d6}) δ 7.979 (brs, 1H), 7.730(brs, 1H), 6.795 (s, 2H), 5.131-5.014 (m, 1H), 4.451-4.012 (m, 2H), 3.489-3.476 (m, 24H), 2.829 (s, 6H), 2.319 (m, 1H), 2.208 (s, 3H), 1.926-1.886 (m, 1H), 1.686-1.662 (m,4H), 1.576 (s, 3H), 1.527(s. 3H)

### 12. Synthesis of compound 12

### Synthesis route

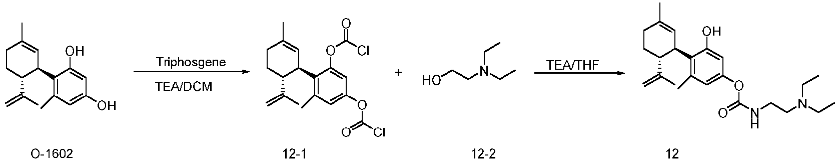

### Operating procedures

Triphosgene (856 mg, 2.91 mmol) was dissolved in 10 mL of anhydrous tetrahydrofuran. A mixed solution of O-1602 (1.0 g, 3.88 mmol) and triethylamine (588 mg, 5.82 mmol) (O-1602 and triethylamine were dissolved in 10 mL of anhydrous tetrahydrofuran) was added under a nitrogen atmosphere at 0°C within 5 min. After completion of the addition, the reaction was continued at 0°C for 2 hours and then at room temperature for 18 hours. The solvent was removed under reduced pressure (30°C water temperature) to obtain a residue. The residue was redissolved in 15 mL of anhydrous tetrahydrofuran and suction-filtered. The collected filtrate was transferred to a separatory funnel and added dropwise to a solution of compound 12-2 (681 mg, 5.82 mmol) and triethylamine (588 mg, 5.82 mmol) at 0°C within 5 min. After the addition was completed, the reaction was continued at room temperature for 2 hours. The solvent was removed by concentration under reduced pressure (30°C water temperature) to give a crude product, which was purified by reverse phase column chromatography, with the filler as follows: 2.5 µm ODS) (A: 0.1% trifluoroacetic acid in water; B: acetonitrile; 0% B-100% B, 60 min; ~62% B in A) 2 times. The peak of interest was collected and lyophilized to give a solid product 12 (290 mg, 0.723 mmol, yield: 18.6 %).
LCMS: MS= 402[M+1]⁺;
¹HNMR (400 MHz, CDCl₃) δ 11.61 (d, J = 13.3 Hz, 1H), 6.56 (d, J = 2.0 Hz, 1H), 6.44 (d, J = 12.9 Hz, 1H), 6.02 (d, J = 2.4 Hz, 1H), 5.14 (t, J = 14.1 Hz, 1H), 4.58 (d, J = 7.8 Hz, 2H), 4.54 - 4.33 (m, 2H), 3.56 (d, J = 67.0 Hz, 1H), 3.43 - 3.33 (m, 2H), 3.20 (d, J = 6.2 Hz, 1H), 2.70 - 2.40 (m, 1H), 2.38 - 2.27 (m, 1H), 2.19 (s, 3H), 2.17-2.13 (m, 1H), 2.12 - 1.91 (m, 1H), 1.86 - 1.66 (m, 3H), 1.60 (s, 3H), 1.58 (s, 3H), 1.29 (dd, J = 15.9, 9.1 Hz, 8H).

### 13. Synthesis of compound 13

### Synthesis route

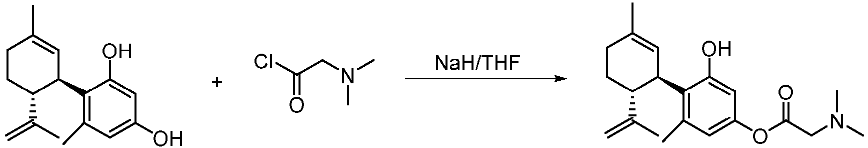

### Operating procedures

0-1602 (645 mg, 2.5 mmol) was dissolved in 25 mL of anhydrous tetrahydrofuran. NaH (150 mg, 3.75 mmol) was added at 0°C under a nitrogen atmosphere. After the addition was completed, the mixture was continuously stirred at 0°C for 30 mins. The temperature was maintained constant, and then, N, N-dimethylacetyl chloride (454 mg, 3.75 mmol, dissolved in 10 mL of anhydrous tetrahydrofuran) was added dropwise to the reaction solution within 10 minutes. After the addition was completed, the reaction was continued at 0°C for 1 hour. The reaction was quenched with 10 mL of saturated ammonium chloride solution. The mixture was extracted with ethyl acetate (50 mL x 2). The organic layers were combined, washed with saturated brine (50 mL x 1), dried over anhydrous sodium sulfate, filtered by suction, and concentrated under reduced pressure (40°C water temperature) to remove the solvent to give the crude product, which was purified by normal phase column chromatography, with the filler as follows: 200-300 mesh silica gel (A: petroleum ether; B: ethyl acetate; 0% A-30% B, 60 min; in 15% of B in A) to give a crude product, followed by a reverse phase column chromatography, with the filler as follows: 2.5 µm ODS (A: 0.1 % aqueous ethyl trifluoroacetate solution; B: acetonitrile; 0% B-100% B, 60 min; ~ 55% B in A). The peak of interest was collected, concentrated, and lyophilized to give product 13 as a white solid (310 mg, yield: 36.2 %).
LCMS: MS= 344 [M+1]⁺
¹HNMR: (400 MHz, CDCl₃) δ 6.587 (s, 1H), 6.432 (s, 1H), 5.185 (d, *J =* 20.0 Hz, 1H), 4.577 (d, *J* = 30.0 Hz, 1H), 4.441 (s, 1H), 4.180 (d, *J* = 16.4 Hz, 1H), 3.694 (d, *J* = 17.2 Hz, 1H), 3.510 (d, *J* = 9.2 Hz, 1H), 2.994 (s, 6H), 2.505 - 2.456 (m, 1H), 2.258 (s, 3H), 2.197 - 1.979 (m, 2H), 1.787 (s, 2H), 1.651 (s, 3H), 1.488 (s, 3H).

### 14. Synthesis of compound 14

### Synthesis route

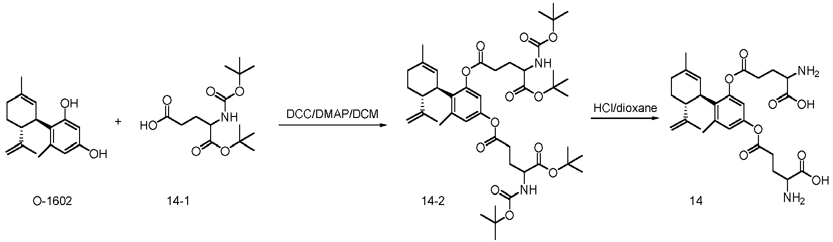

### Operating procedures

### The first step:

Compound 0-1602 (1 g, 3.88 mmol), compound 14-1 (3.5 g, 11.63 mmol), and DMAP (100 mg) were added to dichloromethane. After stirring for 5 minutes, DCC (1.99 g, 9.70 mmol) was added and stirred at room temperature overnight. After the addition of n-hexane, a solid was produced. The solid was removed by filtration. The filtrate was directly mixed with silica gel and purified by normal phase column chromatography with a filler as follows: 200-300 mesh silica gel. Eluent: petroleum ether: ethyl acetate = 1: 9. The product was collected and concentrated under reduced pressure to give 900 mg of a white solid compound, which was the target compound 14-2, yield: 28%.
LCMS: MS=729 [M-99]⁺ is the molecular weight of compound 14 minus one tert-butyl ester

### The second step:

Compound 14-2 (830 mg, 1 mmol) was dissolved in 1,4-dioxane (10 mL). 5 mol/L hydrochloric acid in 1,4-dioxane was added and stirred at room temperature for 3 hours. The reaction mixture was concentrated under reduced pressure to remove the solvent and slurried with ethyl acetate to precipitate a solid, which was filtered to give 310 mg of a white powder, i.e. hydrochloride salt of compound 14, yield: 60%.
LCMS: MS=517 [M+1]⁺
¹HNMR: (400 MHz, D2O) δ 6.80 (d, J=2.4Hz, 1H), 6.70 (d, J=2.4Hz, 1H), 5.02 (d, 1H), 4.44 (d, 2H), 3.912-.3.879 (m, 2H), 3.52-3.50 (m, 1H), 2.62-2.52 (m, 4H), 2.33 (s, 1H), 2.23(s, 3H), 2.00-1.95 (m, 3H), 1.87-1.82 (m, 2H), 1.71-1.60 (m, 4H), 1.52 (s, 3H), 1.45-1.33 (m, 9H)

### Example 1: experimental on 0-1602 releases of 0-1602 derivatives under different pH conditions

### Experimental equipment

Electronic balance, high-performance liquid chromatography, and pH meter

### Experimental reagent

Distilled water, phosphoric acid, sodium dihydrogen phosphate, sodium hydroxide, concentrated hydrochloric acid, and acetonitrile

### Experimental samples

0-1602 derivatives

### Chromatographic conditions

Chromatographic column: shimnex CS C18 5 µm, 4.6*250 mm
Mobile phase A: 0.1% phosphoric acid in water; mobile phase B: acetonitrile (see Table 1)

**Table 1: mobile phase**

| time (min) | A (%) | B (%) |
|---|---|---|
| 0 | 90 | 10 |
| 10 | 10 | 90 |
| 15 | 10 | 90 |
| 20 | 90 | 10 |
| 25 | 90 | 10 |

Wavelength: 210 nm; flow rate: 1.0 mL/min; column temperature: 30°C; and loading volume: 10 µL

### Experimental procedures

### Preparation of buffers

0.2 mol/L hydrochloric acid solution: 18.0 ml of concentrated hydrochloric acid was accurately measured, diluted with water to 1000 ml, and shaken well.

0.2 mol/L sodium dihydrogen phosphate solution: 6.8 g of sodium dihydrogen phosphate was weighed, dissolved with 250 ml of water, and shaken well.

0.2 mol/L sodium hydroxide solution: 1.6 g of sodium hydroxide was weighed, dissolved with 200 mL of water, and shaken well.

### Preparation of pH 1.6-8.0 buffers

Buffer solution (pH 1.6): 32.4 ml of 0.2 mol/L hydrochloric acid was accurately measured, diluted with water to 200 ml, and shaken well.

Phosphate buffer (pH 5.0): a certain amount of 0.2 mol/L sodium dihydrogen phosphate solution was taken and the pH value was adjusted by using 0.2 mol/L sodium hydroxide solution to 5.0.

Phosphate buffer (pH 6.8): a certain amount of 0.2 mol/L sodium dihydrogen phosphate solution was taken and the pH value was adjusted by using 0.2 mol/L sodium hydroxide solution to 6.8.

Phosphate buffer (pH 7.2): a certain amount of 0.2 mol/L sodium dihydrogen phosphate solution was taken and the pH value was adjusted by using 0.2 mol/L sodium hydroxide solution to 7.2.

Phosphate buffer (pH 8.0): a certain amount of 0.2 mol/L sodium dihydrogen phosphate solution was taken and the pH value was adjusted by using 0.2 mol/L sodium hydroxide solution to 8.0.

### Preparation of sample solutions

About 1 mg of 0-1602 derivative was placed in a 2 mL centrifuge tube and dissolved by adding 1 mL of acetonitrile. Then 1 mL of buffer solution was added and shaken well. The obtained solution was incubated at 37°C, and sampled for determination respectively after for 2 hours and 4 hours. (The releases of O-1602 were respectively investigated at pH 1.6, pH 5.0, pH 6.8, pH 7.2, and pH 8.0)
Calculations: the content of O-1602 was calculated by area percentage.

### Experimental results

①For O-1602 derivatives 1-13, under the acidic buffer solution (pH 1.6), only derivatives 12 and 13 incubated at 37°C for 4 hours had a trace amount of O-1602 release, and other compounds were stable;
②The 0-1602 derivatives 2, 8, and 9 had good stability after being incubated at pH 1.6 ~ 8.0 at 37°C for 2 hours and 4 hours, and no O-1602 was released;
③O-1602 derivatives 1, 6, 7, 10, 11, 12, and 13 all have different degrees of release; the release of 0-1602 tended to increase with the increase of time and pH. Among them, O-1602 in 6, 11, and 13 had a small amount of release; the release amount of O-1602 was relatively high in 1, 7, 10, and 12, of which O-1602 was completely released in 12, while O-1602 was only partially released and partially degrade into other impurities in 1, 7, and 10.
④See Table 2 for specific data.

**Table 2: specific data**

| Name | | 0 h | Buffer (pH 1.6) | | Buffer (pH 5.0) | | Buffer (pH 6.8) | | Buffer (pH 7.2) | | Buffer (pH 8.0) | |
|---|---|---|---|---|---|---|---|---|---|---|---|---|
| | | | 2 h | 4 h | 2 h | 4 h | 2 h | 4 h | 2 h | 4 h | 2 h | 4 h |
| 1 | Principal component % | 97.1 85 | 96.9 06 | 94.1 15 | 78.0 30 | 24.6 51 | ND | ND | ND | ND | ND | ND |
| | O-1602% | ND | ND | ND | ND | 0.45 7 | 9.80 1 | 20.8 13 | 6.35 4 | 20.1 72 | 11.4 55 | 33.9 63 |
| 7 | Principal component % | 87.6 91 | 79.0 94 | 81.4 24 | 26.6 93 | 8.30 7 | 3.40 1 | 1.55 1 | ND | ND | ND | ND |
| | O-1602% | ND | ND | ND | 1.08 8 | 3.80 0 | 1.97 2 | 3.51 8 | 29.3 54 | 19.3 43 | 11.9 33 | 43.9 76 |
| 1 0 | Principal component% | 92.0 84 | 95.1 98 | 96.4 45 | 95.0 16 | 92.5 80 | 38.2 7 | 18.6 22 | 32.3 22 | 8.18 5 | 6.82 7 | 0.30 1 |
| | O-1602% | ND | ND | ND | 0.13 3 | 0.13 6 | 2.50 9 | 3.69 9 | 3.45 4 | 10.8 48 | 12.1 61 | 28.3 90 |
| 1 2 | Principal component% | 96.8 71 | 94.1 50 | 94.8 04 | 93.5 33 | 88.1 59 | 90.0 33 | 86.7 39 | 19.4 34 | 5.17 7 | 7.91 6 | 1.22 0 |
| | O-1602% | ND | ND | 0.10 8 | 5.09 1 | 9.62 5 | 7.86 4 | 11.3 77 | 79.1 45 | 92.8 00 | 90.0 80 | 96.6 48 |

### Example 2: use of a low dosage of an 0-1602 derivative alleviates moderate to mild psoriasis induced in mice.

### 1. Induction of moderate to mild psoriasis

C57BL/6 mice, female, 6-8 weeks old were used. On day -7 of the experiment, the experimental animals were weighed, and the mice were randomly divided into cages according to their body weights. The animals were allowed to adapt to the environment of the animal room. The animal room was maintained at a constant temperature (23°C ± 3°C) and constant humidity (50% relative humidity). Animals are fed standard laboratory food and have free access to drinking water. On day 2 of the experiment, mice injected with anesthetic (tribromoethanol, 300-375 mg/kg) were shaved with a shaver from shoulder to hip. Then, the shaved part was sufficiently covered with depilatory cream. After placement of 3-5 min, the back hair was wiped off to expose the skin. On day 0 of the experiment, the mice injected with anesthetic were placed on the test bench. An appropriate amount of IMQ was evenly applied on the back skin of the mice (the amount of IMQ applied on each mouse was the same as possible, e.g., 80 mg/mouse), and weighed and scored. On days 1-4 of the experiment: intradermal injection of rIL-23. Mouse rIL-23 stock solution was diluted in sterile PBS to a working concentration of 25 ng/µl. After aspiration of the diluted recombinant rIL-23 into a 0.5 ml syringe, the needle was inserted into the skin and tilted upwards at as shallow an angle as possible. 25 µl of the solution containing recombinant rIL-23 (1 µg per day) was injected in a slow, continuous motion, without aspiration, to avoid puncture of the blood vessels. An appropriate intradermal injection would result in a small round blister.

### 2. Administration of 0-1602 derivatives

The solid O-1602 derivative was dissolved in DMSO at a concentration of 2 mg/ml. The above solution was diluted to 0.1 mg/ml using PBS. Gavage was performed daily at 14: 00 p. m. in a volume of 200 µl. The treatment schedule for the O-1602 derivatives was days 2-8 of the experiment. TEMOVATE serves as a positive control.

### 3. Daily Observation Index

The body weight and skin condition of the mice were observed daily, and the back skin condition was scored by PASI score.

### 4. Observation Endpoint

The experiment ended on day 8. Mice were euthanized by cervical amputation under isoflurane anesthesia. Mice were dissected and the back skin was taken and subjected to hematoxylin and eosin staining for histological evaluation.

### 5. Experimental results

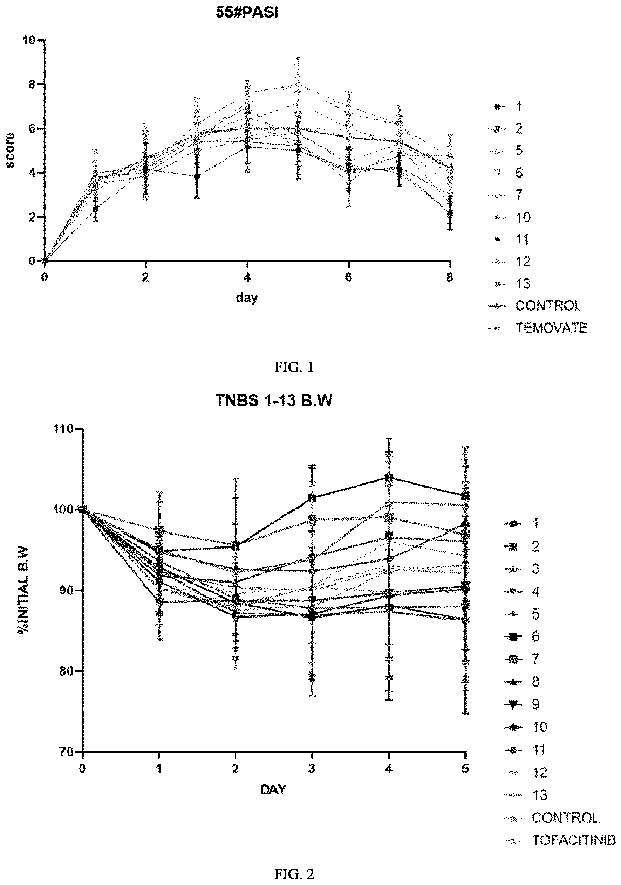

At a dosage of 1 mg/kg, the O-1602 derivative was effective in relieving moderate to mild psoriasis and in relieving the PASI score in mice, which was also confirmed in the pathology score (FIG. 1).

### Example 3: relief of 2,4,6-trinitrobenzene sulfonic acid (TNBS)-induced acute enteritis in mice using O-1602 derivatives

### 1. Induction of TNBS's acute enteritis

C57BL/6 mice, male, and female, 6 to 10 weeks old were used. On day -7 of the experiment, the experimental animals were weighed, and the mice were randomly divided into cages according to their body weights. The animals were allowed to adapt to the environment of the animal room. The animal room was maintained at a constant temperature (23°C ± 3°C) and constant humidity (50% relative humidity). Animals are fed standard laboratory food and have free access to drinking water. On day 0, mice were induced for TNBS colitis by intrarectal injection. The dosage of TNBS was 125 mg/kg. The solvent was 30% alcohol. Each mouse received a volume of 100 µl. After the rectal injection, the mice were inverted for 1 minute to prevent leakage of the drug solution.

### 2. Administration of 0-1602 derivatives

The solid O-1602 derivative was dissolved in DMSO at a concentration of 2 mg/ml. The above solution was diluted to 0.1 mg/ml using PBS. TNBS enteritis mice received once daily gavage in a volume of 200 µl and were dosed daily at 11: 00 a. m. The dosage of the O-1602 derivative was 1 mg/kg. The treatment schedule for the O-1602 derivative was from day 0 to day 4 of the TNBS. The mice in the control group were administered with an equal volume of solvent by gavage. At the same time, the anti-colitis drug JAK inhibitor tofacitinib was used as a positive control. The experimental results of 0-1602 derivatives in this model experiment were compared. The O-1602 derivatives were dissolved in the above-mentioned way; the concentration was 1.5 mg/mL; the volume of intragastric administration was 200 µL; and the dosage was 15 mg/kg.

### 3. Daily observation indices

Mice were observed daily for body weight and anal bleeding, and the daily disease activity index (DAI) was calculated and recorded.

### 4. Observation Endpoint

The experiment was terminated on day 5 of TNBS. Mice were euthanized by cervical amputation under isoflurane anesthesia. The mice were dissected and the whole colon was obtained. The length of the colon was measured and photographed.

### 5. Experimental results

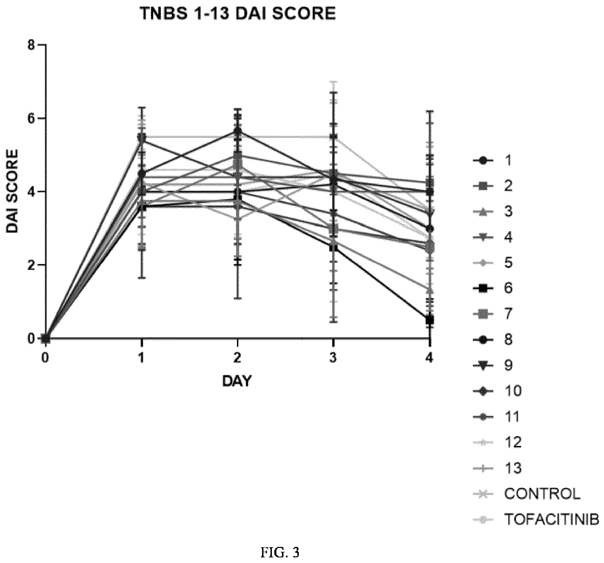

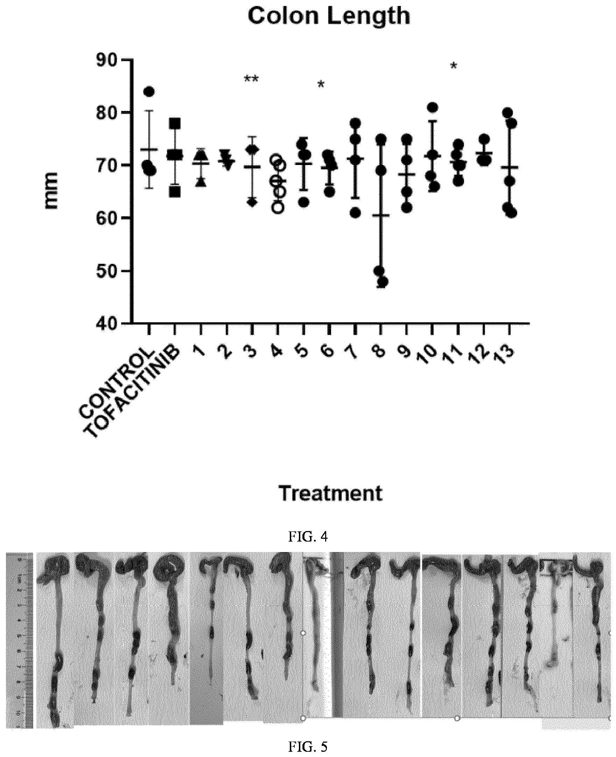

The use of O-1602 derivatives, in which Compounds 1, 3, 6, and 7 significantly alleviated the weight loss in model animals (see FIG. 2), and Compounds 3, 6, 7, and 11 significantly alleviated the DAI (see FIG. 3), increased the length of colon (see FIGs. 4 and 5) and reduced the damage to colon tissue (see FIG. 5), with superior efficacy to that of tofacitinib.

The above is only the preferred implementation of the present invention and does not limit it. Any modifications and equivalent substitutions etc. made within the spirit and principles of the present invention should be included in the scope of protection of the present invention.

The foregoing embodiments and methods described in the present invention may vary based on the capabilities, experience, and preferences of those skilled in the art.

The mere fact that the steps of a method are listed in a certain order does not constitute any limitation on the order of the steps of the method.

## Claims

1. A compound, a stereoisomer of the compound, any composition of the compound and the stereoisomer, any composition of the stereoisomers, or the pharmaceutically acceptable salt, ester, ether, optical isomer, isotope derivative, solvate, prodrug, metal chelate, crystal form of the foregoing compound or composition, the compound having the following structure: 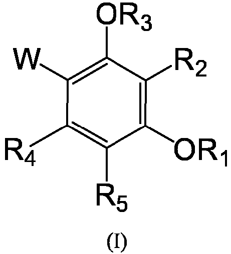 wherein
R₁ has the following structure: 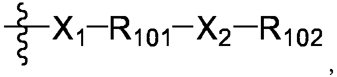 wherein X₁ is selected from a single bond, -C(O)-, -C(O)O-, -C(O)N(R₁₀₃)-, 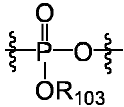 , -S(O)₂-; X₂ is selected from a single bond, -O-, -S-, -S-S-, -N(R₁₀₄)-, -S(O)₂-, -S(O)₂N(R₁₀₄)-, -S(O)-, -S(O)N(R₁₀₄)-, -C(O)-, -C(O)O-, -C(O)N(R₁₀₄)-, -OC(O)-, -OC(O)N(R₁₀₄)-, -N(R₁₀₄)C(O)O-, -N(R₁₀₄)C(O)-, -N(R₁₀₄)S(O)₂-, 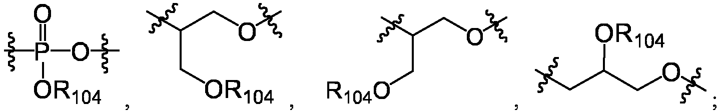
R₁₀₁ is selected from a single bond, alkylene, alkenylene, phenylene, 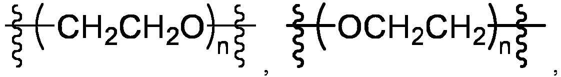 amino acid residue, oligopeptide residue, polypeptide residue, monosaccharide residue, oligosaccharide residue, polysaccharide residue; n is an integer of 1-100; R₁₀₂ is selected from H, alkyl, cycloalkyl, phenyl or heterocyclyl (in particular saturated heterocyclyl); R₁₀₃ and R₁₀₄ are independently selected from H or alkyl;
wherein the alkylene, alkenylene, phenylene, alkyl, cycloalkyl, phenyl, and heterocyclyl are optionally substituted with one or more independent R';
R₂, R₄, and R₅ are independently selected from: H, halogen, hydroxyl, mercapto, amino, cyano, azido, isocyanate, sulfonyl chloride, sulfonyl fluoride, amido, sulfonamido, formic acid group, nitro, formaldehyde group, trifluoromethyl, sulfonic acid group, phosphite group, trialkyltin group, trialkylsilyl, alkyl, alkenyl or alkynyl;
R₃ has the following structure: 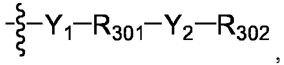 wherein Y₁ is selected from a single bond, -C(O)-, -C(O)O-, -C(O)N(R₃₀₃)-, 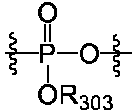 , -S(O)₂-; Y₂ is selected from a single bond, -O-, -S-, -S-S-, -N(R₃₀₄)-, -S(O)₂-, -S(O)₂N(R₃₀₄)-, -S(O)-, -S(O)N(R₁₀₄)-, -C(O)-, -C(O)O-, -C(O)N(R₃₀₄)-, -OC(O)-, -OC(O)N(R₃₀₄)-, -N(R₃₀₄)C(O)O-, -N(R₃₀₄)C(O)-, -N(R₃₀₄)S(O)₂-, 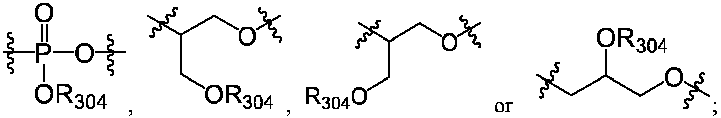
R₃₀₁ is selected from a single bond, alkylene, alkenylene, phenylene, 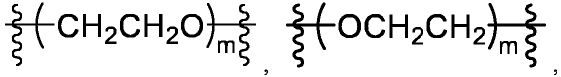 amino acid residue, oligopeptide residue, polypeptide residue, monosaccharide residue, oligosaccharide residue or polysaccharide residue; m is an integer of 1-100; R₃₀₂ is selected from H, alkyl, cycloalkyl, phenyl or heterocyclyl; R₃₀₃ and R₃₀₄ are independently selected from H or alkyl; wherein the alkyl and alkylene are optionally substituted with one or more independent R';
or, R₄ and R₅, together with the carbon atom to which they are attached, form a cyclic group (in particular a saturated cyclic group such as cycloalkyl, heterocyclyl); wherein the cyclic group is optionally substituted with one or more independent R';
W is selected from the following structures: 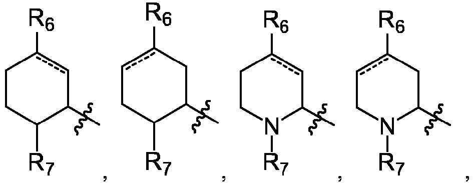 or 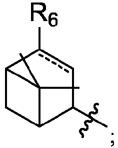
wherein -̅ -̅ -̅ -̅ -̅ -̅ represents a single bond or a double bond;
each R₆ independently has the following structure: 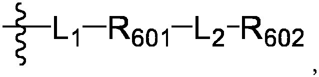 wherein L₁ and L₂ are independently selected from a single bond, -O-, -S-, -N(R₆₀₃)-, -S(O)₂-, -S(O)₂N(R₆₀₃)-, -S(O)-, -S(O)N(R₆₀₃)-, -C(O)-, -C(O)O-, -C(O)N(R₆₀₃)-, -OC(O)-, -OC(O)N(R₆₀₃)-, -N(R₆₀₃)C(O)O-, -N(R₆₀₃)C(O)- or -N(R₆₀₃)S(O)₂-; R₆₀₁ is selected from a single bond or alkylene;
R₆₀₂ is selected from H or alkyl; and R₆₀₃ is selected from H or alkyl; wherein the alkyl and alkylene are optionally substituted with one or more independent R';
each R₇ is independently selected from H, alkyl, alkenyl or alkynyl, wherein the alkyl, alkenyl, and alkynyl are optionally substituted with one or more independent R';
each R' is independently selected from halogen, hydroxyl, mercapto, amino, cyano, azido, isocyanate, sulfonyl chloride, sulfonyl fluoride, amido, sulfonamido, formic acid group, nitro, formaldehyde group, trifluoromethyl, sulfonic acid group, phosphite group, trialkyltin group, trialkylsilyl, -CH=NH, 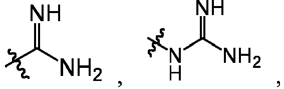
alkoxy, alkylamino, substituted or unsubstituted alkyl, substituted or unsubstituted alkenyl, substituted or unsubstituted alkynyl, substituted or unsubstituted cycloalkyl, substituted or unsubstituted cycloalkylalkyl, substituted or unsubstituted aryl, substituted or unsubstituted aralkyl, substituted or unsubstituted heterocyclyl, substituted or unsubstituted heterocyclylalkyl; and further, the compound represented by formula I is not

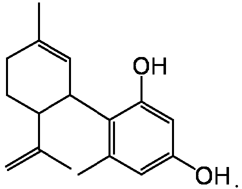

2. The compound, stereoisomer of the compound, any composition of the compound and the stereoisomer, any composition of the stereoisomers, or the pharmaceutically acceptable salt, ester, ether, optical isomer, isotope derivative, solvate, prodrug, metal chelate, crystal form of the foregoing compound or composition of claim 1, wherein X₁ and/or Y₁ is selected from a single bond, -C(O)-, -C(O)O-, -C(O)N(H)-, 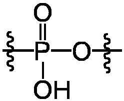 or -S(O)₂-.

3. The compound, stereoisomer of the compound, any composition of the compound and the stereoisomer, any composition of the stereoisomers, or the pharmaceutically acceptable salt, ester, ether, optical isomer, isotope derivative, solvate, prodrug, metal chelate, crystal form of the foregoing compound or composition of claim 1, wherein R₁₀₁ is selected from a single bond, C1-6 alkylene, 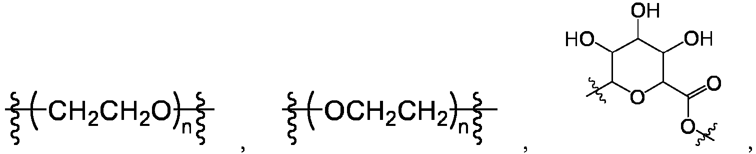 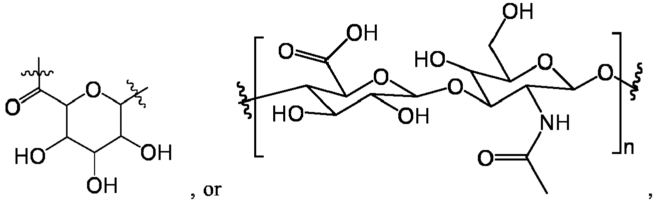 wherein the alkylene is optionally substituted with one or more independent R';
preferably, R' is selected from hydroxyl, mercapto, amino, amido, sulfonamido, formic acid group, nitro, formaldehyde group, trifluoromethyl, 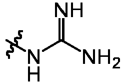 , vinyl, ethynyl, substituted or unsubstituted phenyl (e.g., 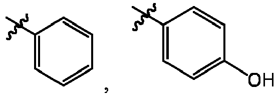 ), heterocyclyl (e.g., 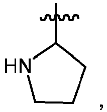 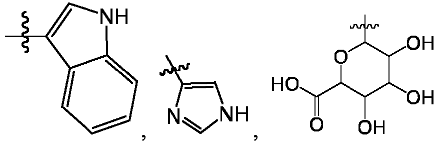
more preferably, R₁₀₁ is selected from a single bond, methylene, ethylene, 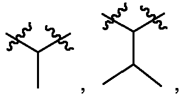 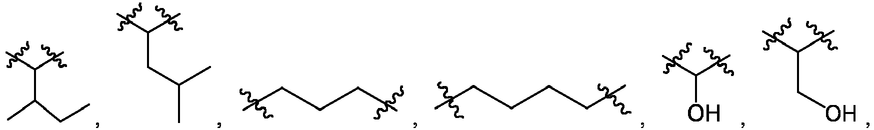 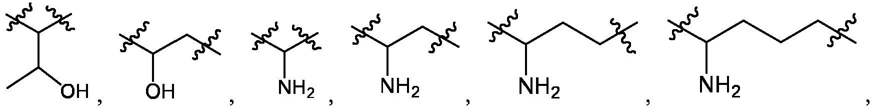 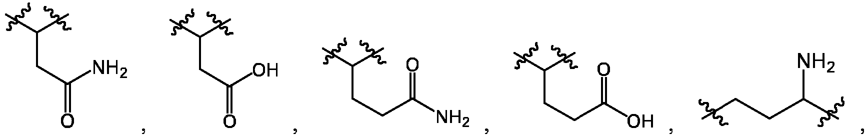 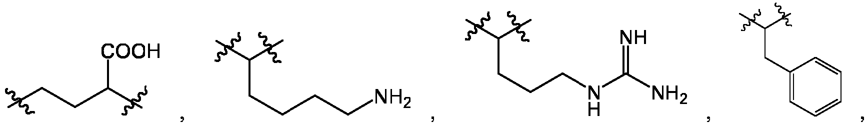 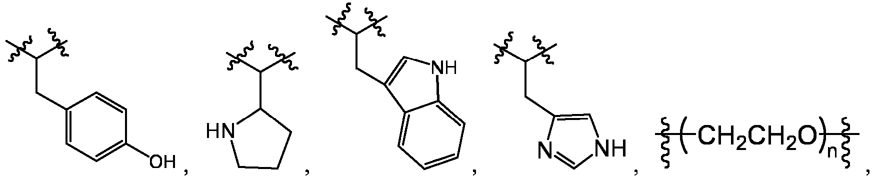 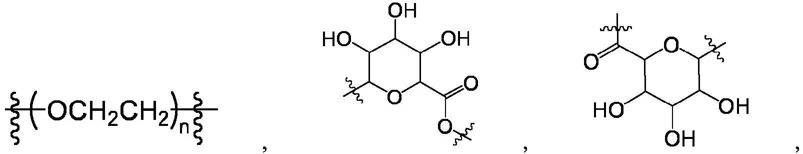 or 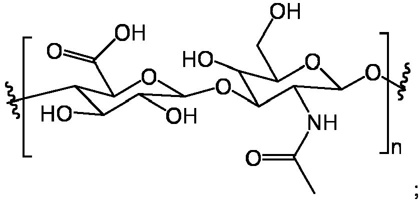 and
preferably, n is an integer of 1-10.

4. The compound, stereoisomer of the compound, any composition of the compound and the stereoisomer, any composition of the stereoisomers, or the pharmaceutically acceptable salt, ester, ether, optical isomer, isotope derivative, solvate, prodrug, metal chelate, crystal form of the foregoing compound or composition of claim 1, wherein R₃₀₁ can be selected from a single bond, C1-6 alkylene, 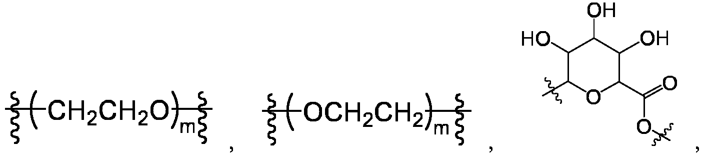 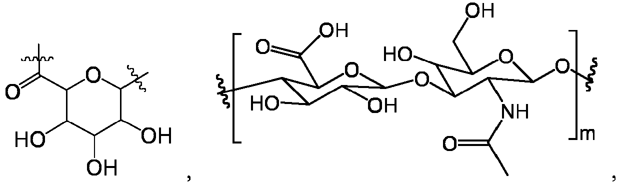 wherein the alkylene is optionally substituted with one or more independent R';
preferably, R' is selected from hydroxyl, mercapto, amino, amido, sulfonamido, formic acid group, nitro, formaldehyde group, trifluoromethyl, 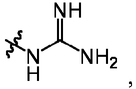 vinyl, ethynyl or substituted or unsubstituted phenyl (e.g., 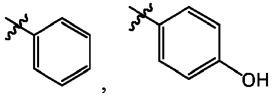 ), heterocyclyl (e.g., 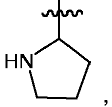 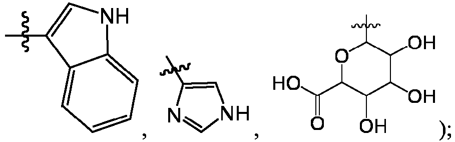
more preferably, R₃₀₁ is selected from a single bond, methylene, ethylene, 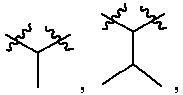 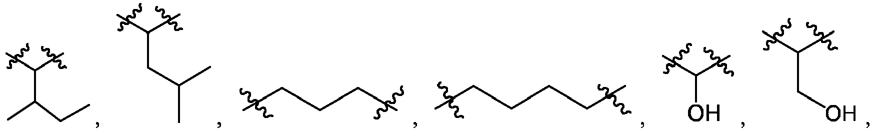 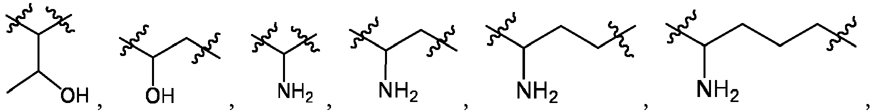 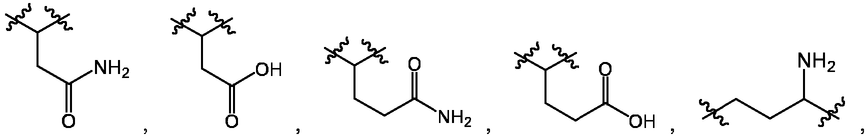 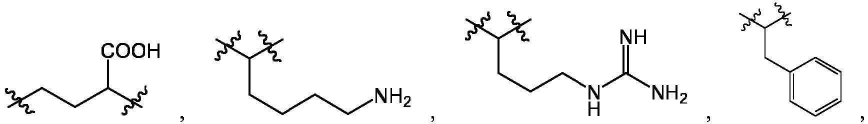 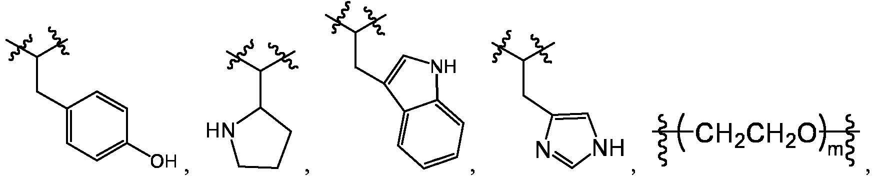 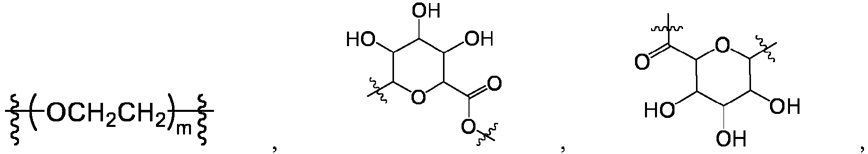 or 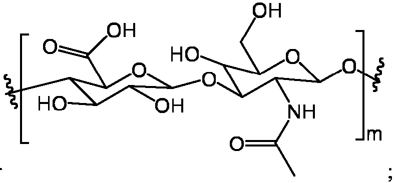 and
preferably, m is an integer of 1-10.

5. The compound, stereoisomer of the compound, any composition of the compound and the stereoisomer, any composition of the stereoisomers, or the pharmaceutically acceptable salt, ester, ether, optical isomer, isotope derivative, solvate, prodrug, metal chelate, crystal form of the foregoing compound or composition of claim 1, wherein X₂ is selected from a single bond, -O-, -S-, -S-S-, -N(R₁₀₄)-, -S(O)₂-, -S(O)₂N(R₁₀₄)-, -C(O)O- or -C(O)N(R₁₀₄)-;
and/or,
Y₂ is selected from a single bond, -O-, -S-, -S-S-, -N(R₃₀₄)-, -S(O)₂-, -S(O)₂N(R₃₀₄)-, -C(O)O- or -C(O)N(R₃₀₄)-;
preferably, R₁₀₄ is selected from H or C1-6 alkyl; and
preferably, R₃₀₄ is selected from H or C1-6 alkyl.

6. The compound, stereoisomer of the compound, any composition of the compound and the stereoisomer, any composition of the stereoisomers, or the pharmaceutically acceptable salt, ester, ether, optical isomer, isotope derivative, solvate, prodrug, metal chelate, crystal form of the foregoing compound or composition of claim 1, wherein R₁₀₂ is selected from H or C1-6 alkyl (in particular C1-3 alkyl, e.g., methyl, ethyl, n-propyl, isopropyl), 4- to 12-membered saturated heterocyclyl (including monocyclic heterocyclyl, polycyclic heterocyclyl (in particular bicyclic heterocyclyl), Polycyclic heterocycle including fused, spiro, bridged polycyclic heterocycle, e.g., 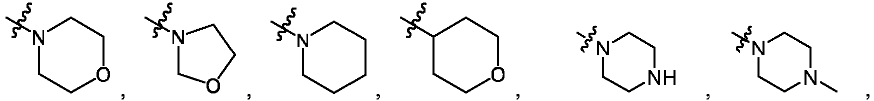 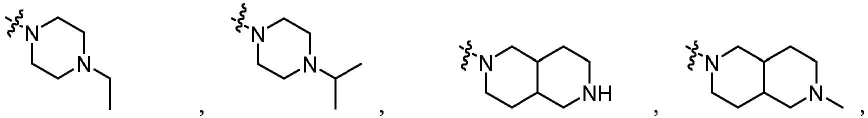 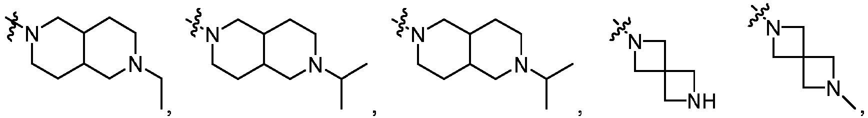 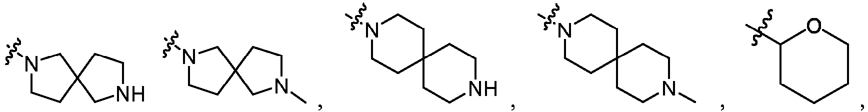 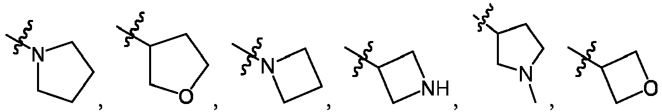 ), wherein the alkyl and heterocyclyl are optionally substituted with one or more independent R';
and/or,
R₃₀₂ is selected from H, C1-6 alkyl (in particular C1-3 alkyl, e.g., methyl, ethyl, n-propyl, isopropyl), 4- to 12-membered saturated heterocyclyl (including monocyclic heterocyclyl, polycyclic heterocyclyl (in particular bicyclic heterocyclyl), including fused, spiro, bridged polycyclic heterocyclyl, e.g., 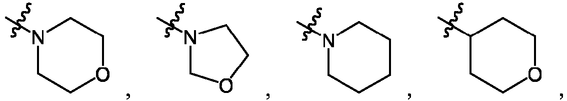 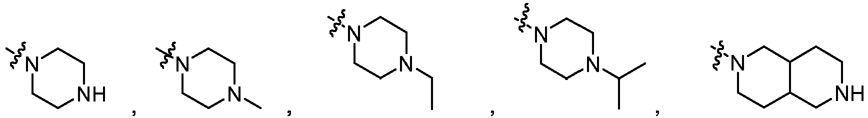 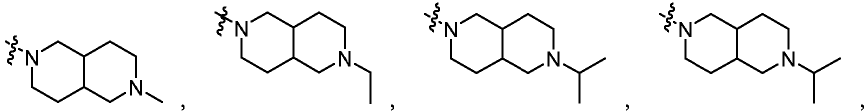 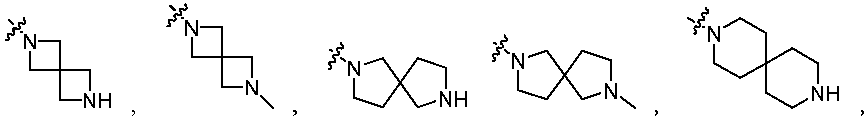 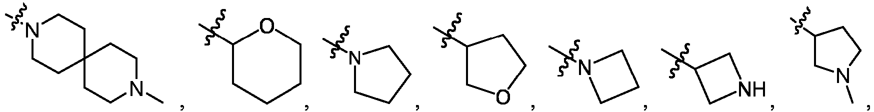 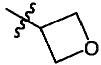 ), wherein the alkyl and heterocyclyl are optionally substituted with one or more independent R'.

7. The compound, stereoisomer of the compound, any composition of the compound and the stereoisomer, any composition of the stereoisomers, or the pharmaceutically acceptable salt, ester, ether, optical isomer, isotope derivative, solvate, prodrug, metal chelate, crystal form of the foregoing compound or composition of claim 1, wherein R₁ is H, or, R₁ is selected from 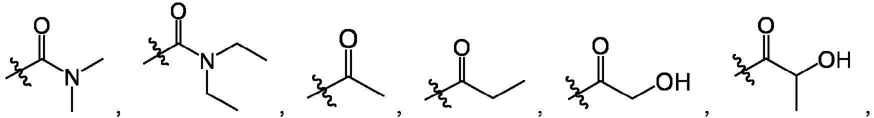 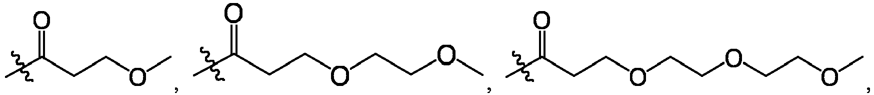 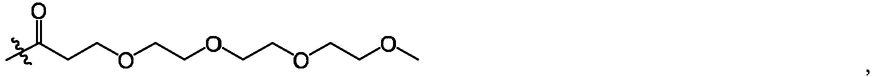 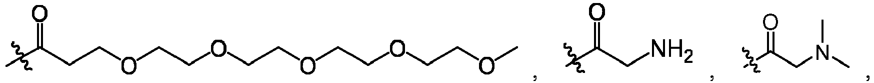 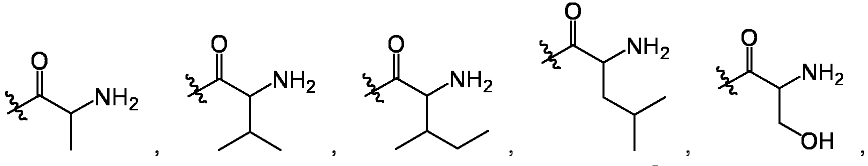 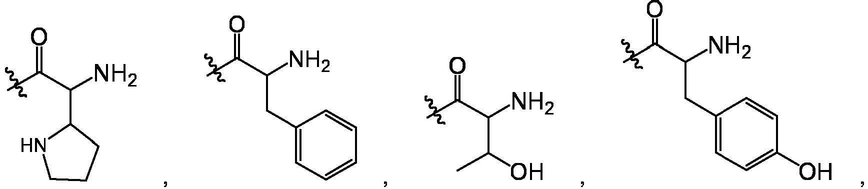 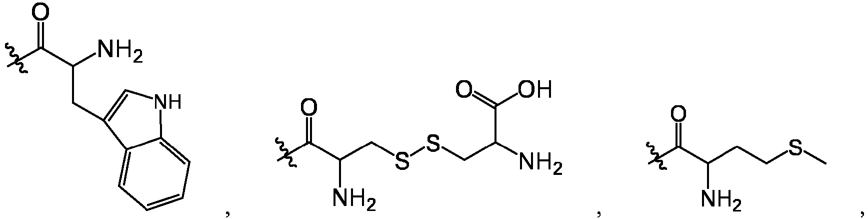 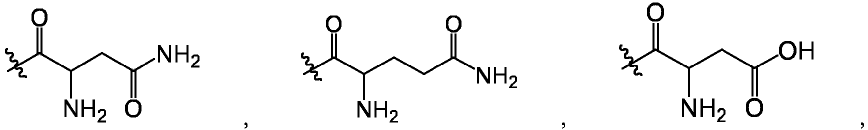 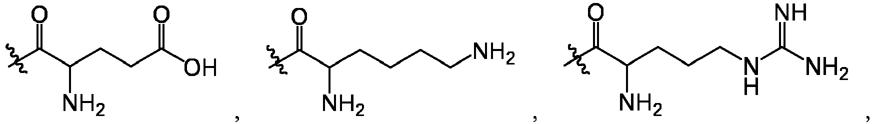 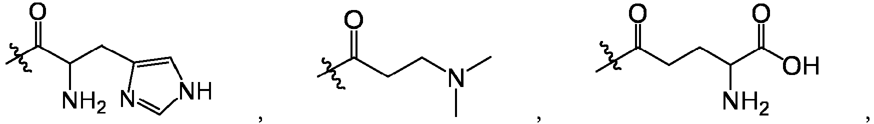 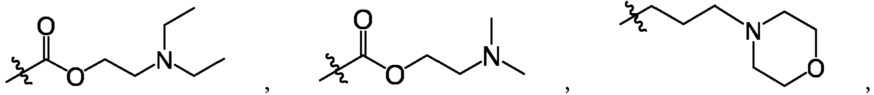 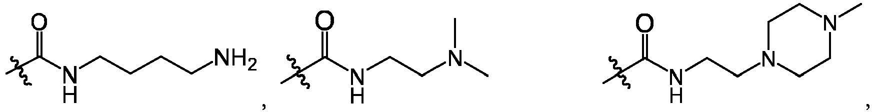 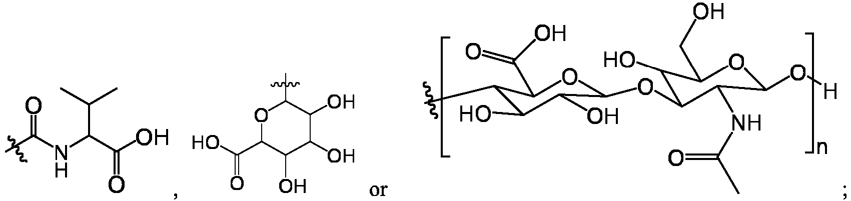 and/or,
R₃ is H, or, R₃ is selected from

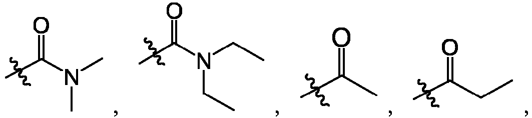

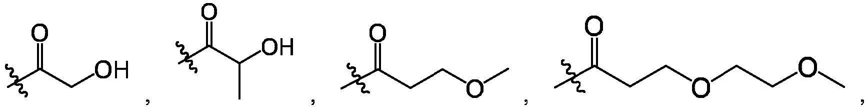

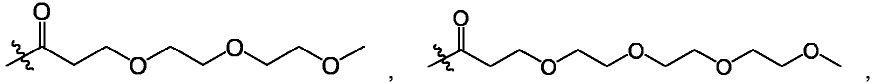

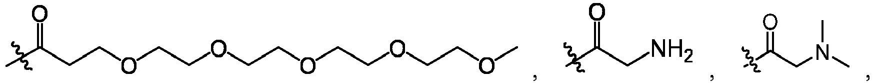

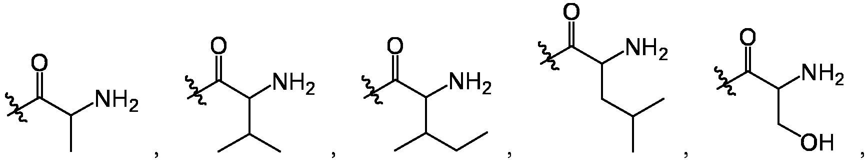

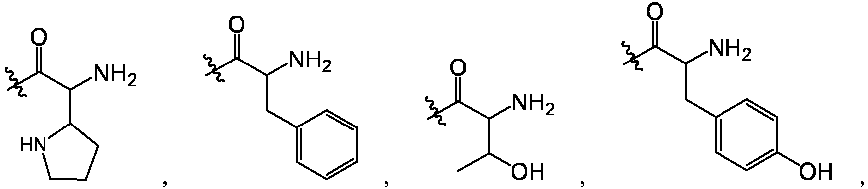

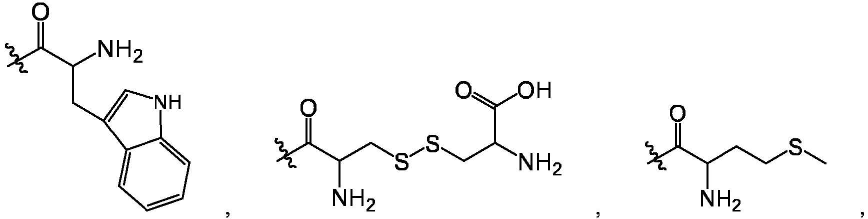

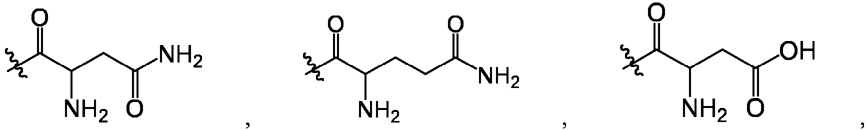

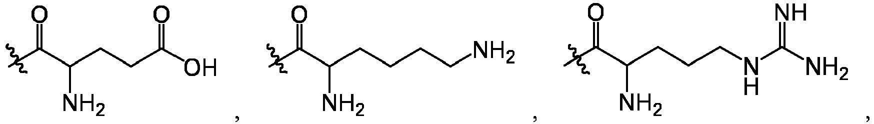

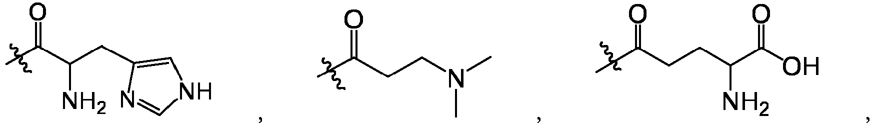

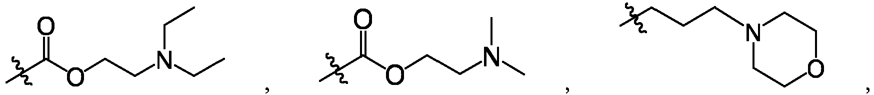

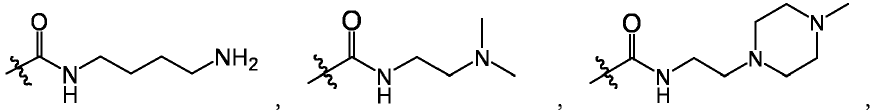

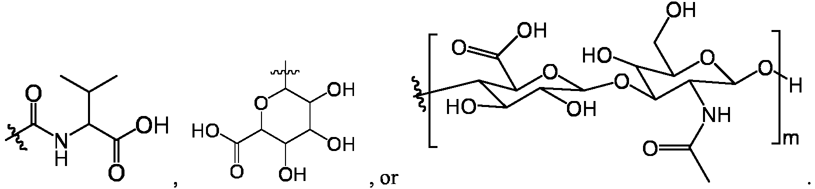

8. The compound, stereoisomer of the compound, any composition of the compound and the stereoisomer, any composition of the stereoisomers, or the pharmaceutically acceptable salt, ester, ether, optical isomer, isotope derivative, solvate, prodrug, metal chelate, crystal form of the foregoing compound or composition of any one of claims 1-7, wherein R₂ is H.

9. The compound, stereoisomer of the compound, any composition of the compound and the stereoisomer, any composition of the stereoisomers, or the pharmaceutically acceptable salt, ester, ether, optical isomer, isotope derivative, solvate, prodrug, metal chelate, crystal form of the foregoing compound or composition of any one of claims 1-8, wherein R₄ is selected from H, C1-6 alkyl; wherein the alkyl is optionally substituted with one or more independent R'.
preferably, R' is selected from fluorine, chlorine, bromine, iodine, hydroxyl, mercapto, amino, formic acid group, nitro, formaldehyde group, trifluoromethyl, vinyl, ethynyl;
more preferably, R₄ is methyl.

10. The compound, stereoisomer of the compound, any composition of the compound and the stereoisomer, any composition of the stereoisomers, or the pharmaceutically acceptable salt, ester, ether, optical isomer, isotope derivative, solvate, prodrug, metal chelate, crystal form of the foregoing compound or composition of any one of claims 1-9, wherein R₅ is selected from H, C1-6 alkyl; wherein the alkyl is optionally substituted with one or more independent R'.
preferably, R' is selected from fluorine, chlorine, bromine, iodine, hydroxyl, mercapto, amino, formic acid group, nitro, formaldehyde group, trifluoromethyl, vinyl, ethynyl;
more preferably, R₅ is H.

11. The compound, stereoisomer of the compound, any composition of the compound and the stereoisomer, any composition of the stereoisomers, or the pharmaceutically acceptable salt, ester, ether, optical isomer, isotope derivative, solvate, prodrug, metal chelate, crystal form of the foregoing compound or composition of any one of claims 1-10, wherein L₁ and L₂ are independently selected from a single bond, -O-, -S-, -N(H), -S(O)₂-, -S(O)₂N(H)-, -C(O)-, -C(O)O-, -C(O)N(H)-, -OC(O)- or -N(H)C(O)-; preferably, R₆ is selected from H, C1-6 alkyl,

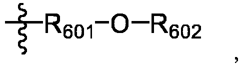

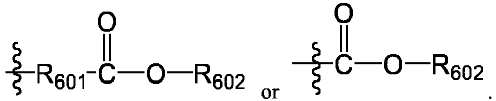

12. The compound, stereoisomer of the compound, any composition of the compound and the stereoisomer, any composition of the stereoisomers, or the pharmaceutically acceptable salt, ester, ether, optical isomer, isotope derivative, solvate, prodrug, metal chelate, crystal form of the foregoing compound or composition of any one of claims 1-11, wherein R₆₀₁ is selected from a single bond or C1-6 alkylene;
R₆₀₂ is selected from H or C1-6 alkyl;
R₆₀₃ is selected from H or C1-6 alkyl;
preferably, R₆ is methyl.

13. The compound, stereoisomer of the compound, any composition of the compound and the stereoisomer, any composition of the stereoisomers, or the pharmaceutically acceptable salt, ester, ether, optical isomer, isotope derivative, solvate, prodrug, metal chelate, crystal form of the foregoing compound or composition of any one of claims 1-12, wherein R₇ is C2-6 alkenyl; preferably, R₇ is selected from vinyl, propenyl, allyl, prop-1-en-2-yl; more preferably, R₇ is

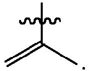

14. The compound, stereoisomer of the compound, any composition of the compound and the stereoisomer, any composition of the stereoisomers, or the pharmaceutically acceptable salt, ester, ether, optical isomer, isotope derivative, solvate, prodrug, metal chelate, crystal form of the foregoing compound or composition of any one of claims 1-13, wherein W is 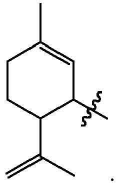 preferably, the compound has the following structure: 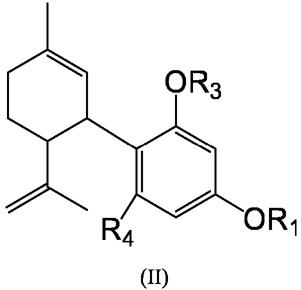 more preferably, in the compound, R₁ is H and R₃ is not H; or, R₁ is H and R₃ is not H; or, neither R₁ nor R₃ is H.

15. The compound, stereoisomer of the compound, any composition of the compound and the stereoisomer, any composition of the stereoisomers, or the pharmaceutically acceptable salt, ester, ether, optical isomer, isotope derivative, solvate, prodrug, metal chelate, crystal form of the foregoing compound or composition of claim 1, wherein the compound is selected from the following structures:

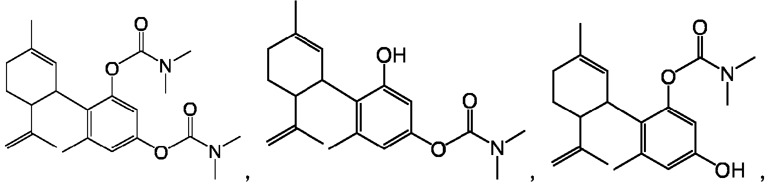

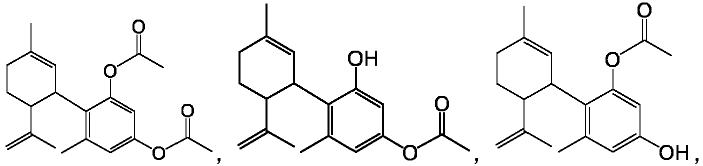

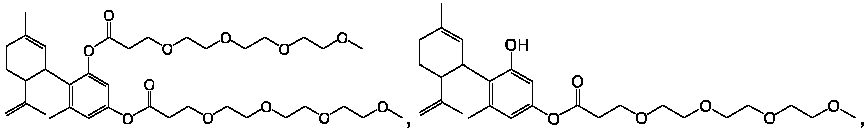

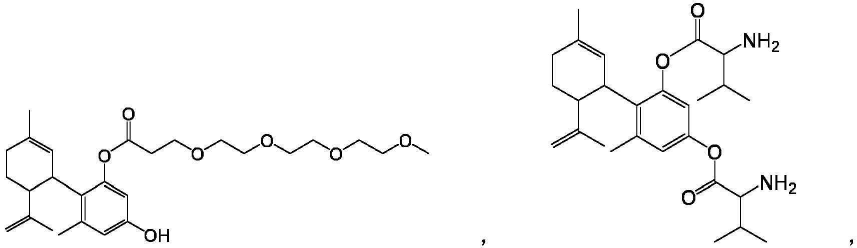

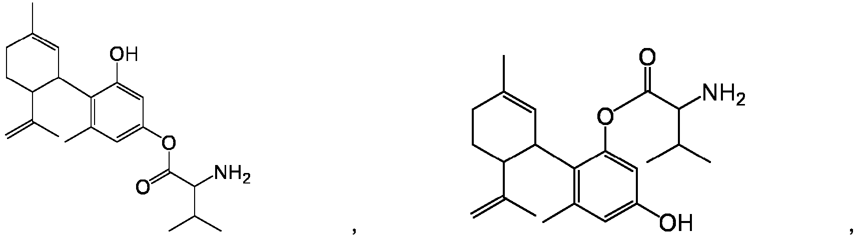

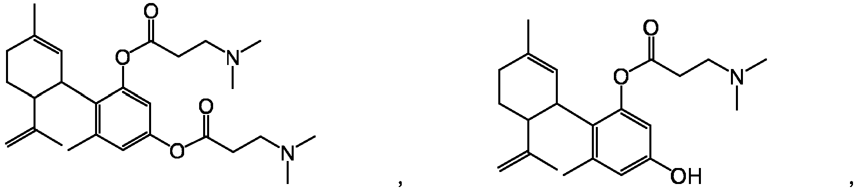

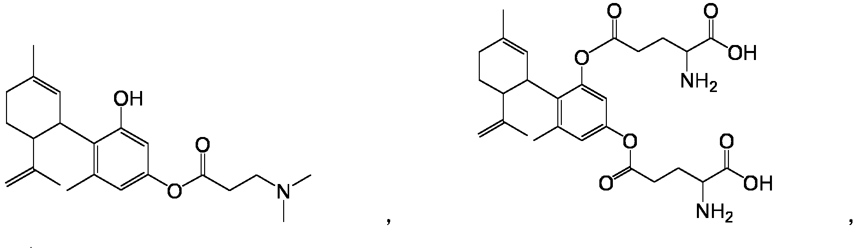

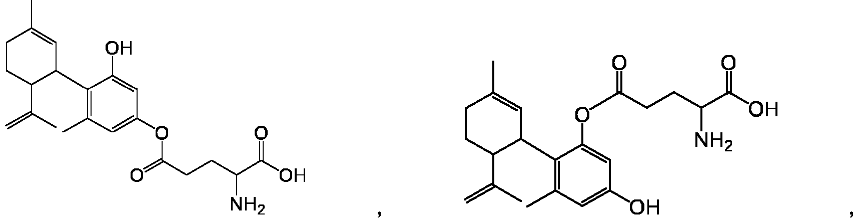

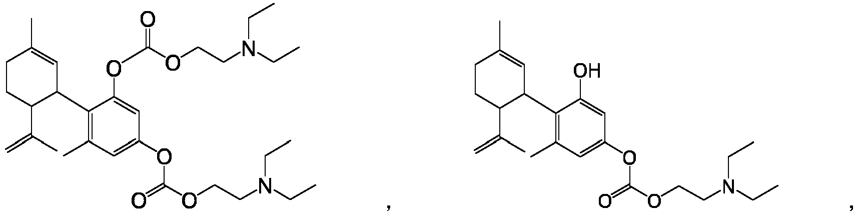

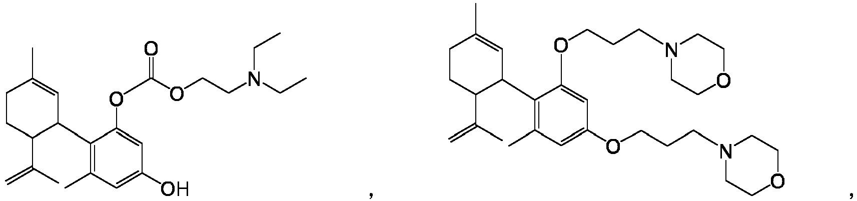

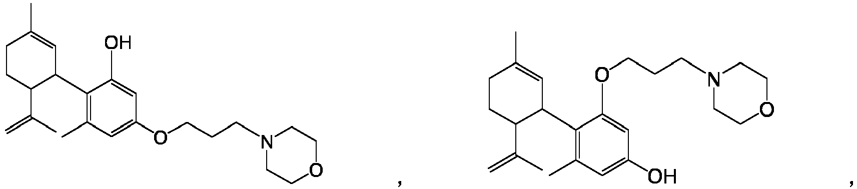

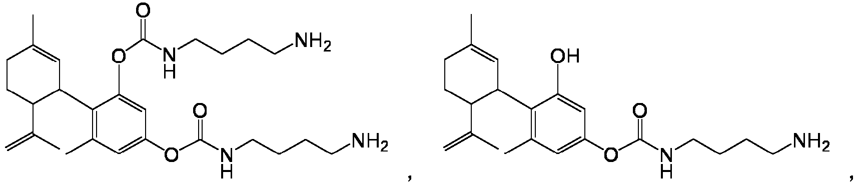

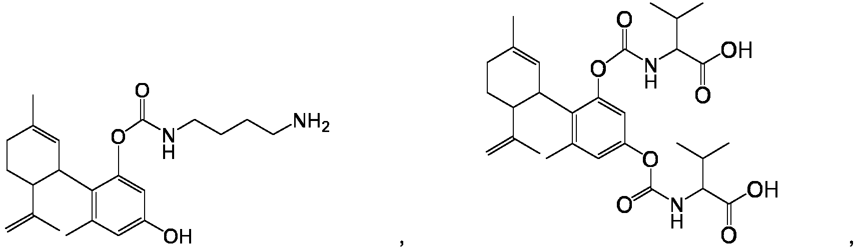

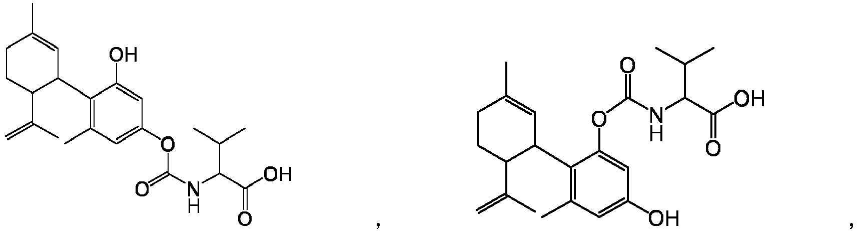

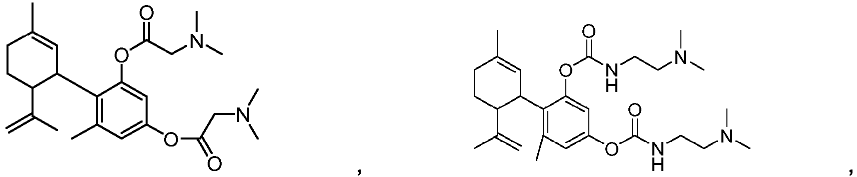

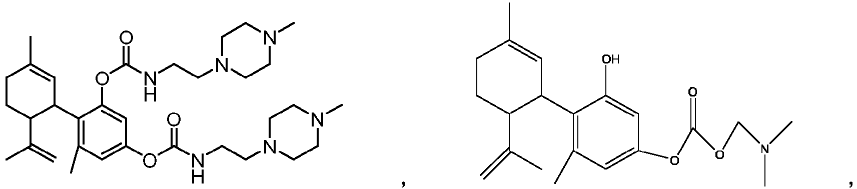

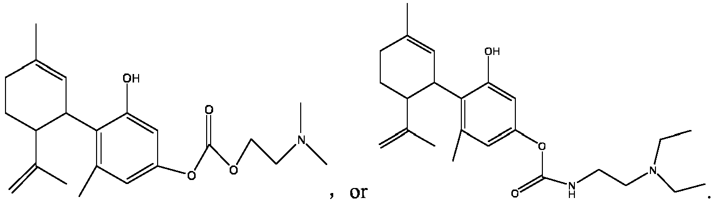

16. The compound, stereoisomer of the compound, any composition of the compound and the stereoisomer, any composition of the stereoisomers, or the pharmaceutically acceptable salt, ester, ether, optical isomer, isotope derivative, solvate, prodrug, metal chelate, crystal form of the foregoing compound or composition of any one of claims 1-13, wherein the stereoisomer is selected from the following structures: 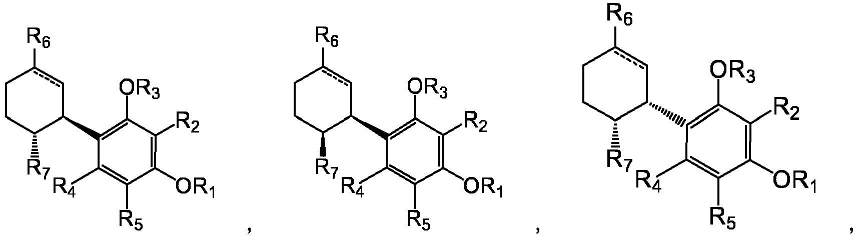 or 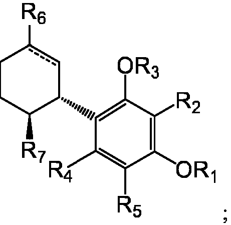
preferably 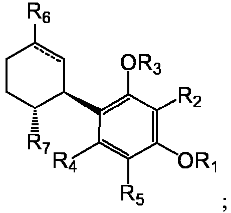
preferably, the stereoisomer is selected from the following structures: 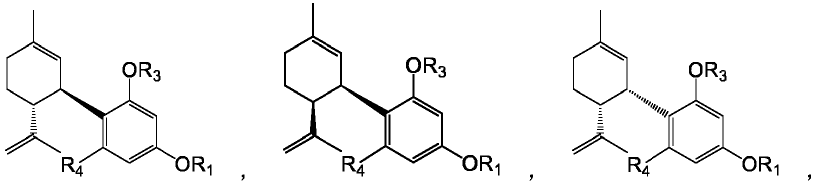 or 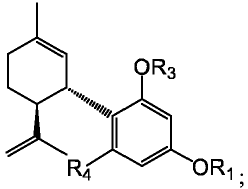
preferably 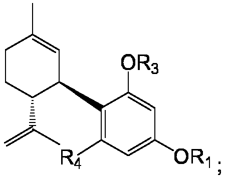
more preferably, the stereoisomer is selected from the following structures:

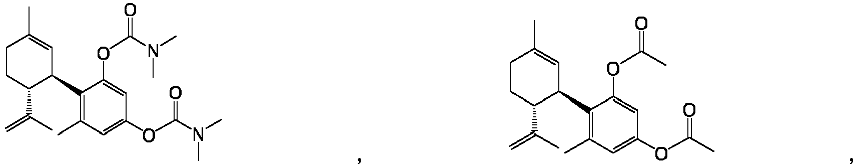

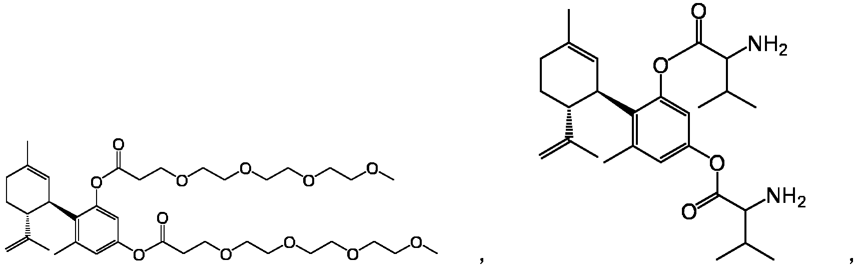

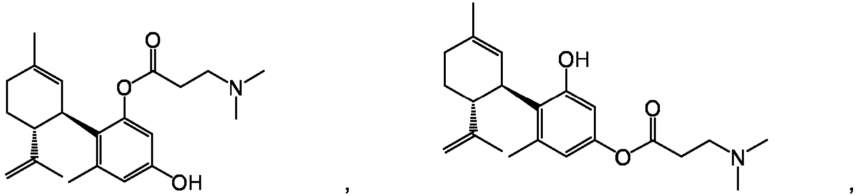

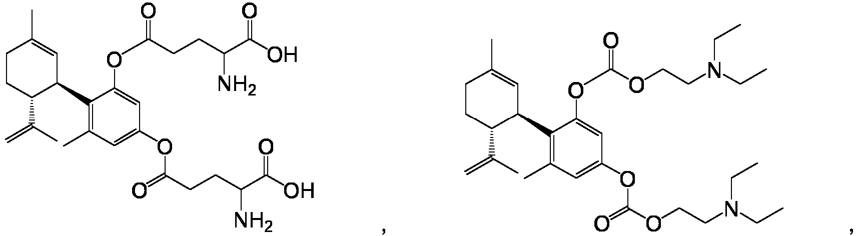

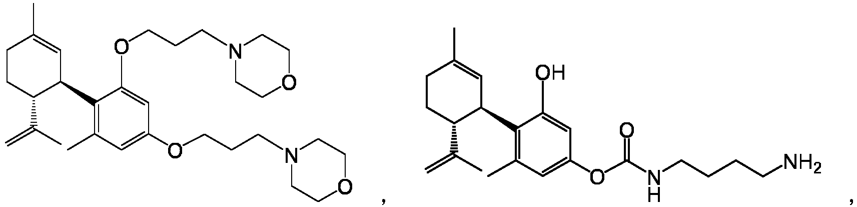

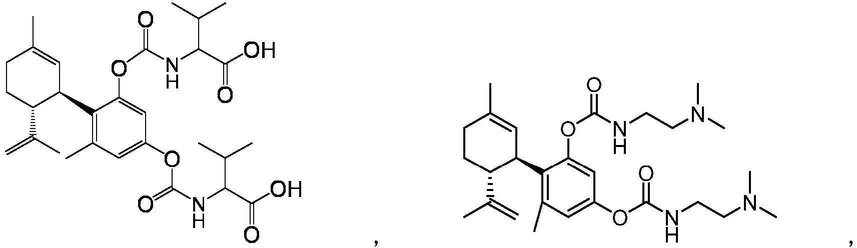

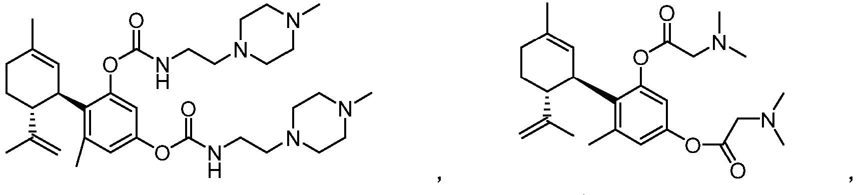

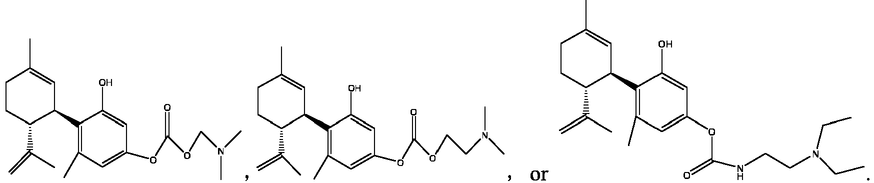

17. A pharmaceutical composition comprising the compound, or the pharmaceutically acceptable salt, ester, ether, optical isomer, isotope derivative, stereoisomer, solvate, prodrug, metal chelate, crystal form thereof of any one of claims 1-16, and one or more pharmaceutically acceptable excipients.

18. Use of the compound, stereoisomer of the compound, any composition of the compound and the stereoisomer, any composition of the stereoisomers, or the pharmaceutically acceptable salt, ester, ether, optical isomer, isotope derivative, solvate, prodrug, metal chelate, crystal form of the foregoing compound or composition of any one of claims 1-16 in preparing a medicament for preventing and/or treating a diseases;
preferably, the diseases are those diseases that benefitfrom an increase in the quantity of Tregs for their prevention or treatment, e.g., an autoimmune disease, an allergic disease, transplant rejection, a graft-versus-host disease (GvHD), and an inflammatory disease.

19. The use of claim 18, wherein the autoimmune disease is selected from achalasia, Addison's disease, adult Still's disease, agammaglobulinemia, alopecia areata, amyloidosis, ankylosing spondylitis, immune-mediated nephropathy, antiphospholipid syndrome (APS), autoimmune angioedema, autoimmune dysautonomia, autoimmune encephalomyelitis, autoimmune hepatitis, autoimmune inner ear disease (AIED), autoimmune myocarditis, autoimmune oophoritis, autoimmune orchitis, autoimmune pancreatitis, autoimmune retinopathy, autoimmune urticaria, axonal & neuronal neuropathy (AMAN), Baló disease , Behcet's disease, benign mucosal pemphigoid , bullous pemphigoid, castleman disease (CD), food allergy related diseases (such as Celiac disease), Chagas disease, chronic inflammatory demyelinating polyneuropathy (CIDP), chronic recurrent multifocal osteomyelitis (CRMO), Churg-Strauss Syndrome (CSS)), Cogan's syndrome, cold agglutinin disease, congenital heart block, Coxsackie myocarditis, CREST syndrome, Crohn's disease, dermatitis herpetiformis, dermatomyositis, Devic's disease, discoid lupus, Dressler's syndrome, endometriosis, eosinophilic esophagitis (EoE), eosinophilic fasciitis, erythema nodosum, essential mixed cryoglobulinemia, Evans syndrome, fibromyalgia, fibrosing alveolitis, giant cell arteritis, giant cell myocarditis, Goodpasture's syndrome, granulomatosis with polyangiitis (GPA), Graves' disease, Guillain-Barré syndrome, Hashimoto's thyroiditis, hemolytic anemia, Henoch-Schonlein purpura (HSP) (allergic purpura), herpes gestationis (pemphigoid gestationis), hidradenitis Suppurativa, hypogammaglobulinemia, IgG4-related sclerosing disease, immune thrombocytopenic purpura (ITP), inclusion body myositis (IBM), interstitial cystitis (IC), juvenile arthritis, juvenile myositis, Kawasaki disease, Lambert-Eaton syndrome, leukocytoclastic vasculitis, lichen planus, lichen sclerosus, ligneous conjunctivitis, linear IgA disease, chronic Lyme disease , Meniere's disease, microscopic polyangiitis, mixed connective tissue disease, Mooren's ulcer, Mucha-Habermann disease, multifocal motor neuropathy, multiple sclerosis, myasthenia gravis, myositis, narcolepsy, neonatal lupus, neutropenia, ocular cicatricial pemphigoid, optic neuritis, palindromic rheumatism, PANDAS, paraneoplastic cerebellar degeneration, paroxysmal nocturnal hemoglobinuria, Parry Romberg syndrome, pars planitis , Parsonage-Turner syndrome, pemphigus, peripheral neuropathy, perivenous encephalomyelitis, pernicious anemia, POEMS syndrome, polyarteritis nodosa, polyglandular syndromes type I, II, III, polymyalgia rheumatica, polymyositis, postmyocardial infarction syndrome, postpericardiotomy syndrome, primary biliary cirrhosis, primary sclerosing cholangitis, progesterone dermatitis, psoriasis, psoriatic arthritis, pure red cell aplasia, pyoderma gangrenosum, Raynaud's phenomenon, reactive Arthritis, reflex sympathetic dystrophy, relapsing polychondritis, restless legs syndrome, retroperitoneal fibrosis, rheumatic fever, rheumatoid arthritis, sarcoidosis, Schmidt syndrome , scleritis, scleroderma, Sjögren's syndrome, Sperm & testicular autoimmunity, stiff person syndrome, subacute bacterial endocarditis, Susac's syndrome, sympathetic ophthalmia, systemic lupus erythematosus (SLE), Takayasu's arteritis, temporal arteritis, thyroid eye disease, Tolosa-Hunt syndrome, Type I diabetes, ulcerative colitis, undifferentiated connective tissue disease, uveitis, vasculitis, vitiligo, or Vogt-Koyanagi-Harada disease;
preferably, the immune nephropathy is selected from anti-glomerular basement membrane antibody nephritis, glomerulonephritis, IgA nephropathy, purpuric nephritis, or lupus nephritis; and
preferably, the autoimmune diseases are selected from systemic lupus erythematosus, type I diabetes, rheumatoid arthritis, multiple sclerosis, ankylosing spondylitis, psoriasis, food allergy and related diseases (e.g., Celiac diseases), ulcerative colitis, or Crohn's disease.

20. The use of claim 18, wherein the allergic disease is selected from allergic conjunctivitis, allergic rhinitis, allergic asthma, allergic bronchopulmonary aspergillosis, allergic gastroenteritis, atopic dermatitis, allergic urticaria, allergic angioedema, allergic immediate contact reaction, anaphylactic shock, or food allergy and related diseases (e.g., Celiac disease).

21. The use of claim 18, wherein for transplant rejection, graft-versus-host disease (GvHD), the transplantation is selected from one or more of cell transplantation, tissue transplantation, or organ transplantation, preferably organ transplantation;
preferably, the cell transplantation is hematopoietic stem cell transplantation;
preferably, the tissue transplantation is selected from skin transplantation, bone marrow transplantation, or corneal transplantation; and
preferably, the organ transplantation is selected from kidney transplantation, heart transplantation, liver transplantation, pancreas and islet transplantation, parathyroid transplantation, lung transplantation, or small intestine transplantation.

22. The use of claim 18, wherein the inflammatory disease is a disease with inflammation caused by the immune response;
preferably, the inflammatory disease is selected from interstitial lung disease, inflammatory bowel disease, chronic obstructive pulmonary disease (COPD), acute lung injury, neuroinflammation, sepsis, asthma, or allergy; and
preferably, the inflammatory bowel disease is selected from chronic colitis, acute colitis, ulcerative colitis, or Crohn's disease.

23. The use of any one of claims 18-22, wherein the disease is inflammatory bowel disease and the medicament is administered by gastrointestinal administration route, in particular by oral administration route.

24. The use of any one of claims 18-23, wherein the subject of the medicament is a mammal, in particular a human, and is administered at a dosage of 0.033-0.407 mg/kg, based on the body weight, with a dosing frequency of once a day.

25. The use of the compound, stereoisomer of the compound, any composition of the compound and the stereoisomer, any composition of the stereoisomers, or the pharmaceutically acceptable salt, ester, ether, optical isomer, isotope derivative, solvate, prodrug, metal chelate, crystal form of the foregoing compound or composition of any one of claims 1-16 in preparing a reagent for inducing or promoting Tregs amplification; and
preferably, the Tregs amplification is performed in vitro or in vivo.

26. A method for Tregs amplification, comprising the step of using the compound, stereoisomer of the compound, any composition of the compound and the stereoisomer, any composition of the stereoisomers, or the pharmaceutically acceptable salt, ester, ether, optical isomer, isotope derivative, solvate, prodrug, metal chelate, crystal form of the foregoing compound or composition of any one of claims 1-16, or a pharmaceutical composition comprising the compound, stereoisomer of the compound, any composition of the compound and the stereoisomer, any composition of the stereoisomers, or the pharmaceutically acceptable salt, ester, ether, optical isomer, isotope derivative, solvate, prodrug, metal chelate, crystal form of the foregoing compound or composition of any one of claims 1-16;
preferably, the method is performed in vitro.

27. A method for preventing and/or treating a diseases, the method comprising administering to a subject in need thereof the compound, stereoisomer of the compound, any composition of the compound and the stereoisomer, any composition of the stereoisomers, or the pharmaceutically acceptable salt, ester, ether, optical isomer, isotope derivative, solvate, prodrug, metal chelate, crystal form of the foregoing compound or composition of any one of claims 1-16; and
preferably, the diseases are those diseases that are benefitfrom an increase in the quantity of Tregs for their prevention or treament, e.g., an autoimmune disease, an allergic disease, transplant rejection, a graft-versus-host disease (GvHD), and an inflammatory disease.

28. The method of claim 27, wherein the autoimmune disease is selected from achalasia, Addison's disease, adult Still's disease, agammaglobulinemia, alopecia areata, amyloidosis, ankylosing spondylitis, immune-mediated nephropathy, antiphospholipid syndrome, autoimmune angioedema, autoimmune dysautonomia, autoimmune encephalomyelitis, autoimmune hepatitis, autoimmune inner ear disease, autoimmune myocarditis, autoimmune oophoritis, autoimmune orchitis, autoimmune pancreatitis, autoimmune retinopathy, autoimmune urticaria, axonal & neuronal neuropathy, Baló disease, Behcet's disease, benign mucosal pemphigoid, bullous pemphigoid, castleman disease, food allergy related diseases (e.g., Celiac disease), Chagas disease, chronic inflammatory demyelinating polyneuropathy , chronic recurrent multifocal osteomyelitis, Churg-Strauss Syndrome , Cogan's syndrome, cold agglutinin disease, congenital heart block, Coxsackie myocarditis, CREST syndrome, Crohn's disease, dermatitis herpetiformis, dermatomyositis, Devic's disease, discoid lupus, Dressler's syndrome, endometriosis, eosinophilic esophagitis , eosinophilic fasciitis, erythema nodosum, essential mixed cryoglobulinemia, Evans syndrome, fibromyalgia, fibrosing alveolitis, giant cell arteritis, giant cell myocarditis, Goodpasture's syndrome, granulomatosis with polyangiitis , Graves' disease, Guillain-Barré syndrome, Hashimoto's thyroiditis, hemolytic anemia, Henoch-Schonlein purpura, herpes gestationis, hidradenitis Suppurativa, hypogammaglobulinemia, IgG4-related sclerosing disease, immune thrombocytopenic purpura , inclusion body myositis, interstitial cystitis, juvenile arthritis, juvenile myositis, Kawasaki disease, Lambert-Eaton syndrome, leukocytoclastic vasculitis, lichen planus, lichen sclerosus, ligneous conjunctivitis, linear IgA disease, chronic Lyme disease , Meniere's disease, microscopic polyangiitis, mixed connective tissue disease, Mooren's ulcer, Mucha-Habermann disease, multifocal motor neuropathy, multiple sclerosis, myasthenia gravis, myositis, narcolepsy, neonatal lupus, neutropenia, ocular cicatricial pemphigoid, optic neuritis, palindromic rheumatism, PANDAS, paraneoplastic cerebellar degeneration, paroxysmal nocturnal hemoglobinuria, Parry Romberg syndrome, pars planitis, Parsonage-Turner syndrome, pemphigus, peripheral neuropathy, perivenous encephalomyelitis, pernicious anemia, POEMS syndrome, polyarteritis nodosa, polyglandular syndromes type I, II, III, polymyalgia rheumatica, polymyositis, postmyocardial infarction syndrome, postpericardiotomy syndrome, primary biliary cirrhosis, primary sclerosing cholangitis, progesterone dermatitis, psoriasis, psoriatic arthritis, pure red cell aplasia, pyoderma gangrenosum, Raynaud's phenomenon, reactive Arthritis, reflex sympathetic dystrophy, relapsing polychondritis, restless legs syndrome, retroperitoneal fibrosis, rheumatic fever, rheumatoid arthritis, sarcoidosis, Schmidt syndrome, scleritis, scleroderma, Sjögren's syndrome, Sperm & testicular autoimmunity, stiff person syndrome, subacute bacterial endocarditis, Susac's syndrome, sympathetic ophthalmia, systemic lupus erythematosus, Takayasu's arteritis, temporal arteritis, thyroid eye disease, Tolosa-Hunt syndrome, Type I diabetes, ulcerative colitis, undifferentiated connective tissue disease, uveitis, vasculitis, vitiligo, or Vogt-Koyanagi-Harada disease;
preferably, the immune nephropathy is selected from anti-glomerular basement membrane antibody nephritis, glomerulonephritis, IgA nephropathy, purpuric nephritis, or lupus nephritis; and
preferably, the autoimmune diseases are selected from systemic lupus erythematosus, type I diabetes, rheumatoid arthritis, multiple sclerosis, ankylosing spondylitis, psoriasis, food allergy and related diseases (e.g., Celiac diseases), ulcerative colitis, or Crohn's disease.

29. The method of claim 27, wherein the allergic disease is selected from allergic conjunctivitis, allergic rhinitis, allergic asthma, allergic bronchopulmonary aspergillosis, allergic gastroenteritis, atopic dermatitis, allergic urticaria, allergic angioedema, allergic immediate contact reaction, anaphylactic shock, or food allergy and related diseases (e.g., Celiac disease).

30. The method of claim 27, wherein for transplant rejection, graft-versus-host disease (GvHD), the transplantation is selected from one or more of cell transplantation, tissue transplantation, or organ transplantation, preferably organ transplantation;
preferably, the cell transplantation is hematopoietic stem cell transplantation;
preferably, the tissue transplantation is selected from skin transplantation, bone marrow transplantation, or corneal transplantation; and
preferably, the organ transplantation is selected from kidney transplantation, heart transplantation, liver transplantation, pancreas and islet transplantation, parathyroid transplantation, lung transplantation, or small intestine transplantation.

31. The method of claim 27, wherein the inflammatory disease is a disease with inflammation caused by the immune response;
preferably, the inflammatory disease is selected from interstitial lung disease, inflammatory bowel disease, chronic obstructive pulmonary disease , acute lung injury, neuroinflammation, sepsis, asthma, or allergy; and
preferably, the inflammatory bowel disease is selected from chronic colitis, acute colitis, ulcerative colitis, or Crohn's disease.

32. The method of any one of claims 27-31, wherein the disease is inflammatory bowel disease and the method is used to prevent or treat the disease through the gastrointestinal route, especially the oral route.

33. The method of any one of claims 27-32, wherein the subject is a mammal, in particular a human, and is administered at a dosage of 0.033-0.407 mg/kg, based on the body weight, with a dosing frequency of once a day.
